(19) Europäisches Patentamt / European Patent Office / Office européen des brevets

(11) **EP 2 087 871 B1**

(12) **EUROPEAN PATENT SPECIFICATION**

(45) Date of publication and mention
of the grant of the patent:
**18.03.2015 Bulletin 2015/12**

(51) Int Cl.:
*A61F 13/496* (2006.01)      *A61F 13/15* (2006.01)
*A61F 13/49* (2006.01)       *A61F 13/515* (2006.01)

(21) Application number: **07832340.9**

(22) Date of filing: **22.11.2007**

(86) International application number:
**PCT/JP2007/072611**

(87) International publication number:
**WO 2008/065953 (05.06.2008 Gazette 2008/23)**

(54) **ABSORBENT ARTICLE**

SAUGFÄHIGER ARTIKEL

ARTICLE ABSORBANT

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **27.11.2006   JP 2006318211
11.12.2006   JP 2006333622
28.12.2006   JP 2006355555
23.03.2007   JP 2007077013**

(43) Date of publication of application:
**12.08.2009   Bulletin 2009/33**

(73) Proprietor: **Kao Corporation
Chuo-ku,
Tokyo 103-8210 (JP)**

(72) Inventors:
• **HAYASE, Toru
Haga-gun
Tochigi 321-3497 (JP)**
• **SASAKI, Jun
Haga-gun
Tochigi 321-3497 (JP)**
• **ONDA, Aiko
Haga-gun
Tochigi 321-3497 (JP)**
• **TAKAHASHI, Akio
Haga-gun
Tochigi 321-3497 (JP)**
• **TOMITA, Mina
Haga-gun
Tochigi 321-3497 (JP)**
• **OKUDA, Yasuyuki
Haga-gun
Tochigi 321-3497 (JP)**

(74) Representative: **Vossius & Partner
Siebertstrasse 3
81675 München (DE)**

(56) References cited:
**EP-A1- 1 547 558      EP-A1- 1 550 424
EP-A1- 1 621 168      US-A1- 2004 186 453**

**Description**

Technical Field

The present invention relates to an absorbent article, such as a disposable diaper or a sanitary napkin.

Background Art

Known disposable pull-on diapers having an absorbent body including an absorbent core and an outer cover joined to the garment-facing side of the absorbent body include a diaper having the outer cover elasticized along the waist and the below-waist portions in both the front region and the rear region to provide a better fit to a wearer's body, such as, e.g., disclosed in Patent Document 1. According to the disclosure, the outer cover is composed of an outer sheet, an inner sheet, and a plurality of elastic members arranged between the two sheets. The below-waist and the waist gathers are formed by the elastic members discontinuously bonded to the outer and inner sheets in the waist and the below-waist portions.

Also known is a disposable diaper that is designed to help perspiration to evaporate and escape along and through the waist flap (the term "waist flap" usually refers to the portion outward of each longitudinal end of an absorbent body including an absorbent core) thereby to prevent a diaper rash, such as, e.g., disclosed in Patent Document 2 and Patent Document 3. The waist flaps of the disposable diapers illustrated in Fig. 7 of Patent Document 2 and Figs. 2 and 6 of Patent Document 3 each include a hydrophobic sheet on both the skin facing side and garment facing side thereof, a hydrophilic sheet, and elastic members, the hydrophilic sheet and the elastic members being interposed between the hydrophobic sheets. That is, the waist flaps of the disposable diapers according to Patent Documents 2 and 3 have a 4-layer structure composed of a hydrophobic sheet, a hydrophilic sheet, an elastic member layer, and a hydrophilic sheet.

Patent Document 1 U.S. 7217261B2
Patent Document 2 JP 8-38548A
Patent Document 3 JP 2003-235892A

The disposable pull-on diaper of Patent Document 1 is inferior in breathability and softness in its waist and below-waist portions because these portions are elasticized by bonding the elastic members to the outer and inner sheets with a hot melt adhesive. Because the elastic members so bonded are hindered from contraction, it is needed to increase the number of elastic members or to use highly contractible elastic members in order to produce sufficient contractibility along the waist and below-waist portions. In other words, the contractibility essentially possessed by the elastic member is not made full use of. Moreover, the elasticized waist and below-waist portions have poor cushioning properties.

It is desired for a disposable pull-on diaper having an absorbent body and an outer cover to gently hold its elasticized waist portion against the waist of an infant (wearer), leaving no indentations or marks on the wearer's body. This applies as well to other absorbent articles, such as open style diapers with fasteners and pants type sanitary napkins.

EP 1 550 424 discloses a pull-on disposable wearing article wherein the region of the article having the auxiliary elastic members therein may be stretchable and contractible within a substantially same range as the range within which the auxiliary elastic members themselves are stretchable and contractible.

EP 1 547 558 discloses a pull-on disposable wearing article which may decrease the anxiety that auxiliary elastic members might locally gather together.

US 2004/0186453 discloses a pants-type disposable wearing article wherein slip down of the article along the wearer's waist and sideway leakage of bodily discharges may be prevented.

The present invention relates to an absorbent article having an elasticized waist portion and an elasticized below-waist portion, wherein the elasticized waist and below-waist portions has good breathability and softness while making full use of the contractibility of the elastic members used for elasticization, have excellent cushioning properties, and gently hugs the infant's body, causing little indentation on the body.

The present invention provides an absorbent article according to claim 1.

Brief Description of the Drawings

[Fig. 1] Fig. 1 is a perspective of a disposable pull-on diaper as an embodiment of the absorbent article of the invention.
[Fig. 2] Fig. 2 is a plan of the disposable pull-on diaper of Fig. 1 in its flat-out, uncontracted state.

[Fig. 3] Fig. 3 is a perspective exploded view of the disposable pull-on diaper of Fig. 2.

[Fig. 4] Fig. 4 is a cross-section taken along line IV-IV of Fig. 2.

[Fig. 5] Fig. 5(a), Fig. 5(b), and Fig. 5(c) illustrate the absorbent core used in the disposable pull-on diaper of Fig. 1, of which Fig. 5(a) is a plan, Fig. 5(b) is a cross-section taken along line B-B of Fig, 5(a), and Fig. 5(c) is a cross-section taken along line C-C of Fig. 5(a).

[Fig. 6] Fig. 6 is a plan of an outer cover in its flat-out, uncontracted state, seen from the inner side, with the inner sheet taken away.

[Fig. 7] Fig. 7 is a plan of an outer sheet in its flat-out, uncontracted state, illustrating areas to apply an adhesive.

[Fig. 8] Fig. 8 is a plan of an inner sheet in its flat-out, uncontracted state, illustrating areas to apply an adhesive.

[Fig. 9] Fig. 9 is an illustration of the ilia.

[Fig. 10] Fig. 10 illustrates how to calculate the sliding force around the waist of a wearer.

[Fig. 11] Fig. 11 illustrates how to measure the anterior-to-posterior length at the height of the anterior superior iliac spine.

[Fig. 12] Fig. 12 is a plan of a composite stretch panel in its stretched state.

[Fig. 13] Fig. 13 schematically illustrates the density of the folds of a gathered composite stretch panel.

[Fig. 14] Fig. 14 is a perspective illustrating a method of producing a disposable pull-on diaper of Fig. 1.

[Fig. 15] Fig. 15 is a conceptual diagram focused on the step of forming composite stretch panels, functionless regions, and leg elastic member cut regions in the method illustrated in Fig. 14.

[Fig. 16] Fig. 16 is a conceptual diagram focused on the step of making a composite stretch panel in the method illustrated in Fig. 14.

[Fig. 17] Fig. 17(a) and Fig. 17(b) are each an enlarged fragmental cross-section of a composite stretch panel, of which Fig. 17(a) shows a stretched state, and Fig. 17(b) a retracted state with folds.

[Fig. 18] Fig. 18(a) and Fig. 18(b) are each an enlarged fragmental cross-section of a composite stretch panel, of which Fig. 18(a) shows a stretched state, and Fig. 18(b) a retracted state with folds.

[Fig. 19] Fig. 19 is a plan of another composite stretch panel in its stretched state (equivalent to Fig. 12).

[Fig. 20] Fig. 20 is a plan of a disposable pull-on diaper as a second embodiment of the absorbent article according to the invention in its flat-out, uncontracted state.

[Fig. 21] Fig. 21 is a perspective exploded view of the disposable pull-on diaper of the second embodiment.

[Fig. 22] Fig. 22 is a cross-section taken along line V-V of Fig. 20.

[Fig. 23] Fig. 23 is a perspective of a composite stretch panel with part cut away.

[Fig. 24] Fig. 24 is a perspective illustrating a method of producing the disposable pull-on diaper of the second embodiment.

[Fig. 25] Fig. 25 is a conceptual diagram focused on the step of forming composite stretch panels, functionless regions, and leg elastic member cut regions in the method illustrated in Fig. 24.

[Fig. 26] Fig. 26 is a cross-section of a half-finished disposable pull-on diaper of the second embodiment, in which a holding sheet is ready to be folded back (corresponding to Fig. 22).

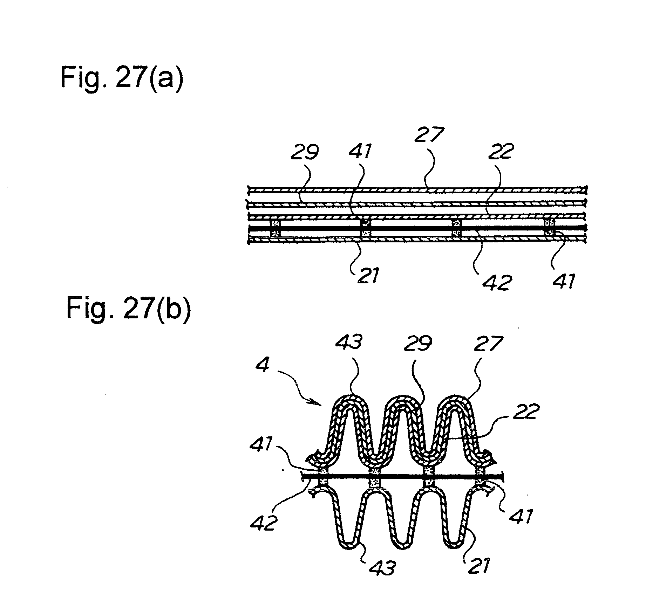

[Fig. 27] Fig. 27(a) and Fig. 27(b) are each an enlarged fragmental cross-section of a composite stretch panel, of which Fig. 27(a) shows a stretched state, and Fig. 27(b) a retracted state with folds.

[Fig. 28] Fig. 28 is a conceptual diagram of the step of forming composite stretch panels, functionless regions, and leg elastic member cut regions, the step being equal to that of Fig. 25 except for altering the position where a continuous holding sheet is joined.

[Fig. 29] Fig. 29 is a cross-section of a longitudinal end portion of a disposable pull-on diaper incorporating a third embodiment of the absorbent article according to the invention (equivalent to Fig. 22).

[Fig. 30] Fig. 30 is a cross-section of a half-finished disposable pull-on diaper of the third embodiment, in which a holding sheet is ready to be folded back (equivalent to Fig. 26).

[Fig. 31] Fig. 31 is a cross-section of a longitudinal end portion of a disposable pull-on diaper incorporating a fourth embodiment of the absorbent article according to the invention (equivalent to Fig. 22).

[Fig. 32] Fig. 32 is a cross-section of a longitudinal end portion of a disposable pull-on diaper incorporating a fifth embodiment of the absorbent article according to the invention (equivalent to Fig. 22).

[Fig. 33] Fig. 33 is a plan of still another composite stretch panel (equivalent to Fig. 12).

[Fig. 34] Fig. 34 is a schematic perspective illustration of an embodiment of a pull-on article of the invention in its worn state.

[Fig. 35] Fig. 35 is an enlarged, fragmental perspective view of region II in Fig. 34 with part cut away.

[Fig. 36] Fig. 36 is an enlarged cross-section, taken along line A-A in Fig. 35.

[Fig. 37] Fig. 37 is a schematic plan of the diaper of Fig. 34 in its flat-out, uncontracted state with part cut away.

[Fig. 38] Fig. 38(a) and Fig. 38(b) are each an enlarged view of elastic member fixing parts around the leg opening of Fig. 37, of which Fig. 38(a) is a schematic enlarged plan, and Fig. 38(b) is a schematic enlarged perspective with the elastic members contracted.

[Fig. 39] Fig. 39(a) and Fig. 39(b) are each an enlarged view of another form of elastic member fixing parts around the leg opening of Fig. 37, of which Fig. 39(a) is a schematic enlarged plan , and Fig. 39(b) is a schematic enlarged perspective with the elastic members contracted.

[Fig. 40] Fig. 40 is a schematic enlarged plan of region VI of Fig. 37 with part cut away.

[Fig. 41] Fig. 41 is a graph showing the stretch characteristics of the waist opening edge of the pull-on diaper illustrated in Fig. 34 in comparison with those of a conventional pull-on diaper.

[Fig. 42] Fig. 42 is a schematic perspective illustrating part of the steps involved in the production of a pull-on article according to the invention.

[Fig. 43] Fig. 43 is a schematic enlarged plan of region IX of Fig. 42.

[Fig. 44] Fig. 44(a) and Fig. 44(b) are each a further enlarged schematic plan of region X of Fig. 43, of which Fig. 44(a) shows a manner of cutting to make an opening in the region X, and Fig. 44(b) shows another manner of cutting.

Detailed Description of the Invention

The absorbent article of the invention will be described with reference to several disposable pull-on diapers as preferred embodiments of the invention by way of the accompanying drawing. In what follows, the term "disposable diaper" or simply "diaper" is substantially equivalent to the term "absorbent article" as contemplated by the present invention.

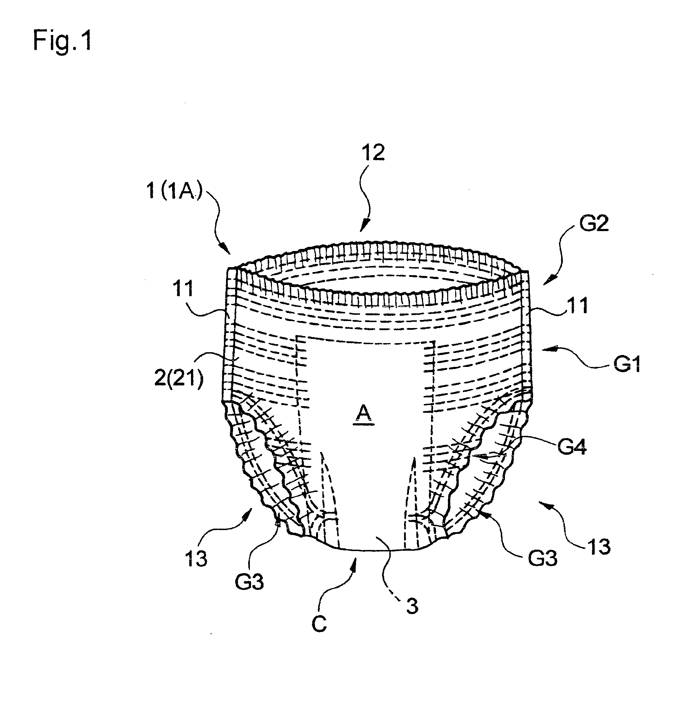

Figs. 1 to 4 illustrate a disposable pull-on diaper 1 according to a first embodiment. As illustrated, the diaper 1 has an absorbent body 3 including an absorbent core 34 and an outer cover 2 joined to the garment facing side of the absorbent body 3. The outer cover 2 is sectioned into a front section A, a crotch section C, and a rear section B along its longitudinal direction. Both side edges of the front section A and both side edges of the rear section B are joined together to make a disposable pull-on diaper having a pair of side seals 11, a waist opening 12, and a pair of leg openings 13.

The disposable pull-on diaper (absorbent article) of the first embodiment has an elasticized below-waist portion forming a below-waist gather and an elasticized waist portion forming a waist gather and exhibits breathability and softness in the below-waist and the waist portions. The elastic members gathering the below-waist and the waist portions are allowed to exhibit their contractibility sufficiently, providing the below-waist and the waist portions with good cushioning properties in their thickness direction. These gathers gently conform to the infant's body, hardly leaving indentations on the body.

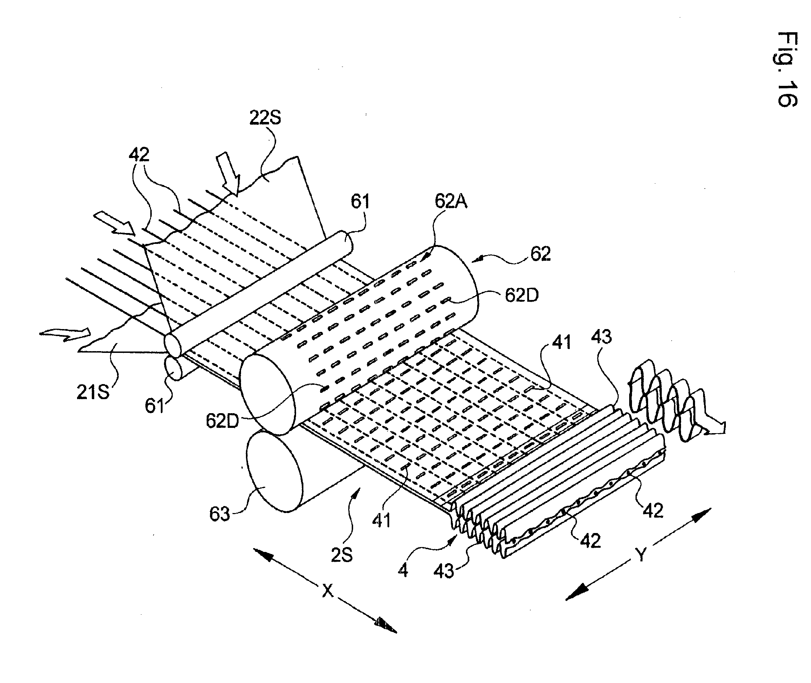

The outer cover 2 has an outer sheet 21, an inner sheet 22, and elastic members 23, 24, 25, and 26 disposed between the sheets 21 and 22. The outer cover 2 has a below-waist gather G1 and a waist gather G2 formed of an composite stretch panel 4 (see Fig. 16) in its below-waist portion D and waist portion F, respectively, of each of the front section A and the rear section B.

In more detail, as illustrated in Figs. 1 through 4, the disposable diaper 1 of the present embodiment includes a substantially oblong absorbent body 3 and an outer cover 2 joined to the absorbent body 3. The absorbent body includes a liquid permeable topsheet 32, a liquid impermeable or water repellent backsheet 33, and a liquid retentive absorbent core 34 interposed between the sheets 32 and 33. The outer cover 2 is joined to the side of the backsheet side 33 (i.e., the garment facing side) of the absorbent body 3. The absorbent body 3 is located to straddle the front section A and the rear section B of the outer cover 2. The longitudinal ends of the absorbent body 3 are longitudinally inward of the longitudinal ends of the outer cover 2. The absorbent body 3 is joined to the inner sheet 22 of the outer cover 2 in a joint region 15 formed by, for example, an adhesive, heat sealing, or ultrasonic sealing.

The outer cover 2 has a longitudinally middle portion thereof narrowed to have the shape of a sandglass and defines the outline of the diaper. The outer cover 2 is sectioned into a front section A designed to be applied to the stomach side of a wearer, a rear section B designed to be applied to the rear side of a wearer, and a crotch section C located between the front section A and the rear section B. The front section A and the rear section B correspond to the longitudinally opposite portions of the outer cover 2, and the crotch section C corresponds to the longitudinally middle portion of the outer cover 2.

The front section A and the rear section B of the outer cover 2 are connected to each other along their both side edges, forming a pair of side seals 11. So connected, the outer cover 2 forms the shape of a pair of pants with a waist opening 12 and a pair of leg openings 13. The connection of the side edges is achieved by, for example, heat sealing, high frequency sealing, or ultrasonic sealing.

The topsheet 32, the backsheet 33, and the absorbent core 34 are each rectangular and united into an oblong absorbent body 3. The topsheet 32, the backsheet 33, and the absorbent core 34 are of conventional types commonly used in this type of diapers. For example, the absorbent core 34 may be an aggregate made up of absorbent polymer particles and a fibrous material and wrapped in tissue (not shown).

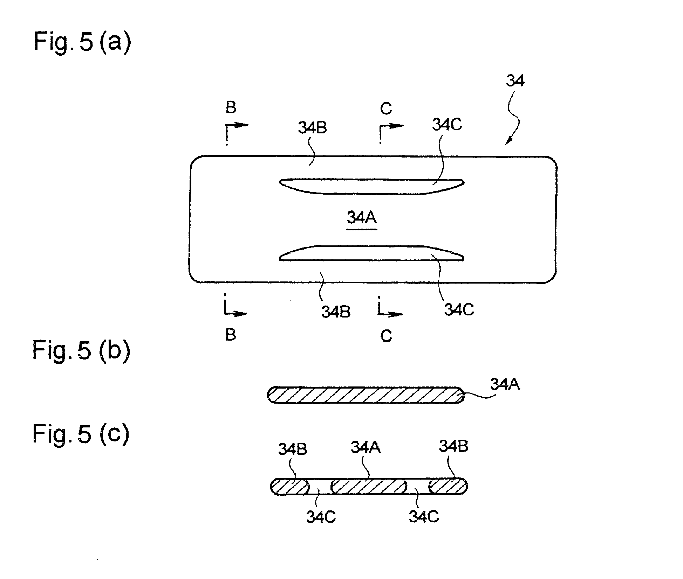

As illustrated in Figs. 5(a) through 5(c), the absorbent core 34 used in the present embodiment includes a sandglass-shaped central absorbent member 34A and a pair of side absorbent members 34B on both sides of the central absorbent member 34A in a symmetrical configuration. The central absorbent member 34A is separate from the side absorbent members 34B in at least the longitudinally middle portion thereof. One longitudinal end portion and the other longitudinal end portion of each side absorbent member 34B are contiguous to one longitudinal end portion and the other longitudinal end portion, respectively, of the central absorbent member 34A. Accordingly, a closed gap 34C is formed between the central absorbent member 34A and each of side absorbent members 34B.

One longitudinal end portion, the other longitudinal end portion, and a longitudinally middle portion of the absorbent core 34 approximately correspond to one-third of the length of the absorbent core 34.
The side edges of the absorbent core 34 rise easily because of the gaps 34C. When the absorbent core 34 is pressed laterally inwardly, the overall width of the absorbent core 34 reduces because of the gaps 34C, whereby lateral contraction of the outer cover 2 is hardly impeded.

The plan-view shape of the absorbent core 34 is not limited to that shown in Figs. 5(a) to 5(c). While not shown, the side absorbent members 34B may be continuous to only one longitudinal end portion of the central absorbent member 34A, or side absorbent members 34B and the central absorbent member 34A may be discontinuous (separate), or the absorbent core 34 may have no gaps 34C.

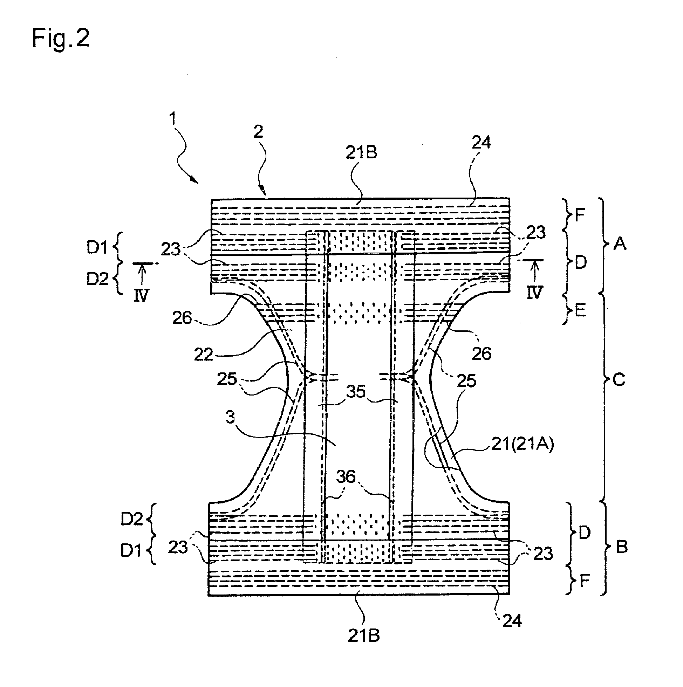

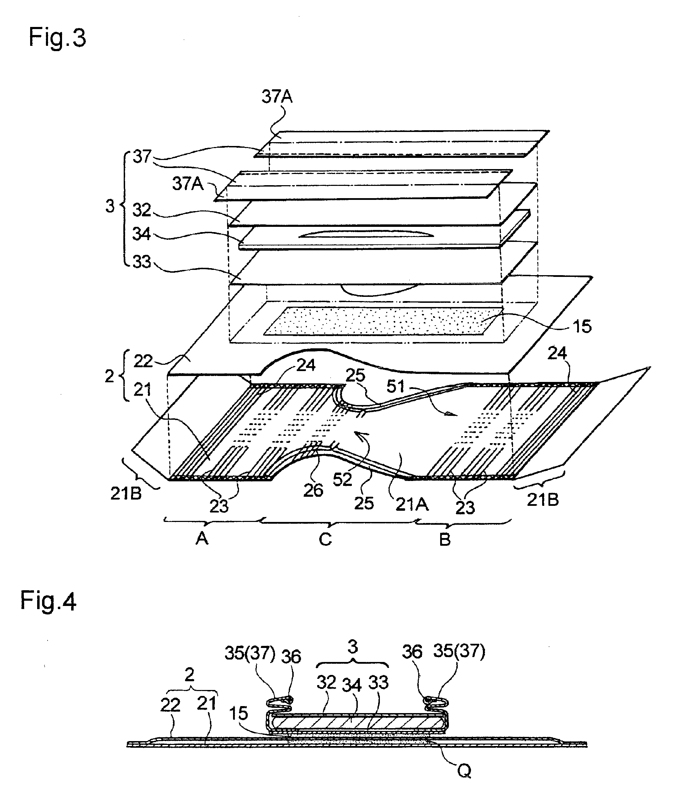

A side cuff 35 formed of a liquid resistant or water repellent and air permeable material is provided along both sides of the absorbent body 3 as illustrated in Figs. 2 to 4. A cuff elastic member 36 is disposed in its stretched state along near the free edge of each side cuff 35. When the disposable diaper 1 is put on a wearer, the cuff elastic members 36 contract to raise the side cuffs 35, thereby to impede the lateral flow of body fluids.
The side cuff 35 is formed of a cuff-forming sheet 37. As illustrated in Figs. 3 and 4, the cuff-forming sheet 37 extends laterally outward from the absorbent body 3 to a prescribed width to provide an extension 37A, which, in an assembled state, wraps around beneath the absorbent core 3 and is secured between the absorbent core 3 and the skin facing side of the backsheet 33.

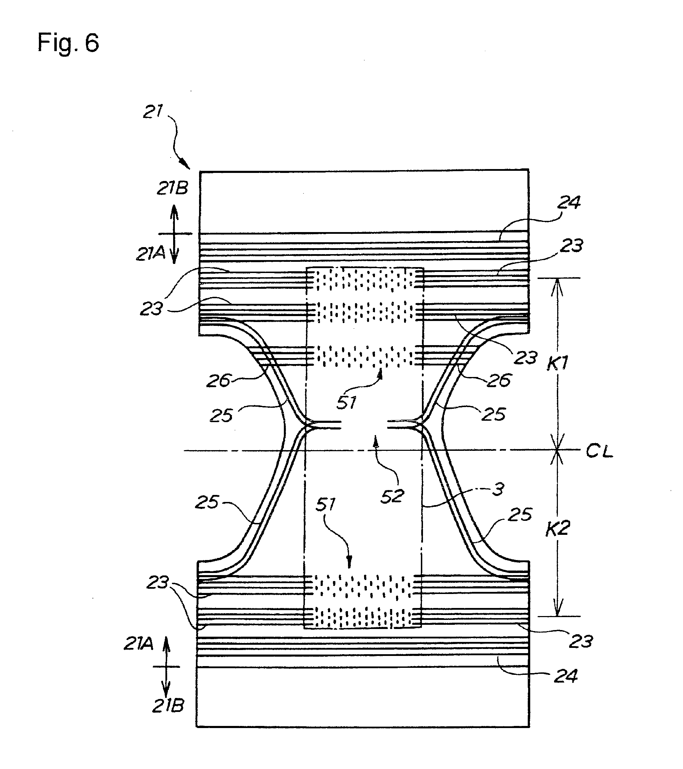

As illustrated in Figs. 2, 3, and 6, the outer cover 2 is composed of an outer sheet 21, an inner sheet 22, and a plurality of elastic members 23, 24, 25, and 26 arranged between the sheets 21 and 22. Fig. 6 is a plan of the outer cover 2 in its flat-out, uncontracted state, seen from the inner side of the outer sheet 21, with the inner sheet 22 imaginarily taken away. The outer sheet 21 provides the exterior side of the diaper. The inner sheet 22 is disposed on the inner side of the outer sheet 21.

As illustrated in Figs. 2, 3, and 6, the outer sheet 21 extends longitudinally outwardly from the portions where the waist elastic members 24 are held between the outer sheet 21 and the inner sheet 22 to provide extensions 21B extending outward from the inner sheet 22. The extensions 21B are folded back over the side of the absorbent body 3.
The region of the outer sheet 21 other than the opposite extensions 21B will be referred to as an essential region 21A. The inner sheet 22 and the essential region 21A of the outer sheet 21 are equal in shape.

The folded extensions 21 B of the outer sheet 21 cover the skin facing side of the longitudinal end portions of the absorbent body 3. The part of each folded extension 21B that overlaps the longitudinal end portion of the absorbent body 3 is bonded to that portion of the absorbent body 3 over the whole width of that portion with an adhesive so that the longitudinal end portion of the absorbent body 3 is secured to the outer cover 2.
The folded extensions 21B prevent the front and rear end portions of the absorbent body 3 from coming into direct contact with the wearer's skin and prevent leakage of absorbent polymer particles of the absorbent core 34 from the front and rear ends of the absorbent body 3.

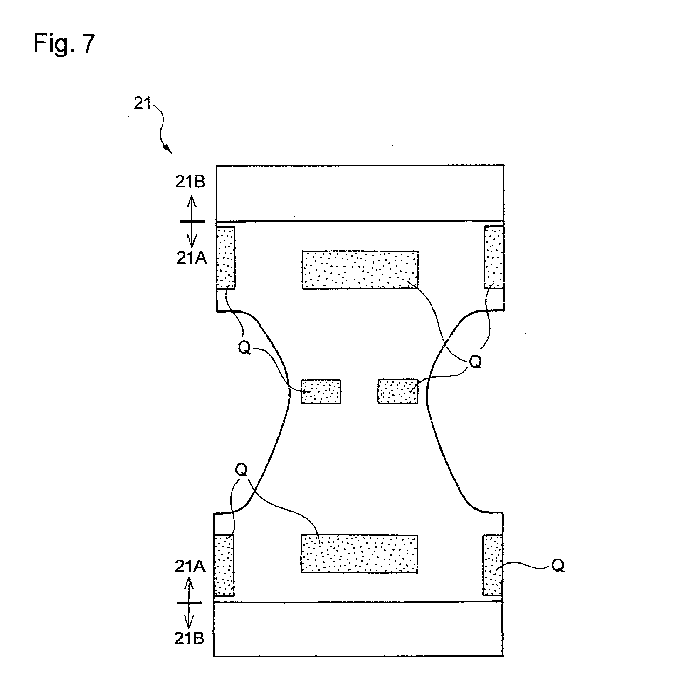

The outer sheet 21 has an adhesive Q (such as a hot melt adhesive) applied to its inner side (the side facing the inner sheet 22) in a pattern depicted in Fig. 7. As illustrated in Fig. 7, the adhesive Q is applied to almost the entire area of regions corresponding to the side seals 11, the regions corresponding to elasticization functionless regions 51 (described *infra*) of the below-waist elastic members 23, and the region corresponding to a leg elastic member cut region 52 (described *infra*).
The adhesive Q is not applied to almost the entire area of the regions corresponding to the composite stretch panels 4 (described later in detail) and almost the entire area of the region corresponding to the crotch section C.

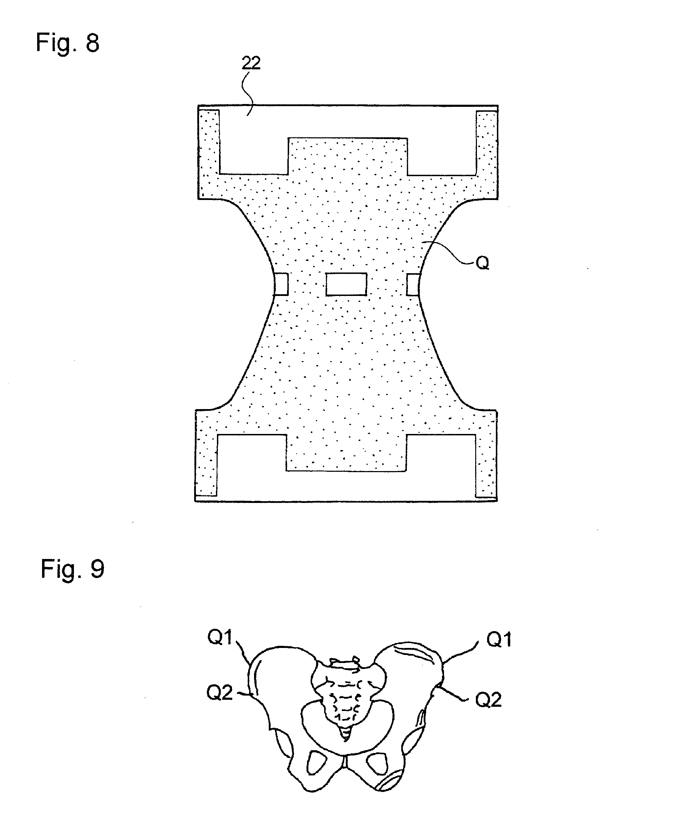

The inner sheet 22 has an adhesive Q (such as a hot melt adhesive) applied to its side facing the outer sheet 21 in a pattern depicted in Fig. 8. As illustrated in Fig. 8, the adhesive Q is applied to almost the entire area of other than the regions corresponding to the composite stretch panels 4.
The adhesive Q is not applied to almost the entire area of the regions corresponding to the composite stretch panels 4 as is similar to the inner side of the outer sheet 21.

It is understood that the application pattern of the adhesive Q on the outer sheet-facing side of the inner sheet 22 includes the application pattern of the adhesive Q on the inner side of the outer sheet 21. Therefore, on joining the outer sheet 21 and the inner sheet 22, the adhesive Q that has been applied to the outer sheet-facing side of the inner sheet 22 adheres to the inner side of the outer sheet 21. This amounts to applying the adhesive Q to the inner side of the outer sheet 21 in the same pattern on the outer sheet-facing side of the inner sheet 22.

In the disposable diaper 1 of the present embodiment, as shown in Figs. 3 and 4, the outer sheet 21 and the inner sheet 22 are bonded together at the side seals 11 in the front section A and the rear section B by heat sealing, high frequency sealing, ultrasonic sealing, or like means.

The outer cover 2 gathers in its waist portions F in the front section A and the rear section B to make the respective waist gathers G2. As used herein, the term "waist portion F" refers to the region from the circumferential edge of the waist opening 12 to a position 30 mm below the circumferential edge. In the case where the vertical position of the circumferential edge of the front side waist opening 12 and that of the rear side waist opening 12 are different (not shown), the part of the waist portion that sticks over the edge of the other waist portion is ignored in deciding the regions of the waist portions F.

Going into the details of the waist portion F, the waist portion F in each of the front section A and the rear section B has waist elastic members 24 arranged along the circumferential edge of the waist opening 12. The outer sheet 21, the inner sheet 22, and the waist elastic members 24 form a composite stretch panel 4 (the details of which will be described later). The composite stretch panel 4 gathers between the pair of side seals 11, that is, a waist gather G2 is formed over the whole circumference of the waist portion F.

The outer cover 2 also gathers along the pair of leg openings 13 to form respective leg gathers G3. In detail, leg elastic members 25 are provided along the circumferential edge of each leg opening 13 curving in the crotch section C. The leg elastic members 25 are fixed in their stretched state between the outer sheet 21 and the inner sheet 22 via the adhesive Q, whereby the leg gather G3 is formed along each leg opening 13.

In the present embodiment, in relation to the method of production described later, the leg elastic members 25 provided along the whole circumference of each leg opening 13 are composed of leg elastic members 25 provided along half of the circumferential edge of the leg opening 13 in the front section A and different leg elastic members 25 provided along the other half of the circumferential edge of the leg opening 13 in the rear section B. It is possible, however, which depends on the method of production, to continuously dispose leg elastic members 25 along the whole circumference of each leg opening 13.

The outer cover 2 has a below-waist portion D in each of the front section A and the rear section B. The below-waist portion D has a composite stretch panel 4 (the details of which will be described later) that gathers to form a below-waist gather G1. As used herein, the term "below-waist portion" refers to a portion from the lower border of the waist portion F to the upper border of the leg openings 13. In detail, the below-waist portion D has a plurality of below-waist elastic members 23 running in the lateral direction of the outer cover 2. The below-waist elastic members 23 extend between near the side edge of the absorbent member 3 and the side seal 11. The below-waist elastic members 23 are not disposed between positions near the opposite side edges of the absorbent member 3.

The composite stretch panels 4 are each composed mainly of the outer sheet 21, the inner sheet 22, and the below-waist elastic members 23. The composite stretch panel 4 gathers between each side seal 11 and near each side edge of the absorbent member 3 to form a pair of laterally spaced below-waist gathers G1. The below-waist gather G1 is not formed between near the opposite side edges of the absorbent member 3. The term "near the side edge of the absorbent body 3" as used herein is intended to mean 30 mm or less inboard and 20 mm or less outboard of each side edge of the absorbent member 3.

The below-waist portion D is divided into an upper below-waist subportion D1 and a lower below-waist subportion D2. The upper below-waist subportion D1 is preferably in the region of the diaper 1 which, while worn by a wearer, is applied to a wearer's body part between the left and right iliac crests and the left and right anterior superior iliac spines. That part of a wearer's body will sometimes be referred to as the iliac region. The anatomical terms "iliac crest" and "anterior superior iliac spine" refer to the sites indicated by the reference numerals Q1 and Q2, respectively, in Fig. 9.

In order to prevent a pull-on diaper, particularly when worn by an infant, from sliding down, it has been considered effective to increase the constrictive pressure of the elastic members disposed in the waist opening portion thus bringing the pull-on diaper into close contact with a wearer's body.

In contrast, it has been found more effective in preventing sliding down of a pull-on diaper during use to increase the constrictive pressure of the portion corresponding to the wearer's iliac region than to increase the constrictive pressure of the waist opening portion. The reason is as follows. Because a diaper wearer, especially an infant has a protruding abdomen as a physical characteristic, an increased constrictive pressure of the waist of a pull-on diaper applied around the periphery of the protruding belly gradually makes the waist of the diaper to constrict, thereby causing the diaper to droop until it fits a less protruding part of the body.

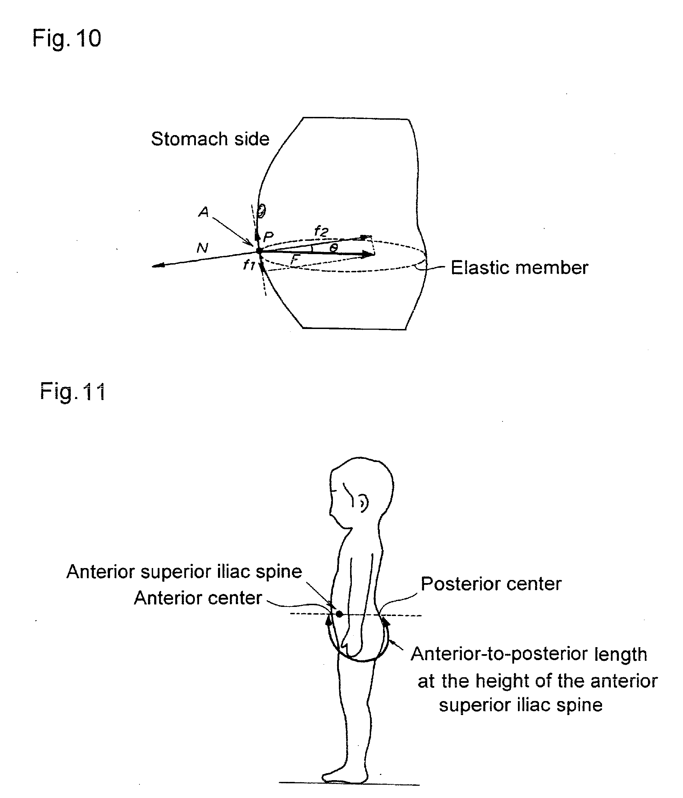

Fig. 10 is an illustration of an infant's abdomen comparing an infant's body to a circular cone. In Fig. 10, $\theta$ is an angle between a normal to the tangent at a waist point A and a horizon directing to the center of the body; F represents a constrictive force of an elastic member; P represents a frictional force attributed to the constrictive force F; f1 represents a sliding force attributed to the constrictive force F; and f2 represents a normal force. $f1 = F\sin\theta$, and $P = \nu N = \nu f2 = \nu F\cos\theta$ (where $\nu$ represents a coefficient of friction). Accordingly, a downward sliding force Z at point A is expressed in equation form as:

$$Z = f1 - P = F\sin\theta - \nu F\cos\theta = F(\sin\theta - \nu\cos\theta)$$

It is understood from the equation above that a larger constrictive force (F) creates a larger sliding force when the waist portion of a diaper is in a state ready to slide down.

There is a certain distance between the iliac crest and the anterior superior iliac spine of a wearer, i.e., the width of the iliac region. The diaper 1 is effectively kept from moving down by applying the upper below-waist subportion D1 of the diaper 1 to a body part within that width of the iliac region. From this point of view, the upper below-waist subportion D1 of the diaper 1 according to the present embodiment preferably has a width, measured in the diaper length direction, of 12 to 35 mm. With that width being 20 to 35 mm, more desirably 25 to 30 mm, the diaper 1 will be kept in place more effectively, and the appearance of the diaper 1 while worn and the ease of diapering with the diaper 1 will be further improved.

In order for the upper below-waist subportion D1 to be applied to the wearer's body part between the iliac crest and the anterior superior iliac spine (i.e., the iliac region), the relation between the size of the diaper 1 and the physical size of a wearer is of importance. Considering, for instance, infants, primarily targeted wearers, the upper below-waist subportion D1 can successfully be applied to the iliac region of a wearer when the distance K1 (see Fig. 6) from the widthwise middle of the upper below-waist subportion D1 (i.e., the middle of the upper below-waist subportion D1 along the diaper length direction) of the front section A to the lateral centerline CL of the diaper 1 is 180 to 230 mm as measured in the flat-out state of the diaper 1, and also when the distance K2 (see Fig. 6) from the widthwise middle of the upper below-waist subportion D1 (i.e., the middle of the upper below-waist subportion D1 along the diaper length direction) of the rear section B to the lateral centerline CL of the diaper 1 is 180 to 230 mm as measured in the flat-out state of the diaper 1.

The recited K1 and K2 values were decided as a result of body measurement of about 350 infants for whom pull-on type diapers are primarily designed. These values will be described more specifically by referring to Fig. 11. The midpoint between, and at the height of, the left and right anterior superior iliac spines in the anterior view of an infant is designated "anterior center". The midpoint similarly defined but in the posterior view of the infant is designated "posterior center". The length from the anterior center via the crotch to the posterior center is designated "anterior-to-posterior length". The sum of the anterior-to-posterior length and an allowance for the thickness of the diaper materials and the like is divided by two to give the above-recited K1 and K2 values. In order to apply the upper below-waist subportion D1 to the iliac region of a wearer more successfully, the distances K1 and K2 are more preferably 185 to 220 mm, even more preferably 195 to 215 mm. In designing diapers for adults, the upper below-waist subportion D1 will be applied to the iliac region of an adult wearer more successfully by adjusting the K1 and K2 values preferably to a range of 300 to 350 mm, more preferably 305 to 335 mm.

As used herein, the term "lateral centerline CL" of the diaper 1 refers to the transverse straight line passing through the lengthwise midpoint of the diaper 1 in the flat-out state in the case where the position of the circumferential edge of the waist opening 12 in the front stomach side is in substantial alignment with the position of the circumferential edge of the waist opening 12 in the rear side.
Unlike that, when, while not shown, the position of the circumferential edge of the waist opening 12 in the front side is out of alignment with that on the rear side, the part of the waist portion that sticks over the edge of the other waist portion is ignored in deciding the lateral centerline CL.

The diaper 1 according to the present embodiment is secured to the wearer's body by the constrictive force that primarily rests on the below-waist elastic members 23 disposed in the upper below-waist subportion D1. In other words, the constrictive force of the elastic members 24 disposed in the waist portion F is not a primary means for securing the diaper 1 to the wearer's body unlike the conventional pull-on diapers. On the contrary, an increased constrictive force of the waist portion F helps the diaper 1 to move down.

The lower below-waist subportion D2 is preferably applied to the body part below the iliac region, namely the lower abdominal region of a wearer. The width (measured in the longitudinal direction of the diaper 1) of the lower below-waist subportion D2 is preferably 40 to 70 mm, more preferably 45 to 65 mm.

A frontal crotch portion E will then be described. The term "frontal crotch portion E" as used herein denotes the most frontal of quarters into which the crotch section C is divided in the diaper longitudinal direction. The frontal crotch portion E has a plurality of frontal crotch elastic members 26 arranged therein over the whole width of the outer cover 2. The frontal crotch elastic members 26 are fixed in their stretched state between the outer sheet 21 and the inner sheet 22 via the adhesive Q, whereby the frontal crotch portion E gathers to form a frontal crotch gather G4.

The outer cover 2 has elasticization functionless regions (hereinafter simply referred to as "functionless region(s)") 51 within an area where it faces the absorbent body 3, in which regions the contractibility of the elastic members is lost. In the present embodiment, the functionless regions 51 are formed by making the below-waist elastic member 23 and the frontal crotch elastic members 26 lose their contractibility. As will be described in more detail with respect to the method of production, the functionless regions 51 are the results of providing continuous length below-waist elastic members 23S and continuous length frontal crotch elastic members 26S over the whole width of the outer cover 2 and depriving part of the continuous elastic members 23S and 26S of contractibility. Depriving the continuous elastic members of ability of contraction may be achieved by means of, for example, a number of cutting projections or blades that segmentalize the elastic members or a number of embossing pins that thermally harden the elastic members.

By providing the functionless regions 51, the continuous below-waist elastic members 23S and the continuous frontal crotch elastic members 26S provided over the whole width of the outer cover 2 discontinuously gather to form a laterally spaced pair of below-waist gathers G1 and a laterally spaced pair of frontal crotch gathers G4.

The functionless region 51 located between the laterally spaced pair of below-waist gathers G1 is the result of depriving the continuous below-waist elastic members 23 S bonded between the outer sheet 21 and the inner sheet 22 of their contractibility, whereas the functionless region 51 located between the laterally spaced frontal crotch gathers G4 is the result of depriving the continuous frontal crotch elastic members 26S bonded between the outer sheet 21 and the inner sheet 22 of their contractibility. Thus, the absorbent body 3 is prevented from bunching up and exhibits a good appearance and absorbing performance.

There is formed a leg elastic member cut region 52 in the laterally middle part of the crotch section C. As will be described in detail with reference to the method of production, the leg elastic member cut region 52 is the result of curvingly providing continuous leg elastic members 25S over the whole width of the outer cover 2 and cutting the continuous elastic members 25S at the midpoint of the width of the crotch section C. The leg elastic member cut region 52 is formed by means of, for example, a number of cutting projections or blades that cut the elastic members. By providing the leg elastic member cut region 52, the continuous leg elastic members 25S provided curvingly over the whole width of the outer cover 2 form a laterally spaced pair of leg elastic members 25 in the crotch section C.

In the present embodiment, the outer cover 2 does not have a composite stretch panel 4 in the crotch section C. Thus, urine discharged in the frontal part of the absorbent body 3 is allowed to smoothly move rearward, and the absorbing performance of the diaper 1 is markedly improved. The leg elastic members 25 and the frontal crotch elastic members 26 are preferably strings or tapes of stretch materials, such as natural rubber, polyurethane resins, foamed urethane resins, and hot-melt stretch materials.

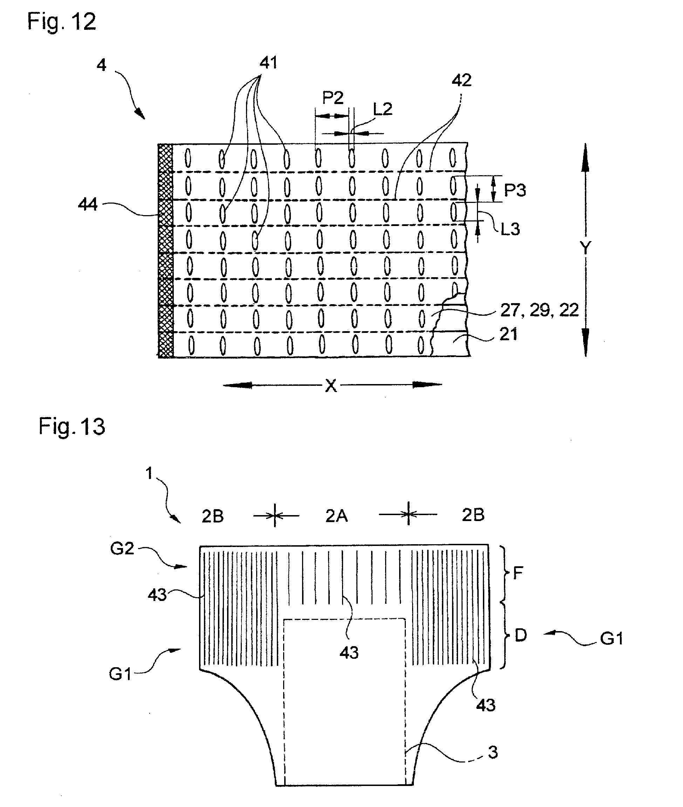

The composite stretch panel 4 that forms the below-waist gathers G1 and the waist gather G2 will be described in detail. The composite stretch panel 4 has the following configurational characteristics (1) to (3) as illustrated in Figs. 12 and 16.

(1) The outer sheet 21 and the inner sheet 22 are bonded together at bonds 41 that are arranged discretely in both the stretch direction (direction X) of the composite stretch panel 4 and a direction perpendicular to the stretch direction (direction Y).

(2) Elastic members 42 are disposed at positions not to pass through the bonds 41 and are not fixed to the outer sheet 21 and the inner sheet 22 at other than their opposite ends.

(3) Each of the outer sheet 21 and the inner sheet 22 gathers to form folds 43 each extending continuously over a plurality of the elastic members 42.

Each of the folds 43 of the waist gathers G2 formed of the respective composite stretch panels 4 adjacent to the longitudinal ends of the absorbent body 3 and those of the below-waist gathers G1 formed of the respective composite stretch panels 4 is continuous over the whole width of the respective composite stretch panels 4 in the direction perpendicular to the stretch direction of the composite stretch panels 4. With the folds 43 being continuous over the whole width of the respective composite stretch panels 4 in the direction perpendicular to the stretch direction of the composite stretch panels 4, the diaper 1 is excellent in appearance, cushioning properties, and breathability in its lateral side regions (i.e., the regions between the side edges of the absorbent body 3 and the side seals 11, except the side seals 11 *per se*).

As previously described, in the present embodiment, the composite stretch panel 4 forming the waist gather G2 adjacent to each longitudinal end of the absorbent body 3 and the composite stretch panel 4 forming the below-waist gather G1 are present between near each side edge of the absorbent body 3 and the side seals 11 but the composite stretch panel 4 forming the below-waist gather G1 is absent between positions near the opposite side edges of the absorbent member 3. To put it another way, there is no below-waist gathers G1 but the waist gather G2 between near the side edges of the absorbent body 3.

In the present embodiment, the composite stretch panel 4 forming the below-waist gather G1 is composed mainly of the outer sheet 21, the inner sheet 22, and the below-waist elastic members 23, while the composite stretch panel 4 forming the waist gather G2 is composed mainly of the outer sheet 21, the inner sheet 22, and the waist elastic members 24. Although the designation of the elastic members differs according to the designation of the gathers, all the elastic members constructing the composite stretch panels 4 will hereinafter be inclusively referred to as "elastic members 42".

It is preferred that all the elastic members 42 be disposed not to pass through any of the bonds 41. However, a manufacturing error can cause a few elastic members 42 to pass through a few bonds 41. Even if such is the case, the effects of the present invention are achieved sufficiently. When the elastic members 42 do not pass through 70% or more by number of the bonds 41, the elastic members 42 are regarded to be disposed not to pass through the bonds 41.

As illustrated in Figs. 12 and 16, the outer sheet 21 and the inner sheet 22 are bonded together at the bonds 41 that are discretely arranged in both the stretch direction (direction X) of the composite stretch panel 4 and the direction perpendicular to that direction (direction Y). The bonds 41 are preferably formed by heat fusion.

The plurality of elastic members 42 extend in parallel to each other in the longitudinal direction of the composite stretch panel 4. In an embodiment like the present one where elastic members 42 are parallel to each other, the stretch direction of the composite stretch panel 4 is the same as the stretch direction of the elastic members 42. In an embodiment where the elastic members 42 are not parallel to each other, the stretch direction of the composite stretch panel 4 is a direction perpendicular to the direction of a fold 43 (hereinafter described) running across a plurality of the elastic members 42.

Fig. 12 illustrates a pattern of arranging the bonds 41. The bonds 41 are arranged to line up in both the stretch direction of the composite stretch panel (direction X) and the direction perpendicular thereto (direction Y). The bonds 41 lining in the perpendicular direction are placed between every adjacent elastic members 42. When the composite stretch panel 4 is in a relaxed state (with no external force applied), the elastic members 42 contract, whereby each of the outer sheet 21 and the inner sheet 22 gathers to form a plurality of folds (gathers) 43 continuously running across over the elastic members 42. In the present embodiment, a fold 43 forms between two bonds 41 adjacent in the stretch direction of the composite stretch panel 4 (direction X).

In order to assure formation of folds 43 continuously extending over a plurality of the elastic members 42, it is preferred that the bonds 41 be arranged at a pitch P2 (see Fig. 12) of 1 to 20 mm, more preferably 3 to 10 mm, in the stretch direction (direction X) of the composite stretch panel 4 with the composite stretch panel 4 being in the stretched state; that the individual bonds 41 have a length L2 (see Fig. 12) of 0.1 to 5 mm, more preferably 0.2 to 1.5 mm in the same direction and in the same state; and that the ratio of the pitch P2 to the length L2 (P2/L2) be in the range of from 1.1 to 200, more preferably from 2 to 50.

It is preferred that the pitch P3 (see Fig. 12) of the bonds 41 in the direction (direction Y) perpendicular to the stretch direction of the composite stretch panel 4 be 1 to 40 mm, more preferably 2 to 15 mm; that the length L3 (see Fig. 12) of the individual bonds 41 be 0.5 to 20 mm, more preferably 1 to 10 mm in direction Y; and that the ratio of the pitch P3 to the length L3 (P3/L3) be in a range of from 1.05 to 80, more preferably of from 1.05 to 15.

It is preferred that the outer sheet 21 and the inner sheet 22 not be joined to each other in/at other than bonds 41. Each of the elastic members 42 is arranged in the composite stretch panel 4 avoiding the bonds 41 of the outer sheet 21 and the inner sheet 22, and is fixed neither to the outer sheet 21 nor the inner sheet 22 at other than both ends thereof. The elastic members 42 are fixedly held only at their opposite ends in between the outer sheet 21 and the inner sheet 22.

Each elastic member 42 is held between the outer sheet 21 and the inner sheet 22 with only their ends fixed between the two sheets 21 and 22 at the respective end portions 44 of the composite stretch panel 4 (only one of the end portions 44 is depicted in Fig. 12). The positions of the end portions 44 of the composite stretch panel 4 differ depending on the gather to be formed. In the embodiment of Fig. 1, the end portions 44 of the composite stretch panel 4 forming the below-waist gather G1 are at near one of the side edges of the absorbent body 3 and one of the side seals 11. Those of the composite stretch panel 4 forming the waist gather G2 are at the opposite side seals 11. The two end portions 44 of the composite stretch panel 4 may be stretchable.

The folds 43 of the outer sheet 21 and the inner sheet 23 protrude on the respective sides of the composite stretch panel 4. Each fold 43 has a curved surface whose cross-section is arc-shaped at the top. The curved surface continues in direction Y perpendicular to the stretch direction of the composite stretch panel 4. In the present embodiment, every fold 43 forms between every two bonds 41 that are adjacent in the stretch direction (direction X) of the composite stretch panel 4. Every fold 43 continuously runs in direction Y perpendicular to the stretch direction of the composite stretch panel 4 without being discontinued at positions where it crosses the elastic members 42.

The composite stretch panel 4 of the first embodiment has a neat, attractive appearance since a number of the folds 43 created by the contraction of the elastic members 42 individually continue in the direction (direction Y) perpendicular to the stretch direction of the composite stretch panel 4.

The structure of the composite stretch panel 4 of the present embodiment does not cause an increase in stiffness and allows the folds 43 to be deformed with more freedom under external force. These effects are the results of the following structural characteristics. (1) The individual elastic members 42 run between the valleys of adjacent folds 43 formed of the outer sheet 21 and the valleys of adjacent folds 43 formed of the inner sheet 22 and are not bonded to either the outer sheet 21 or the inner sheet 22 at these valleys as well as the other parts of the sheets. (2) The outer sheet 21 and the inner sheet 22 are not continuously joined to each other in either the stretch direction (direction X) of the composite stretch panel 4 or the perpendicular direction (direction Y).

Additionally, the folds 43 having a curved surface at the top provide the outer sheet 21 and the inner sheet 22 with an increased volume in the thickness direction and feel very soft and pleasant to the touch.

Since the elastic members 42 in the composite stretch panel 4 are not fixed to either the outer sheet 21 or the inner sheet 22, the amount of a hot melt adhesive to be used can be minimized or reduced to zero. Particularly when the outer sheet 21 and the inner sheet 22 are joined together by fusion bonding as in the present embodiment, the effect in reducing the adhesive is outstanding. With a reduced amount a hot melt adhesive, breathability and moisture permeability will not be impaired.

Materials making the composite stretch panel 4 of the first embodiment are described below.

To create folds 43 pleasant to the eyes and to the touch, the outer sheet 21 and the inner sheet 22 are preferably formed of a single sheet or a laminate sheet of air-through nonwoven, heat-rolled nonwoven, hydroentangled nonwoven, spunbonded nonwoven, and meltblown nonwoven fabrics, and the like. Air-through nonwoven fabric is particularly preferred for its soft touch.

Physical properties desirable for materials making the outer sheet 21 will be described. Some of the properties are also desirable for materials making the inner sheet 22. The direction of orientation of the fibers constituting the outer sheet 21 is preferably substantially coincide with the stretch direction (direction X) of the composite stretch panel 4. It is also preferred that the direction of orientation of the fibers constituting the inner sheet 22 be substantially coincide with the stretch direction (direction X) of the composite stretch panel 4. In other words, it is preferred that the fiber orientation direction of the outer sheet 21 and that of the inner sheet 22 be substantially coincide with each other.

For the fibers of the sheets to have substantially the same orientation direction as the stretch direction of the composite stretch panel 4 means that a nonwoven fabric web as a sheet material has its fibers oriented in the web moving direction (MD of the web) in the course of the production of the nonwoven fabric web and that the web moving direction (MD of the web) becomes the stretch direction of the composite stretch panel 4. The tensile strength of the web (sheet) in the fiber orientation direction (MD of the web) is by far higher than that in the direction perpendicular to the fiber orientation direction (CD of the web).

Since the fiber orientation direction and the stretch direction of the composite stretch panel 4 substantially coincide with each other, the bonds 41 of the composite stretch panel 4 exhibit strong bonding strength. Although the bonds 41, by which the outer sheet 21 and the inner sheet 22 are discretely bonded to each other, are subject to stronger force in the stretch direction of the composite stretch panel 4, the bonds 41 have strong bonding strength by virtue of the high tensile strength of the sheets. Thus, the bonds 41 are less likely to break.

Because of the high bonding strength of the bonds 41, the folds 43 form easily and keep their shape while worn, thereby retaining the cushioning properties.

It is only necessary that the fibers of the outer sheet 21 or the inner sheet 22 be oriented such that the vector connecting the leading end and the trailing end of the individual fibers is coincide with the stretch direction of the composite stretch panel 4. It is acceptable that some of the individual fibers are vertically directed (in the thickness direction of the outer sheet 21 or the inner sheet 22) due to twisting or entanglement between the leading and trailing ends as long as the constituent fibers are generally oriented in the stretch direction of the composite stretch panel 4.

The degree of orientation of the fibers of the outer sheet 21 is preferably 1.2 or higher, more preferably 1.3 or higher. Although there is no particular upper limit of the degree of orientation as long as the sheet exhibits satisfactory strength properties including tensile strength and tear strength, but the degree of orientation is preferably 5 or less. This is because it is necessary to secure tensile strength of the sheet in both the MD (the fiber orientation direction) and the CD (the direction perpendicular to the fiber orientation direction) so as to provide convenience of use, i.e., to secure tear resistance during diaper change and ease of tear along the side seals 11 when the diaper is disposed of. The degree of orientation of the constituent fibers is measured with a microwave molecular orientation analyzer MOA-2001A manufactured by Kanzaki Co., Ltd. Measurement is made on three points of a rectangular sample measuring 100 mm in lateral direction and 100 mm in longitudinal direction to obtain an average. Where a sample is smaller than that size, two or more samples adjoining to each other without overlap are used.

When the fibers making up the outer sheet 21 have a high degree of orientation, the orientation direction of the fibers is easily seen from the outside as a pattern of the sheet. Since the fiber orientation direction of the composite stretch panel 4 is generally the same as the stretch direction of the elastic members 42, the elastic members 42 are thus integrated with the pattern of the sheet and become less visible or less distinct through the outer sheet 21. To put it another way, the outer sheet 21 has improved hiding properties.

A different in bending stiffness between the fiber orientation direction and the direction perpendicular thereto is taken advantage of in facilitating folding back the extensions 21B of the outer sheet 21. That is, the sheet is used with its fiber orientation direction (the MD of the sheet) parallel to the stretch direction of the waist gather G2 so as to secure high bonding strength of the composite stretch panel 4 and also with its CD (i.e., the direction perpendicular to the fiber orientation direction) parallel to the direction perpendicular to the stretch direction of the waist gather G2. By so designing, the sheet has a smaller bending stiffness in the direction perpendicular to the fiber orientation direction, which makes it easier to fold back the extensions 21B as in Fig. 3.

Thus, the waist gather G2 proximate to the edge of the waist opening 12 has the tops of the folds 43 thereof aligned to provide a neat edge along the waist opening 12. The tops of the folds 43 proximate to the edge of the waist opening 12 provide not only an attractive appearance but feel soft and pleasant in direct contact with the wearer's body, causing no discomfort while the diaper is worn. Furthermore, the difference of bending stiffness of the outer sheet 21 between the stretch direction of the composite stretch panel 4 (direction X) and the direction perpendicular thereto (direction Y), as will be discussed later, is easily produced.

In order for the folds 43 to have a nicely curved cross-section as desired, the bending stiffness particularly of the outer sheet 21 is of importance. In order to form less collapsible and neat folds 43 in good balance with the stretchability of the elastic members 42, the sheet needs to have a bending stiffness in a certain range. While pliability of the sheet as a whole and the bending stiffness are conflicting requirements, they are achieved relatively easily when the sheet has at least a certain degree of orientation. When the sheet has a certain bending stiffness in its fiber orientation direction, the composite stretch panel 4 easily forms the fold 43.

Folds 43 with a round top gently contact the skin, and high folds 43 have excellent cushioning properties. Folds 43 having the facing side walls obtusely rising from the bonds 41 have a large skin contact area and good resilient cushioning properties. Folds 43 with a round top and resiliency are excellent in hand and cushioning properties. Since the gathered portions make contact with the skin on the top of the folds, the contact area with the skin is reduced, the burden on the skin is reduced, and frictional irritation to the skin is reduced.

One of the features of the disposable pull-on diaper of the invention is good breathability. The elastic members and the supporting outer and inner sheets 21 and 22 are not united with an adhesive and form regular folds 43. The folds 43 stretch and retract in accordance with the wearer's body contour. The larger the circumference of the wearer, the lower the folds 43. The smaller the circumference of the wearer, the higher the folds 43. Since the elastic members 42 have no bonding joints with the outer and inner sheets 21 and 22, the folds 43 freely change in shape and size.

This means that the folds 43 change in shape and size in accordance with the stretch and contraction of a disposable pull-on diaper while it is worn by a wearer. It is known that the circumference of infant's waist and abdomen changes by about 5 cm according to whether the wearer is sitting or standing. The folds 43 stretch and contract in conformity to the changing circumference. This creates bellows effect, causing a flow of air and accelerating ventilation of the inside of the diaper, which is a great feature of the invention. The shape of the folds 43 is important to enhance the bellows effect. It is desirable that the folds 43 have as large a volume as possible so as to make a great change with the changing circumference. The change of the folds 43 produces the bellows effect (an airflow, the effect of controlling humidity inside the diaper). When the folds 43 have a round shape and maintain a large cross-sectional area, they have a large volume over their length. As a result, a large amount of air, particularly damp air is allowed to stay in the folds 43, leading to enhanced exhalation of humidity through the bellows effect.

The outer sheet 21 preferably has a bending stiffness of 0.008 to 0.020 cN·cm$^2$/cm, more preferably 0.009 to 0.015 cN·cm$^2$/cm, in the stretch direction (direction X) of the composite stretch panel 4.
Bending stiffness (B value) is measured using an automatic pure bending tester (KES FB2-AUTO-A, from Kato Tech Co., Ltd.) in accordance with the method taught in Sueo Kawabata, HUAI HYOUKANO HYOUJYUNKATO KAISEKI, 2nd ed., published by The Textile Machinery Society of Japan (Jul., 10, 1980).

In greater detail, bending stiffness is measured as follows.

Method of measuring bending stiffness:

A specimen measuring 20 cm by 20 cm is prepared. The specimen is placed on the mount and clamped at a chuck spacing of 1 cm and bend in an arc of curvature radius (K) ranging from -2.5 to +2.5 cm$^{-1}$ at a constant rate of bending deformation of 0.50 cm$^{-1}$/sec to determine hysteresis of one-cycle deformation. According to the description of *HUAI HYOUKANO HYOUJYUNKATO KAISEKI supra*, the B value is calculated from the slope of the bending moment curve between K=0.5 and K=1.5 and from that between K=-0.5 and K=-1.5. However, kinking can occur in these ranges of curvature radius in the evaluation of nonwovens, which makes the results inaccurate. Then, the bending stiffness is calculated from the slopes from 0 to the maximum bending moment value and from 0 to the minimum bending moment value. The measurements are made in the MD and the CD to obtain the bending stiffness in the respective directions.

If the bending stiffness of the outer sheet 21 in direction X is too low, the folds 43 of the composite stretch panel 4 tends to have sharp tops, the individual folds 43 tend to fail to continue straight in the diaper longitudinal direction, and the folds 43 are liable to be collapsed when the composite stretch panel 4 is stretched in its stretch direction (direction X). If the bending stiffness in direction X is too high, the folds 43 also tend to have sharp and hard tops, resulting in reduced hand.

The outer sheet 21 preferably has a bending stiffness of 0.001 to 0.010 cN·cm$^2$/cm, more preferably 0.0025 to 0.005 cN·cm$^2$/cm, in the direction perpendicular to the stretch direction of the composite stretch panel 4 (direction Y). The bending stiffness of the outer sheet 21 in direction Y is preferably smaller than that in direction X by at least 0.003 cN·cm$^2$/cm, more preferably by at least 0.005 cN·cm$^2$/cm, even more preferably by at least 0.007 cN·cm$^2$/cm.

If the bending stiffness of the outer sheet 21 in direction Y is too low, the outer sheet 21 has insufficient stiffness for performing the step of folding the extensions 21B in a stable manner, and the folded edges (the longitudinal ends of the outer cover 2) have an instable shape.
If, on the other hand, the bending stiffness of the outer sheet 21 in direction Y is too high, the extensions 21B are hard to fold nicely, and the folded edges are hard.
The outer sheet 21 whose bending stiffness in direction Y falls within the above recited preferred range readily forms a folding line extending in direction X, which helps the extensions 21B to be folded back. With the moderate stiffness, the folded extensions 21B are wrinkle-resistant.

The thickness of the outer sheet 21 is preferably 0.25 to 0.7 mm, more preferably 0.3 to 0.6 mm. The outer sheet 21 with too small a thickness tends to form sharp folds 43, tending to fail to form round folds 43. If the outer sheet 21 is too thick, the folds 43 tend to have a sharp top, failing to depict a neat curve.

The outer sheet 21 preferably has a density of 0.07 g/cm$^3$ or less, more preferably 0.06 g/cm$^3$ or less, even more preferably 0.02 to 0.06 g/cm$^3$. The outer sheet 21 with too high a density forms folds 43 with reduced softness and pliability.

It is preferred that the outer sheet 21 not be subjected to substantial embossing. The term "embossing" as used herein is intended to exclude the process of forming the bonds 41 of the composite stretch panel 4. If the outer sheet 21 is an embossed sheet, the folds 43 of the resulting composite stretch panel 4 are liable to be nonuniform, the outer sheet 21 has reduced pliability, and it is difficult to make a difference in bending stiffness between direction Y and direction X.

The composite stretch panel 4 forms the folds 43 as a result of deformation of the outer sheet 21 and the inner sheet 22 with the contraction of the elastic members 42. That is, the stiffness of the outer sheet 21 and that of the inner sheet 22 are one of factors decisive of the fold forming properties and the cushioning properties of the composite stretch panel 4. The stiffness of each sheet can be represented by its buckling strength. It is preferred for the sheets used to make the composite stretch panel 4 to have a buckling strength of 100 cN or less, more preferably 70 cN or less. The buckling strength of the sheets is measured with a Tensilon universal tensile tester from Orientec Co., Ltd. in compressive mode as follows.

Buckling strength testing method (CD):

Test pieces measuring 150 mm in the machine direction (MD) and 30 mm in the cross-machine direction (CD) are cut out of a sample and each looped into a 45 mm diameter cylinder. The overlapped ends were stapled together at their upper and lower parts to make specimens. Measurement is taken with the Tensilon universal tensile tester in compressive mode under measuring conditions of a rate of compression of 10 mm/min and a measuring distance of 20 mm in a measuring environment of 20°C and 65% RH. The maximum strength of the specimen when compressed by 20 mm is read for every specimen to obtain an average, which is taken as the buckling strength of the sample.

As stated previously, nonwoven fabric is preferably used as the outer sheet 21 and the inner sheet 22. The nonwoven fabric preferably has a basis weight of 5 to 50 g/m$^2$, more preferably 18 to 30 g/m$^2$. Nonwoven fabric having a basis weight in that range preferably has a buckling strength of 50 cN or lower, more preferably 30 cN or lower, in the CD and of 70 cN or lower, more preferably 50 cN or lower, in the MD.

The material making the outer sheet 21 and the inner sheet 22 (e.g., fibers of nonwoven fabric) preferably comprises a heat fusible resin, such as polyethylene and polypropylene. The fibers constituting nonwoven fabrics may be sheath/core conjugate fibers having a heat fusible resin as the sheath.

The outer sheet 21 and the inner sheet may be of the same or different materials.

While the outer sheet 21 and the inner sheet 22 have different shapes in the embodiment illustrated in Fig. 1, they may have the same shape. In that case, it is preferred to use holding sheets separately from the outer sheet 21 and the inner sheet 22 in place of the extensions 21B of the outer sheet 21 used in the present embodiment. The holding sheet is applied to cover the skin facing side of each longitudinal end portion of the absorbent body 3. The part of the holding sheet that overlaps the longitudinal end portion of the absorbent body 3 is preferably bonded via an adhesive applied over the whole width of the absorbent body 3, thereby to secure the longitudinal end portion of the absorbent body 3 to the outer cover 2.

While in the present embodiment the outer sheet 21 and the inner sheet 22 are separate sheets, a single sheet may be folded into two facing panels, one of which serves as the outer sheet 21, and the other as the inner sheet 22.

Any known elastic materials of various kinds that have been used in absorbent articles, such as disposable diapers and sanitary napkins, can be used with no particular limitation as a material of the elastic members 42 of the composite stretch panel 4. Examples include synthetic rubbers, such as styrene-butadiene, butadiene, isoprene, and neoprene, natural rubber, EVA, stretch polyolefins, and polyurethane. Forms of the elastic members 42 include a thread with a rectangular, square, circular or polygonal section, tape, or multifilamentous yarn.

Other factors decisive of fold forming properties of the composite stretch panel 4 are a stretch ratio and a tensile load of the elastic member 42. The elastic member 42 is required to have a prescribed stretch ratio and tensile load in order to create folds with a protruding cross-section. The stretch ratio is calculated from formula: stretch ration (%) = (B/A) x 100, where A (mm) is the length before stretch, and B (mm) is the length after stretch.

The elastic members 42 are disposed between the outer sheet 21 and the inner sheet 22 in a stretched state preferably at a stretch ratio of 120% to 1100%, more preferably 150% to 500%. Upon the elastic members 42 retracting, the excess of the non-contractible outer sheet 21 and inner sheet 22 protrudes to the respective sides opposite to their facing sides to form folds 43.

The height of the folds 43, which is an important factor concerning fold forming properties and cushioning properties, can be designed freely by selecting the pattern of arrangement and the pitch of the bonds 41, the materials of the outer sheet 21 and the inner sheet 22, and the material of the elastic members. The height of the folds 43 is preferably about 1 to 15 mm per side. High folds 43 are formed when the elastic members 42 contract to bring adjacent bonds 41 very close to form protruding folds 43, thereby to provide a composite stretch panel 4 that feels soft and voluminous. To form folds 43 with a height h on one side, the distance between adjacent bonds 41 must be at least 2h. When the distance is just 2h, the elastic members 42 must contract such that the adjacent bonds 41 almost come into contact with each other.

Among preferred materials of the elastic members 42 is natural (or synthetic) rubber. An elastic member 42 of natural (or synthetic) rubber may be a low modulus elastic member having a thickness of 0.05 to 1.5 mm and a width of 0.2 to 5 mm. A monofilament of such a material preferably has a load at 200% elongation of 1 to 70 gf, more preferably 1 to 40 gf, even more preferably 1 to 30 gf. A composite stretch panel 4 that softly stretches and retracts to form neat and nice folds 43 can be obtained by disposing a plurality of monofilaments of the above-described low modulus elastic member at a high stretch ratio preferably of 200% or higher, more preferably of 300% or higher.

Also included in preferred elastic members is Spandex fiber (elastic polyurethane fiber). A monofilamentous Spandex fiber having a fineness preferably of 10 to 3360 denier, more preferably of 70 to 1120 denier, is used. "Denier" is a unit of thickness of a yarn. A yarn weighing one gram per 9,000 meters is one denier. A plurality of such Spandex fibers are used at a stretch ratio preferably of 130% to 600%.

The extension percentage of the waist portion F will then be described. The waist portion F of the outer cover 2 has a smaller extension percentage in a laterally middle part 2A of the outer cover 2 than in laterally outer parts 2B of the outer cover 2. Such a difference in extension percentage is obtained easily by forming the waist gather G2 of the composite stretch panel 4.

The laterally middle part 2A (see Fig. 13) of the outer cover 2 is a portion facing the absorbent body 3 in the flat-out state. In the case where the longitudinal ends of the absorbent body 3 are inward of the longitudinal ends of the outer cover 2 as in the embodiment shown in Fig. 1, also included in the laterally middle part 2A of the outer cover 2 is the part defined by each longitudinal end of the absorbent body 3, each longitudinal end of the outer cover 2, and two straight lines extended from the two corners at each longitudinal end of the absorbent body 3 to the longitudinal end of the outer cover 2. The laterally outer part 2B of the outer cover 2 is a part located on either lateral side of the laterally middle part 2A as illustrated in Fig. 13.

The extension percentage of the waist portion F is measured as follows. Method of measuring extension percentage of waist portion F:

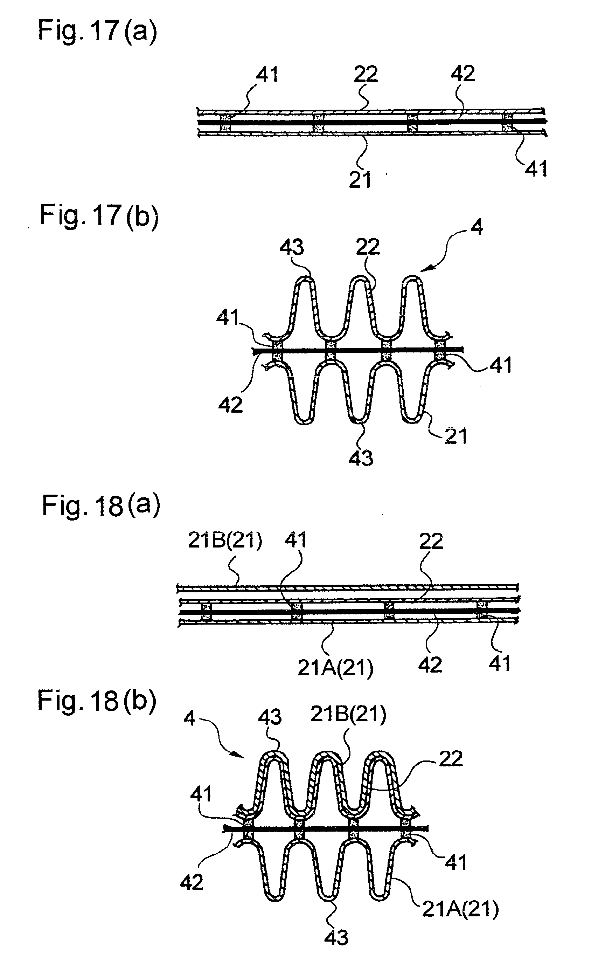

(1) The waist portion F in the laterally middle part 2A of a diaper 1 in the relaxed state is marked with a line having a length of 20 mm in the lateral direction.
(2) The laterally middle part 2A was held at the ends of the mark and extended laterally until the folds 43 of the gathering composite stretch panel 4 are flattened out (see Figs. 17(a) and 18(a)).
(3) The length L4 of the mark as extended (the distance between the ends of the mark) is measured.
(4) (L4/20)x100% is taken as the extension percentage of the waist portion F in the laterally middle part 2A.
(5) The extension percentage of the waist portion F in the laterally outer part 2B can be measured in the same manner as described above, except for making the marks in the laterally outer part 2B.

The extension percentage of the waist portion F in the laterally middle part 2A is preferably 130% to 500%, more preferably 150% to 400%. The extension percentage of the waist portion F in the laterally outer parts 2B is preferably 150% to 600%, more preferably 200% to 500%. The difference in extension percentage of the waist portion F between the laterally outer parts 2B and the laterally middle part 2A is preferably 30% to 200%, more preferably 50% to 150%.

The tensile load of the waist portion F will then be described. It is preferred that, when the annular waist portions F is extended to a circumference of 300 mm, it has a load (tensile load) of at least 20 gf, more preferably at least 25 gf. The reason why the total circumference of the annular waist portion F at which the load is measured is specifically set at 300 mm is that diapers for infants have a circumference of about 300 mm in the annular waist portion F in their relaxed or unworn state. A diaper having a load of less than 20 gf when extended to a circumference of 300 mm in its annular waist portion F has a poor appearance when worn because the contraction of the annular waist portion F is insufficient to make a good contact to the body.

It is preferred that, when the annular waist portion F is extended to a circumference of 500 mm, it has a load of 200 gf or less, more preferably 180 gf or less. The reason why the circumference of the annular waist portion F at which the load is measured is specifically set at 500 mm is that the circumference at the waist of infants of larger size, i.e., the largest circumference at the annular waist portion F of the diaper while worn is about 500 mm. An annular waist portion F having a load of more than 200 gf when extended to a circumference of 500 mm causes indentations to be left on the wearer's skin and also causes the diaper to slide down while worn.

The tensile load of the annular waist portion F is controlled by the adjustment of the material, thickness, and extension percentage of the elastic members 42 to be used, the pitch and number of the elastic members to be disposed, and the like.

The tensile load of the annular waist portion F is measured as follows. Method of measuring tensile load of annular waist portion F:

(1) The annular waist portion F is cut out of a diaper to prepare a hoop with the side seals 11 intact. The hoop is placed on a horizontal surface in its relaxed state, and the length between the opposite side seals 11 is measured as an initial length.

(2) The hoop is clamped on its side seals between chucks of a Tensilon tensile tester (RTC-1150A, from Orientec Co., Ltd.). The load at this stage is defined as zero.

(3) The hoop is pulled in the opposite directions (the same direction as the diaper lateral direction) at a rate of 300 mm/min until the circumference of the waist hoop reaches 300 mm and 500 mm. The load read at the circumference of 300 mm or 500 mm is taken as the "load when the annular waist portion F is extended to a circumference of 300 mm or 500 mm", respectively. As used herein, the term "circumference" refers to the circumference, specifically the inner circumference, of the hoop with the side seals 11 intact.

Comparing the tensile load between the waist portion F, the upper below-waist subportion D1, and the lower below-waist subportion D2, it is preferred that the upper below-waist subportion D1 have the highest tensile load, the lower below-waist subportion D2 have the second highest tensile load, and the waist portion F have the lowest tensile load. The tensile loads of the upper below-waist subportion D1 and the lower below-waist subportion D2 are controlled by the adjustment of the material, thickness, and extension percentage of the elastic members to be used, the pitch and number of the elastic members to be disposed, and the like. In comparing the tensile load between different portions, samples are cut out of the respective portions with the same width (preferably 30 mm) in the diaper longitudinal direction. By providing such a difference in tensile load between different portions, sliding down of the diaper 1 while worn is effectively prevented, and the diaper 1 snugly fits the wearer's body to effectively prevent leakage without causing discomfort. The tensile loads of the upper below-waist subportion D1 and the lower below-waist subportion D2 are measured in the same manner as for the tensile load of the waist portion F.

The diaper will further be described with respect to the physical properties it has when stretched between the opposite side seals 11 in the form of a complete diaper. When stretched between the side seals 11 in the lateral direction under a tensile load of up to 2400 gf, the diaper preferably has a circumference of at least 660 mm, more preferably at least 680 mm, at its waist opening edge. As used herein, the term "circumference at the waist opening edge stretched between the side seals 11 in the lateral direction up to a tensile load of 2400 gf" is the inner circumference of the waist opening edge measured at the time when the tensile load reaches 2400 gf in stretching the whole diaper held at its opposite side seals 11 gradually in the lateral direction.

The reason why the load for measuring the circumference is set at 2400 gf is that 2400 gf is the maximum load ordinary mothers are able to exert when they widen the diaper in the lateral direction. The waist opening circumference under the load of 2400 gf is related to the ease for a mother to widen (stretch) the diaper.

It is preferred that the energy required to stretch the waist opening edge portion of the pull-on absorbent article be 4 N·m at the most, more preferably 3.5 N·m or less. When the energy required to stretch the waist opening 12 exceeds 4 N.m, it is difficult for a mother to widen the diaper in the lateral direction (a mother does not feel easy to widen). The smaller the energy, the easier widening the diaper laterally.

The energy needed to stretch the diaper between the opposite side seals 11 in the lateral direction until the waist opening 12 increases its circumference to 600 mm is preferably 2 N·m or less, more preferably 1.8 N·m or less. The circumference is specified as 600 mm because the diaper is widen to a circumference of about 600 mm when a mother changes diapers on an infant. Therefore, the energy required to stretch the circumference of the waist opening 12 to 600 mm will be a measure for expressing ease of diapering an infant. The energy of 2 N·m or less makes a mother feel easy to diaper with significance.

The energy required to stretch the waist opening of a pull-on absorbent article is measured as follows. Method of measuring energy required to stretch the waist opening of pull-on absorbent article:

(1) A complete diaper *per se* is used as a sample. The sample is placed on a horizontal surface in its relaxed state, and the length between the opposite side seals 11 is measured as an initial length.

(2) The sample is clamped on its side seals between chucks of a Tensilon tensile tester (RTC-1150A). The load at this stage is defined as zero.

(3) The sample is pulled in the opposite directions (the same direction as the diaper lateral direction) at a rate of 300 mm/min until the load reaches 2400 gf or the circumference reaches 600 mm to obtain an S-S (stress-strain) curve.

(4) The energy required for the stretch of the waist opening 12 is obtained from the area of the S-S curve.

According to the structure of the disposable pull-on diaper of the present embodiment, the below-waist gather G1 and the waist gather G2 are formed of the respective composite stretch panels 4. Since these gathers provide stretchability substantially without using a hot melt adhesive. The diaper therefore has good breathability through both the front section A and the rear section B. The outer sheet 21 and the inner sheet 22 of the outer cover 2 are allowed to show their essential texture and feel soft or non-stiff.

In the composite stretch panel 4 contractibility of the elastic members 42, being little or not hindered, is made full use of. When the stretch characteristics of, for example, the below-waist portion D are graphically represented by plotting the circumference as abscissa and the tensile load as ordinate, the characteristic curve has a gentle slope, indicating high stretchability. The characteristic curve in a stretch mode and that in a retraction mode show a small difference of load, which indicates that a variation in load with a variation in circumference is small. This means that the diaper imposes an equal wearing pressure to an infant with a smaller waist and an infant with a larger waist. As a result, the tendencies of a diaper to slide/slip down on an infant of relatively small size and to leave indentation marks on an infant of relatively large size are eliminated, and there is provided a diaper that fits wearers of broader range of sizes.

Thus, the diaper of the present embodiment gently fits a wearer and hardly slides down during use by virtue of its high contractibility. The diaper shows its full contractibility as a whole, which offers the advantage of compactness. Additionally, the amount of the elastic members 42 may be reduced, which is economically advantageous. The composite stretch panel 4 gathers to form on its interior side folds 43 that are to come into contact with the wearer's body to provide good cushioning properties and gentle fit with little damage to the skin during use. The folds 43 are also formed on the exterior side of the outer cover 2 (the exterior side during use) to make the diaper feel pleasant to the touch.

The folds 43 of the waist gather G2 formed of the composite stretch panel 4 adjoining each longitudinal end of the absorbent body 3 and those of the below-waist gather G1 formed of the composite stretch panels 4 continuously extend in the direction perpendicular to the stretch direction of these composite stretch panels 4, and the stretch direction (direction X) of the composite stretch panels 4 and the orientation direction of the fibers making up the outer sheet 21 are substantially coincide with each other. As a result, the outer cover 2 has a good appearance and also exhibits high hiding properties to make the elastic members (including the waist elastic members 24 and the below-waist elastic members 23) less visible particularly from the side of the outer sheet 21.

The waist portion F of the outer cover 2 has a lower extension percentage in its laterally middle part 2A than in both the laterally outer parts 2B. As a result, the waist gather G2 gently fits the infant's waist with less likelihood of leaving indentations on the skin.

Because of the above described difference in extension percentage within the waist portion F, the waist gather G2 has folds at a lower density (density of folds = the number of folds per unit length in the lateral direction of the outer cover 2) in the laterally middle part 2A than in the laterally outer parts 2A. Usually, the size of the laterally middle part 2A and that of the laterally outer parts 2B in the lateral direction of the outer cover 2 are not so different. The number of the folds formed in the laterally middle part 2A is therefore usually smaller than that in the laterally outer parts 2B. This is advantageous in that the laterally middle part 2A is easy to print.

Since the load measured when the annular waist portion F is extended to a circumference of 300 mm is 20 gf or more, the diaper has a nice appearance. Since the load measured when the annular waist portion F is extended to a circumference of 500 mm is 200 gf or less, the annular waist portion F gently hugs the wearer's abdomen that largely changes in circumference and prevents leaving marks on the skin. Both improvement of appearance and reduction of load are thus achieved.

Since the circumference of the annular waist portion F extended between the pair of side seals 11 under a load of up to 2400 gf is 660 mm or more, it is easy for a mother to stretch the diaper 1 in the lateral direction and to put the diaper 1 on a wearer.

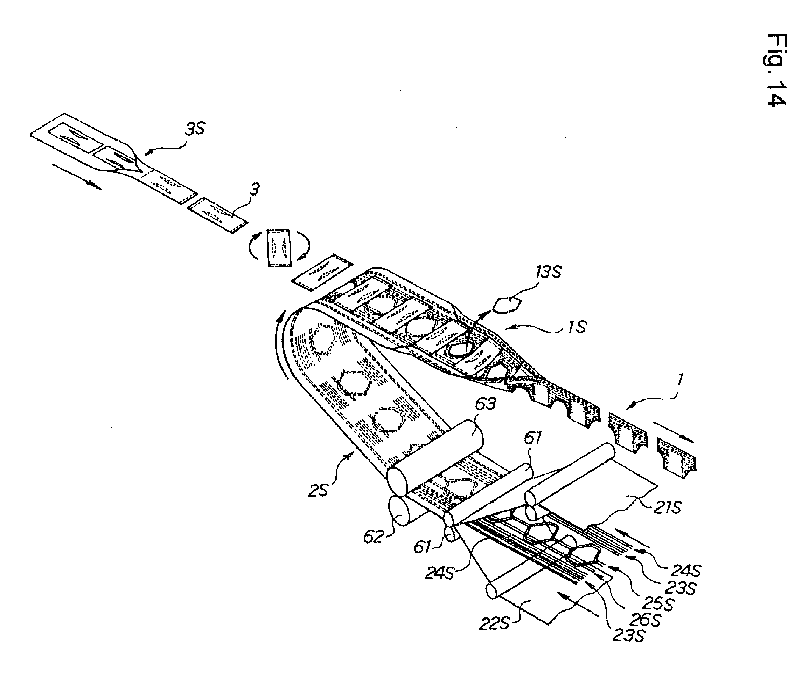

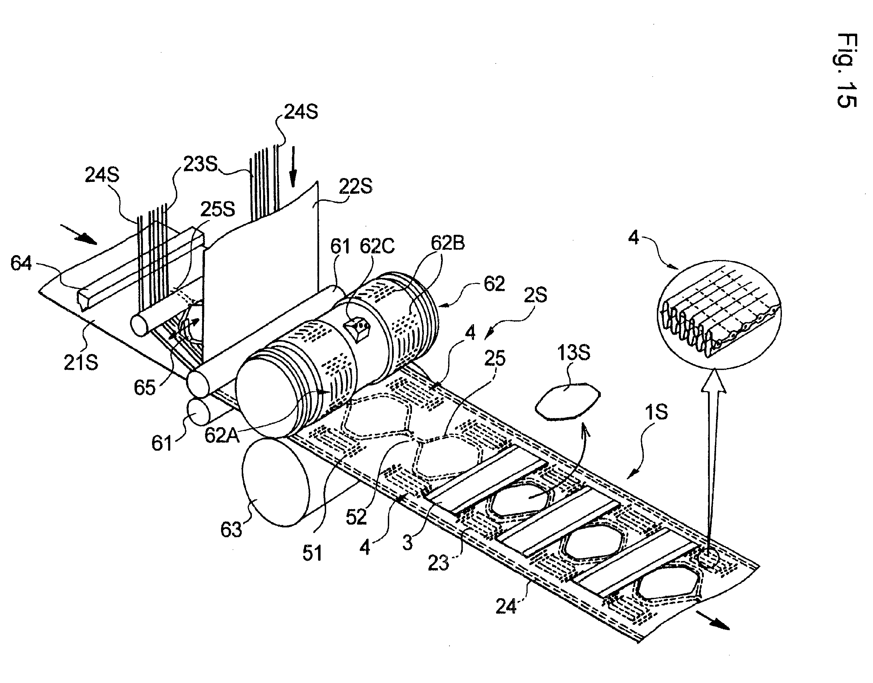

A method for continuously producing the disposable pull-on diaper of Fig. 1 will then be described with reference to the accompanying drawing. Fig. 14 is a perspective showing the present method. Fig. 15 is a view focused on the step of forming composite stretch panels, functionless regions, and leg elastic member cut regions in the present method. Fig. 16 is a conceptual diagram of processing focused on the step of making a composite stretch panel in the present method.

As illustrated in Figs. 14 through 16, the present method uses the same roller 62 to carry out the step of joining the outer sheet 21 and the inner sheet 22 at the bonds 41 to form composite stretch panels 4 and the step of depriving elastic members 23 and 26 of contractibility to form functionless regions 51. The present method is a method of producing a disposable pull-on diaper including the steps of joining a continuous outer sheet 21S (a continuous web of an outer sheet 21) and a continuous inner sheet 22S (a continuous web of an inner sheet 22) while disposing continuous elastic members 23S, 24S, 25S, and 26S (elastic members 23, 24, 25, and 26, respectively, of continuous length) therebetween to form a continuous outer cover 2S (an outer cover 2 of continuous length), bonding absorbent bodies 3 to the side of the continuous inner sheet 22S of the continuous outer cover 2S each via a joint region 15 (see Fig. 4) to form a continuous diaper 1S (a diaper 1 of continuous form), and cutting the continuous diaper 1S into individual diapers 1.

The present method will be described in detail. A continuous outer sheet 21S is used as a material of an outer sheet 21, and a continuous inner sheet 22S is used as a material of an inner sheet 22.

The continuous elastic members 23S, 24S, 25S, and 26S (elastic members 23, 24, 25, and 26 of continuous length) are introduced into between the continuous outer sheet 21S and the continuous inner sheet 22S in their stretched state.

The continuous outer sheet 21S has its fibers oriented in the longitudinal direction thereof. The continuous inner sheet 22S similarly has its fibers oriented in the longitudinal direction thereof. The present method adopts what we call a transverse feed system to produce the diapers 1. Accordingly, in the outer cover 2 of the resulting diaper (product), the stretch direction (lateral direction) of the composite stretch panels 4 and the orientation direction of the fibers of the outer sheet 21 and the inner sheet 22 are substantially the same.

The continuous frontal crotch elastic members 26S (frontal crotch elastic members 26 of continuous length) are omitted from Fig. 15. Before the continuous outer sheet 21S and the continuous inner sheet 22S are joined, a hot melt adhesive Q is applied to their facing sides in respective predetermined patterns illustrated in Figs. 7 and 8 by means of, e.g., an adhesive applicator 64 (see Fig. 15). The continuous leg elastic members 25S are introduced into between the continuous outer sheet 21S and the continuous inner sheet 22S while being rocked across the moving sheets by rocking guides 65 (see Fig. 15).

The two continuous sheets 21S and 22S with the continuous elastic members 23S, 24S, 25S, and 26S disposed therebetween are pressed between a pair of nip rollers 61. There is thus obtained a continuous outer cover 2S having the two continuous sheets 21S and 22S and the continuous elastic members 23S, 24S, 25S, and 26S sandwiched therebetween. The below-waist elastic members 23 are not bonded with the hot melt adhesive Q in the parts between near each side edge of the absorbent body 3 and each side seal 11. Similarly, the waist elastic members 24 are not bonded with the hot melt adhesive Q in the parts between the opposite side seals 11.

In this stage, the continuous outer cover 2S has formed therein none of composite stretch panels 4, functionless regions 51, and a leg elastic member cut region 52.

The continuous outer cover 2S not having formed composite stretch panels 4 is then introduced between a composite stretch panel-forming roller 62 and an anvil roller 63. The composite stretch panel-forming roller 62 has, on its peripheral surface, composite stretch panel-forming parts 62A that function to form composite stretch panels 4, functionless region-forming parts 62B that function to form functionless regions 51, and a leg elastic member cutting region-forming part 62C that functions to form a leg elastic member cut region 52.

The anvil roller 63 is a roller with a smooth surface and backs up the composite stretch panel-forming roller 62.

The composite stretch panel-forming parts 62A of the composite stretch panel-forming roller 62 have a number of embossing pins 62D (see Fig. 16) corresponding to bonds 41 of composite stretch panels 4. The continuous outer cover 2S not having formed composite stretch panels 4 is provided with bonds 41 in the regions designed to become composite stretch panels 4 by the composite stretch panel-forming parts 62A. The embossing pins 62D may be heat embossing pins or ultrasonic embossing pins. Note that Fig. 16 is a conceptual diagram showing only extracted elements relating to the formation of the composite stretch panel 4. Therefore, the composite stretch panel-forming roller 62 is depicted as having only the composite stretch panel-forming part 62A on its periphery, and the continuous outer cover 2S is depicted as having only the composite stretch panel 4.

As illustrated in Figs. 17(a), 17(b), 18(a), and 18(b), there are thus formed composite stretch panels 4 in prescribed regions of the continuous outer cover 2S, of which the elastic members 42 (which correspond, in the present method, to the below-waist elastic members 23 and the waist elastic members 24) are to contract to make below-waist gathers G1 and waist gathers G2 with folds 43. In the region where an extension 21B of the outer sheet 21 is present, the extension 21B also gathers to form folds as illustrated in Fig. 18(b).

Figs. 17(a) and 17(b) are each a cross-section of a part of the composite stretch panel 4 where the extension 21B is absent, and Figs. 18(a) and 18(b) are each a cross-section of a part of the composite stretch panel 4 where the extension 21B is present.

The functionless region-forming parts 62B of the composite stretch panel-forming roller 62 are each a part that deprives the elastic members of contractibility to form functionless regions 51 in the continuous outer cover 2S. The functionless region-forming parts 62B are composed of, e.g., a number of projections or cutting blades that segmentalize the elastic members or a number of heating embossing pins that fuse and harden the elastic members.

The leg elastic member cut region-forming part 62C of the composite stretch panel-forming roller 62 is a part by which the continuous leg elastic members 25S are cut in the laterally middle part of the crotch section C to form a leg elastic member cut region 52. The leg elastic member cut region-forming part 62C is composed of a number of projections or cutting blades. The continuous leg elastic members 25S are cut by the leg elastic member cut region-forming part 62C to provide leg elastic members 25 spaced apart in the diaper lateral direction in the laterally middle part of the crotch section C.

Introducing the continuous outer cover 2S having none of composite stretch panels 4, functionless regions 51, and leg elastic member cut regions 52 between the composite stretch panel-forming roller 62 having the above described configuration and the anvil roller 63 results in the formation of the continuous outer cover 2S having formed in prescribed portions thereof the composite stretch panels 4, functionless regions 51, and leg elastic member cut regions 52.

The disposable pull-on diaper 1 of the first embodiment is produced in a usual manner for the production of disposable pull-on diapers in a transverse feed system, except for using the continuous outer cover 2S having formed therein the composite stretch panels 4, functionless regions 51, and leg elastic member cut regions 52.

For example, as shown in Fig. 14, a continuous-form absorbent body 3S is cut into individual absorbent bodies 3, which are turned 90° with respect to the running direction and spacedly fixed on the continuous outer cover 2S via a joint region 15 (see Fig. 4). Every absorbent body 3 is fixed to the continuous outer cover 2S while maintaining the continuous outer cover 2S in the stretched state. In other words, the absorbent body 3 is fixed while holding the continuous outer cover 2S not to contract due to the retractability of the composite stretch portions 4, etc.

As illustrated in Fig. 14, both edges of the continuous outer cover 2S are folded over the end portions of the absorbent body 3 and fixed to the end portions of the absorbent body 3, and the continuous outer cover 2S is then folded in two together with the absorbent body 3. Prior to this, an adhesive is applied to both edges of the continuous outer cover 2S that correspond to the extensions 21B, the parts of the continuous inner sheet 22S that are to face the extensions 21B, and the predetermined parts of the absorbent body 3.

As illustrated in Figs. 14 and 15, the unnecessary part 13S corresponding to leg openings is cut out of the continuous outer cover 2S with a rotary cutter, a laser cutter, etc. to make a continuous-form diaper 1S.

The continuous diaper 1S is folded longitudinally (along a lateral direction of the disposable diaper 1) in two. The folded continuous diaper 1S is spacedly sealed to form side seals 11 by heat sealing, ultrasonic sealing, high frequency sealing or like means, and the continuous diaper 1S is cut into individual disposable pull-on diapers 1 of the first embodiment after or simultaneously with the formation of the side seals 11.

Ninety-degree turning the individual absorbent bodies 3 and spacedly securing the absorbent bodies 3 to the continuous outer cover 2S are carried out by, for example, the method taught in JP 4-166150A. A joint region 15 (see Fig. 4) for fixing the absorbent body 3 to the continuous outer cover 2S is provided on either one or both of the absorbent body 3 and the continuous outer cover 2S. Removal of the unnecessary part 13S for making the leg openings may be carried out before fixing the absorbent body 3 to the continuous outer cover 2S.

According to the present method, disposable pull-on diapers having the composite stretch panels 4 and the functionless regions 51 can be produced efficiently because the step of joining the outer sheet 21 and the inner sheet 22 at the bonds 41 to make the composite stretch panels 4 and the step of rendering the elastic members 23 and 26 functionless to form the functionless regions 51 are achieved by using the same roller 62.

The absorbent article of the present invention will further be illustrated with reference to its second embodiment. The description will generally be confined to the differences from the first embodiment. Common parts and elements are identified by the same numerals as in the previously described embodiment and will not be redundantly described. The configuration of the first embodiment applies to the second embodiment unless otherwise specified.

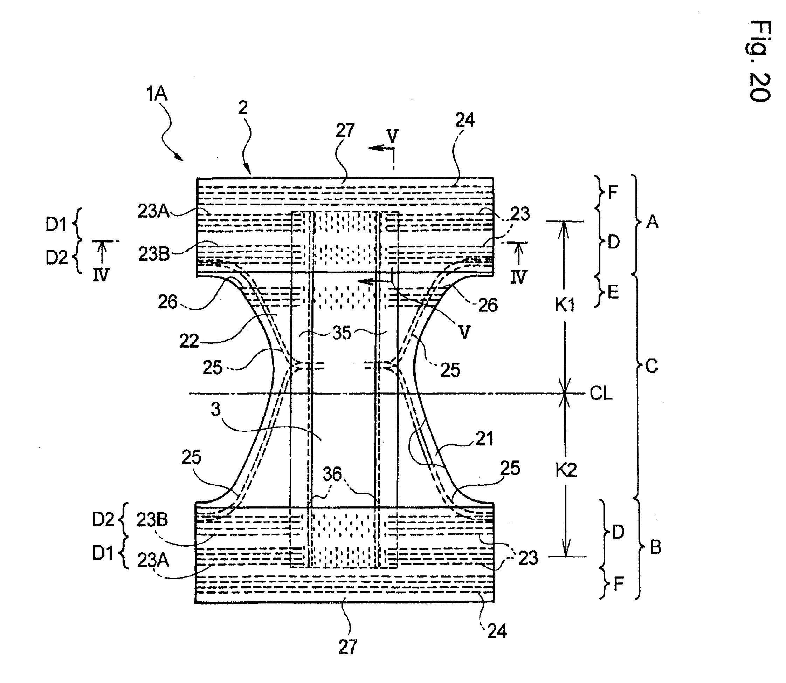

The disposable pull-on diaper 1A according to the second embodiment has an absorbent body 3 including an absorbent core 34 and a waist flap F, a portion outward of each longitudinal end of the absorbent body 3. A perspective view of the diaper 1A of the second embodiment is the same as that of the diaper 1 shown Fig. 1, and a cross-section of the diaper 1A taken along line IV-IV of Fig. 20 is the same as the cross-section of Fig. 4.

Each of the waist flap F of the front section and the waist flap F of the rear section has a skin-contacting surface-defining sheet 27 that defines the skin-contacting surface of the waist flap F and a garment facing surface-defining sheet 21 that defines the garment facing surface of the waist flap F, both being a hydrophobic sheet. Each waist flap F further has a hydrophilic sheet 29 between the skin contacting surface-defining sheet 27 and the garment facing surface-defining sheet 21 and elastic members 24 between the hydrophilic sheet 29 and the garment facing surface-defining sheet 21.

In addition to the absorbent body 3, the disposable pull-on diaper 1A of the second embodiment further has an outer cover 2 bonded to the garment facing side of the absorbent body 3 and holding sheets 27 each covering the skin facing side of each longitudinal end portion of the absorbent body 3.

The outer cover 2 includes an outer sheet 21, an inner sheet 22, and a plurality of elastic members 23 (including 23A and 23B), 24, 25, and 26. The outer sheet 21 provides the exterior side of the diaper. The inner sheet 22 is disposed on the inner side of the outer sheet 21. That is, the aforementioned garment facing surface-defining sheet of the waist flap F is a part of the outer sheet 21.

The holding sheet 27 is integral with the outer sheet 21. That is, the outer sheet 21 is folded back over the skin facing side of the absorbent body 3 to provide the holding sheet 27. That is, the aforementioned skin facing surface-defining sheet of the waist flap F is a part of the holding sheet 27.

In the second embodiment, the skin contacting surface-defining sheet and the garment facing surface-defining sheet are both hydrophobic sheets. Seeing that the skin contacting surface-defining sheet and the garment facing surface-defining sheet are a part of the holding sheet 27 and the outer sheet 21, respectively, and the holding sheet 27 and the outer sheet 21 are integral with each other, the integral sheet is a hydrophobic sheet. The inner sheet 22 is also a hydrophobic sheet.

The term "hydrophobic sheet" as used herein refers to a sheet having a water pressure resistance of 1 to 10 gf/cm$^2$, preferably 1 to 5 gf/cm$^2$, as measured in accordance with the method below. The hydrophobic sheet preferably has a basis weight of 5 to 50 g/m$^2$, more preferably 7 to 30 g/m$^2$. Method of measuring water pressure resistance: A sample sheet is held between vertically connected two cylindrical tubes having a diameter of 35 mm. Physiological saline is poured into the upper tube at a rate of 4 g/min. The time required for the physiological saline to pass through the sample sheet and starts to flow into the lower tube is recorded. The amount (weight) of physiological saline poured is calculated from the flow rate and the time thus recorded. A quotient obtained by dividing the calculated amount of saline (gf) by the cross-sectional area (cm$^2$) of the tube is taken as a water pressure resistance (gf/cm$^2$).

In order for composite stretch panels 4 to form folds 43 (the details of which will be given later) that are attractive in appearance, nice to the touch, and pliable, the hydrophobic sheet forming the holding sheet 27 (including the skin contacting surface-defining sheet), the hydrophobic sheet forming the outer sheet 21 (including the garment facing surface-defining sheet), and the hydrophobic sheet forming the inner sheet 22 are each preferably a single or laminate sheet of through-air nonwoven, heat-rolled nonwoven, hydroentangled nonwoven, spunbonded nonwoven, meltblown nonwoven, and so forth.

The hydrophilic sheet 29 is different from both the outer sheet 21 and the inner sheet 22 and is disposed on the garment facing side of each longitudinal end portion of the absorbent body 3. In detail, the position of the hydrophilic sheet 29 in the waist flap F is between the holding sheet 27 and the inner sheet 22, and that in a below-waist portion D (hereinafter described) is between the garment facing side of the absorbent body 3 and the inner sheet 22.

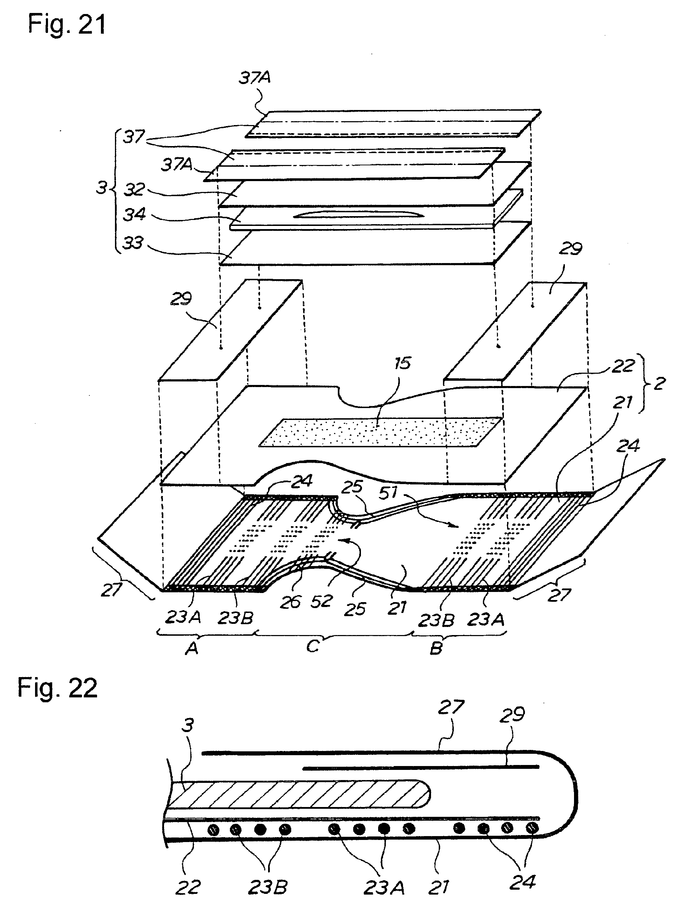

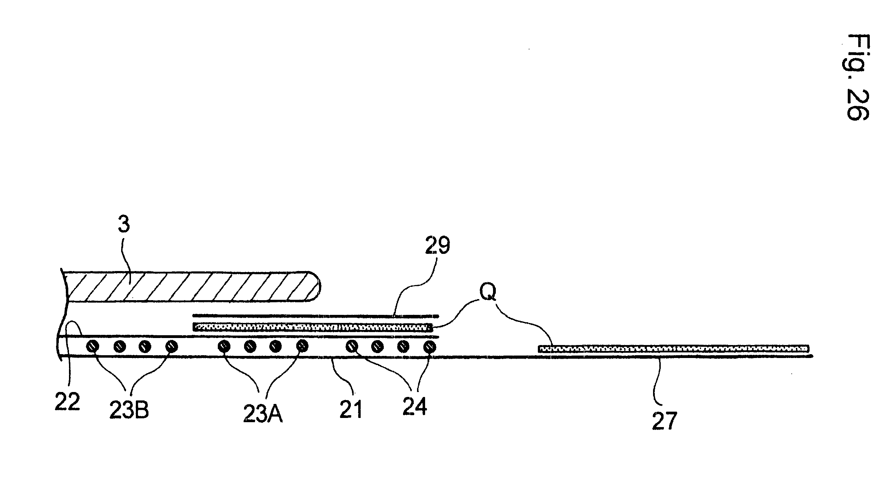

The holding sheet 27 and the hydrophilic sheet 29 directly face each other without any other sheet therebetween. The holding sheet 27 and the hydrophilic sheet 29 are bonded to each other with, e.g., a hot melt adhesive (not shown in Fig. 22 but shown in Fig. 26).

The hydrophilic sheet 29 and the inner sheet 22 directly face each other without any other sheet therebetween. The hydrophilic sheet 29 is bonded on its garment facing side to the inner sheet 22. The means for bonding the hydrophilic sheet 29 and the inner sheet 22 include, though not exclusively, a hot melt adhesive, heat sealing, and ultrasonic sealing. The hydrophilic sheet 29 and the inner sheet 22 may be bonded to each other by making use of the bonds 41 of a composite stretch panel 4, as will be described in detail with respect to the method of production.

The disposable diaper 1A of the second embodiment will be described further. The disposable diaper 1A of the second embodiment includes the absorbent body 3 and the outer cover 2 joined to the backsheet side (garment facing side) of the absorbent body 3.

The outer sheet 21 and the inner sheet 22 that make the outer cover 2 of the diaper 1A have the same shape. The waist elastic members 24 are fixedly held between the longitudinal end portions of the outer sheet 21 and the longitudinal end portions of the inner sheet 22 as illustrated in Figs. 20, 21, and 22. The outer sheet 21 is integral with the holding sheet 27. The holding sheet 27 is an extension extending from each longitudinal end of the outer sheet 21 and being folded back over the skin facing side of the absorbent body 3.

The holding sheet 27 (the folded-over portion of the integral sheet) covers almost the entire area of the skin facing side of a below-waist portion D (the details of which will be described later) including the longitudinal end portion of the absorbent body 3. The holding sheet 27 is bonded to the longitudinal end portion of the absorbent body 3 over the whole width of the absorbent body 3 with an adhesive (not shown), whereby each of the longitudinal end portions of the absorbent body 3 is secured to the outer cover 2.

Covering the skin facing side of the longitudinal end portions of the absorbent body 3 with the respective holding sheets 27 prevents the front and rear end portions of the absorbent body 3 from coming into direct contact with the wearer's skin and prevents leakage of the absorbent polymer of the absorbent core 34 from the front and rear ends of the absorbent body 3.

The outer sheet 21 has an adhesive (such as a hot melt adhesive) applied to its inner side (the side facing the inner sheet 22) in a prescribed pattern. The adhesive is applied to almost the entire areas corresponding to the side seals 11, the areas corresponding to functionless regions 51 (described *infra*) of the below-waist elastic members 23, and the area corresponding to a leg elastic member cut region 52 (described *infra*). The adhesive is not applied to almost the entire area corresponding to composite stretch panels 4 (described later in detail) and almost the entire area corresponding to the crotch section C.

The inner sheet 22 has an adhesive (such as a hot melt adhesive) applied to its side facing the outer sheet 21 in a prescribed pattern. The adhesive is applied to almost the entire area of other than the regions corresponding to the composite stretch panels 4.

It is understood that the application pattern of the adhesive on the outer sheet-facing side of the inner sheet 22 includes the application pattern of the adhesive on the inner side of the outer sheet 21. Therefore, on joining the outer sheet 21 and the inner sheet 22, the adhesive that has been applied to the outer sheet-facing side of the inner sheet 22 adheres to the inner side of the outer sheet 21. This amounts to applying the adhesive to the inner side of the outer sheet 21 in the same pattern on the outer sheet-facing side of the inner sheet 22.

As illustrated in Figs. 21 and 22, the hydrophilic sheet 29 is disposed with its upper edge (the edge closer to the waist opening edge) aligned with the longitudinal end edge of the inner sheet 22 and with its lower edge (the edge farther from the waist opening edge) located on the garment facing surface of the absorbent body 3. In detail, the position of the lower edge of the hydrophilic sheet 29 is such that the hydrophilic sheet 29 covers the region where below-waist elastic members 23A forming an upper below-waist subportion D1 (the details of which will be described later) are disposed.

The hydrophilic sheet 29 may be made of essentially hydrophilic fibers or fibers that are essentially non-hydrophilic but have been rendered hydrophilic by a hydrophilization treatment. The latter fibers are preferred. Examples of essentially hydrophilic fibers include cellulosic fibers, such as rayon, cellulose acetate, and cotton.

Hydrophilization of the essentially non-hydrophilic fibers is achieved by, for example, treating the surface of the fibers with a hydrophilizing agent or incorporating a hydrophilizing agent into fibers. The non-hydrophilic fibers include one-component fibers made of a polyolefin resin, such as polyethylene (PE) or polypropylene (PP) or a polyester resin, such as polyethylene terephthalate (PET), and conjugate fibers made of a plurality of these resins in, e.g., a sheath/core or a side-by-side configuration. Of sheath/core conjugate fibers preferred are those having a low melting component sheath and a high melting component core, particularly those having polyethylene as a low melting component and polypropylene or polyethylene terephthalate as a high melting component.

It is preferred for the hydrophilic sheet 29 to have high fluid wicking ability and low fluid retentivity. Specifically, the fluid wicking ability of the hydrophilic sheet 29 is preferably such that the fluid spread area measured as described below is at least 2 cm$^2$, more preferably 5 cm$^2$ or more. The broader the fluid spread area of the hydrophilic sheet 29, the higher the properties of allowing perspiration to evaporate from the waist flap F. The hydrophilic sheet 29 having the above recited preferred fluid spread area is exemplified by SMMS (spunbonded-meltblown-meltblown-spunbonded) nonwoven fabric made of hydrophilized PP fiber or hydroentangled nonwoven fabric made of rayon and PET.

Method of measuring fluid spread area:

A 10 cm square cut piece of a hydrophilic sheet is placed on an acrylic resin plate, and 0.05 ml of colored water is dropped from 30 mm above the cut piece. Ten minutes later, the area of the colored water spread in the cut piece is measured using an image analyzer.

The fluid retentivity of the hydrophilic sheet 29 is preferably such that the fluid retention measured as follows is 30 g/m$^2$ or less, more preferably 5 g/m$^2$ or less. The smaller the fluid retention of the hydrophilic sheet 29, the higher the properties of allowing sweat to evaporate when sweat is continuously absorbed by the hydrophilic sheet 29. The hydrophilic sheet 29 having the above recited preferred fluid retention is exemplified by SMMS nonwoven fabric made of hydrophilized PP fiber.

Method of measuring fluid retention:

A 10 cm square cut sample of a hydrophilic sheet, the mass of which has been measured, is put in a beaker containing 500 ml of distilled water and left to stand for 1 minute. The sample is taken out of water and left hanging on a clothesline for 10 minutes. The sample is centrifuged at 800 rpm for 10 minutes and then weighed. A fluid retention is calculated from formula:

$$\text{Fluid retention (g/m}^2) = \{\text{mass (g) of sample after centrifugation - initial mass (g) of sample}\}/\text{area (m}^2) \text{ of initial sample}$$

The outer cover 2 has a waist gather G2 and below-waist gathers G1 in the waist flap F and the below-waist portion D, respectively, in each of the front section A and the rear section B, each gather being formed of a composite stretch panel 4 (the details of which will be described *infra*). In a configuration having the outer cover 2 bonded to the garment facing side of the absorbent body 3 as in the second embodiment, the term "waist flap F" is intended to include not only the portion of the outer cover 2 that is longitudinally outward of each longitudinal edge of the absorbent body 3 but also the portions of the outer cover 2 on both lateral sides of that portion.

Going into the details of the waist gather G2, the waist flap F in each of the front section A and the rear section B has waist elastic members 24 arranged along the circumferential edge of the waist opening 12. The holding sheet 27, the hydrophilic sheet 29, the inner sheet 22, the waist elastic members 24, and the outer sheet 21 form a composite stretch panel 4 (the details of which will be described later). The composite stretch panel 4 gathers between the pair of side seals 11, that is, over the whole circumference of the annular waist flap F to form the waist gather G2.

The outer cover 2 also gathers along the pair of leg openings 13 to form respective leg gathers G3. In detail,

19

leg elastic members 25 are provided along the circumferential edge of each leg opening 13 curving in the crotch section C. The leg elastic members 25 are fixed in their stretched state between the outer sheet 21 and the inner sheet 22, whereby the leg gather G3 is formed along each leg opening 13.

In the present embodiment, in relation to the process of production described later, the leg elastic members 25 provided along the whole circumference of each leg opening 13 are composed of leg elastic members 25 provided along half of the circumferential edge of the leg opening 13 in the front section A and different leg elastic members 25 provided along the other half of the circumferential edge of the leg opening 13 in the rear section B. However, it is possible, depending on the process of production, to continuously dispose leg elastic members along the whole circumference of each leg opening 13.

Going into the details of the below-waist gathers G1, the outer cover 2 has a below-waist portion D in each of the front section A and the rear section B. The below-waist portion D has composite stretch panels 4 (the details of which will be described later) each gathering to form a below-waist gather G1. As used herein, the term "below-waist portion" refers to a portion from the lower border of the waist flap F to the upper border of the leg openings 13. In detail, the below-waist portion D has a plurality of below-waist elastic members 23 arranged in the diaper lateral direction. The below-waist elastic members 23 extend between near the side edge of the absorbent member 3 and the side seal 11. The below-waist elastic members 23 are not disposed between positions near the opposite side edges of the absorbent member 3.

The composite stretch panels 4 are each composed mainly of the holding sheet 27, the hydrophilic sheet 29, (which may be absent in part of the composite stretch panel, as will be described *infra*), the inner sheet 22, the below-waist elastic members 23, and the outer sheet 21. The composite stretch panel 4 gathers between each side seal 11 and near each side edge of the absorbent member 3 to form a pair of laterally spaced below-waist gathers G1. The below-waist gather G1 is not formed between near the opposite side edges of the absorbent member 3. The term "near the side edge of the absorbent body 3" as used herein is intended to mean 30 mm or less inboard and 20 mm or less outboard of each side edge of the absorbent member 3.

The below-waist portion D is divided into an upper below-waist subportion D1 and a lower below-waist subportion D2. The upper below-waist subportion D1 is preferably in the region of the diaper 1 which, while worn by a wearer, is applied to a wearer's body part between the left and right iliac crests and the left and right anterior superior iliac spines. That part of a wearer's body will sometimes be referred to as the iliac region.

In the present embodiment, as illustrated in Fig. 22, the whole or the most part of the hydrophilic sheet 29 is located in the upper below-waist subportion D1. The hydrophilic sheet 29 is not present or substantially absent in the lower below-waist subportion D2. To put it another way, the below-waist portion D includes a first subportion formed of a laminate of four sheets: the holding sheet 27, the hydrophilic sheet 29, the inner sheet 22, and the outer sheet 21 and a second subportion formed of a laminate of three sheets: the holding sheet 27, the inner sheet 22, and the outer sheet 21. The first subportion roughly corresponds to the upper below-waist subportion D1, and the second subportion roughly corresponds to the lower below-waist subportion D2.

Similarly to the diaper 1 of the first embodiment, the diaper 1A according to the second embodiment is secured to the wearer's body by the constrictive force that primarily rests on the below-waist elastic members 23A disposed in the upper below-waist subportion D1. In other words, the constrictive force of the elastic members 24 disposed in the waist flap F is not a primary means for securing the diaper 1A to the wearer's body unlike the conventional pull-on diapers. On the contrary, an increased constrictive force of the waist flap F helps the diaper 1A to move down.

It should be noted, however, that it is preferred for the waist flap F to have at least a certain constrictive force. Since the skin-contacting surface of the waist flap F is formed of a hydrophobic sheet, sweat adhering to the skin-contacting side of the waist flap F will not be absorbed through the hydrophobic sheet into the hydrophilic sheet unless the waist flap F has a certain level of constrictive force. The constrictive force of the waist flap F is preferably at least 0.2 kPa, more preferably at least 0.5 kPa.

The lower below-waist subportion D2 is preferably applied to the body part below the iliac region, namely the lower abdominal region of a wearer. The width (measured in the longitudinal direction of the diaper 1A) of the lower below-waist subportion D2 is preferably 40 to 70 mm, more preferably 45 to 65 mm. The lower below-waist subportion D2 has disposed therein a plurality of below-waist elastic members 23B.

Comparing the tensile load between the waist flap F, the upper below-waist subportion D1, and the lower below-waist subportion D2, it is preferred that the upper below-waist subportion D1 have the highest tensile load, the lower below-waist subportion D2 have the second highest tensile load, and the waist flap F have the lowest tensile load. By providing such a difference in tensile load between different portions, sliding down of the diaper 1A while worn is effectively prevented, and the diaper 1A snugly fits the wearer's body to effectively prevent leakage without causing discomfort.

A frontal crotch portion E will then be described. The term "frontal crotch portion E" as used herein denotes the most frontal of quarters into which the crotch section C is divided in the diaper longitudinal direction. The frontal crotch portion E has a plurality of frontal crotch elastic members 26 arranged therein over the whole width of the outer cover 2. The frontal crotch elastic members 26 are fixed in their stretched state between the outer sheet 21 and the inner sheet

22, whereby the frontal crotch portion E gathers to form a frontal crotch gather G4.

The outer cover 2 has functionless regions 51 within an area where it faces the absorbent body 3, in which regions the contractibility of the elastic members is lost. In the present embodiment, the functionless regions 51 are formed by making the below-waist elastic member 23 and the frontal crotch elastic members 26 lose their contractibility. As will be described in more detail with respect to the method of production, the functionless regions 51 are the results of providing continuous length below-waist elastic members 23S and continuous length frontal crotch elastic members 26S over the whole width of the outer cover 2 and depriving part of the continuous elastic members 23S and 26S of contractibility. Depriving the continuous elastic members of ability of contraction may be achieved by means of, for example, a number of cutting projections or blades that segmentalize the elastic members or a number of embossing pins that thermally harden the elastic members.

By providing the functionless regions 51, the continuous below-waist elastic members 23S and the continuous frontal crotch elastic members 26S provided over the whole width of the outer cover 2 discontinuously gather to form a laterally spaced pair of below-waist gathers G1 and a laterally spaced pair of frontal crotch gathers G4.

The functionless region 51 located between the laterally spaced pair of below-waist gathers G1 is the result of depriving the continuous below-waist elastic members 23S bonded between the outer sheet 21 and the inner sheet 22 of their contractibility, whereas the functionless region 51 located between the laterally spaced frontal crotch gathers G4 is the result of depriving the continuous frontal crotch elastic members 26S bonded between the outer sheet 21 and the inner sheet 22 of their contractibility. Thus, the absorbent body 3 is prevented from bunching up and exhibits a good appearance and absorbing performance.

There is formed a leg elastic member cut region 52 in the laterally middle part of the crotch section C. As will be described in detail with reference to the method of production, the leg elastic member cut region 52 is the result of curvingly providing continuous leg elastic members 25S over the whole width of the outer cover 2 and cutting the continuous elastic members 25S at the midpoint of the width of the crotch section C. The leg elastic member cut region 52 is formed by means of, for example, a number of cutting projections or blades that cut the elastic members. By providing the leg elastic member cut region 52, the continuous leg elastic members 25S provided curvingly over the whole width of the outer cover 2 form a laterally spaced pair of leg elastic members 25 in the crotch section C.

In the present embodiment, the outer cover 2 does not have a composite stretch panel 4 in the crotch section C. Thus, urine discharged in the frontal part of the absorbent body 3 is allowed to smoothly move rearward, and the absorbing performance of the diaper 1A is markedly improved. The leg elastic members 25 and the frontal crotch elastic members 26 are preferably strings or tapes of stretch materials, such as natural rubber, polyurethane resins, foamed urethane resins, and hot-melt stretch materials.

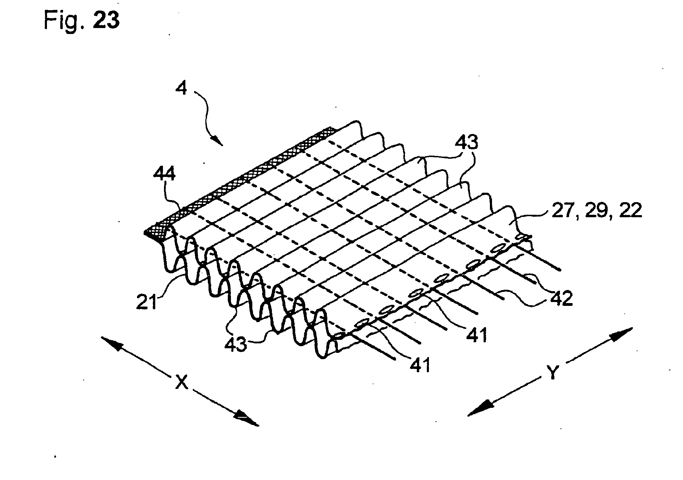

The composite stretch panel 4 forming the below-waist gathers G1 and the waist gather G2 will be described in detail. The composite stretch panel 4 has the following configurational characteristics (1) to (3) as illustrated in Figs. 23 and 12.

(1) The composite stretch panel-forming sheets 21 and 22, which face each other, are bonded together at bonds 41 that are arranged discretely in both the stretch direction (direction X) of the composite stretch panel 4 and a direction perpendicular to the stretch direction (direction Y).

(2) Composite stretch panel-forming elastic members 42 are disposed at positions not to pass through the bonds 41 and are not fixed to the composite stretch panel-forming sheets 21 and 22 at other than their opposite ends.

(3) Each of the facing composite stretch panel-forming sheets 21 and 22 gathers to form folds 43 each extending continuously over a plurality of the elastic members 42.

Every part of the composite stretch panel 4 forming the waist gather G2 is a four-sheet laminate composed of the holding sheet 27, which is the skin contacting surface-defining sheet, the hydrophilic sheet 29, the inner sheet 22, and the outer sheet 21, which is the garment facing surface-defining sheet. On the other hand, the composite stretch panel 4 forming the below-waist gather G1 includes a first part formed of a four-sheet laminate composed of the holding sheet 27, the hydrophilic sheet 29, the inner sheet 22, and the outer sheet 21 and a second part formed of a three-sheet laminate composed of the holding sheet 27, the inner sheet 22, and the outer sheet 21. The first part nearly corresponds to the upper below-waist subportion D1, and the second part nearly corresponds to the lower below-waist subportion D2 in the present embodiment. The composite stretch panel 4 forming the waist gather G2 will hereinafter be described with particular reference to the first part formed of the four-sheet laminate. While description of the second part formed of the three-sheet laminate is omitted, the description of the first part applies to the second part. In Figs. 23 and 12, the holding sheet 27, the hydrophilic sheet 29, and the inner sheet 22 are depicted as being integral with each other.

In the present embodiment, the facing composite stretch panel-forming sheets are the outer sheet 21 (the garment facing surface-defining sheet) and the inner sheet 22 which is a separate sheet interposed between the hydrophilic sheet 29 and the outer sheet 21. The phrase "separate sheet" as used herein means a sheet not integral with or independent of any of the skin contacting surface-defining sheet (i.e., the holding sheet 27), the garment facing surface-defining sheet (i.e., the outer sheet 21), and the hydrophilic sheet 29.

In the present embodiment, the composite stretch panel 4 forming the waist gather G2 is composed mainly of the holding sheet 27, the hydrophilic sheet 29, the inner sheet 22, the outer sheet 21, and the waist elastic members 24. The composite stretch panel 4 forming the below-waist gather G1 has a first part composed mainly of the holding sheet 27, the hydrophilic sheet 29, the inner sheet 22, the outer sheet 21, and the below-waist elastic members 23 (nearly corresponding to the upper below-waist subportion D1) and a second part that is devoid of the hydrophilic sheet 29 as compared with the first part (nearly corresponding to the lower below-waist subportion D2).

Although the designation of the elastic members differs according to the designation of the gathers in that way, all the elastic members constructing the composite stretch panels 4 will hereinafter be inclusively referred to as "elastic members 42".

It is preferred that all the elastic members 42 be disposed not to pass through any of the bonds 41. However, a manufacturing error can cause a few elastic members 42 to pass through a few bonds 41. Even if such is the case, the effects of the present invention are achieved sufficiently. When the elastic members 42 do not pass through 70% or more by number of the bonds 41, the elastic members 42 are regarded to be disposed not to pass through the bonds 41.

As illustrated in Figs. 23 and 12, the outer sheet 21 and the inner sheet 22 are bonded together at the bonds 41 that are discretely arranged in both the stretch direction (direction X) of the composite stretch panel 4 and the direction perpendicular to that direction (direction Y). The bonds 41 are preferably formed by heat fusion.

The plurality of elastic members 42 extend in parallel to each other in the longitudinal direction of the composite stretch panel 4. In an embodiment like the present one where elastic members 42 are parallel to each other, the stretch direction of the composite stretch panel 4 is the same as the stretch direction of the elastic members 42. In an embodiment where the elastic members 42 are not parallel to each other, the stretch direction of the composite stretch panel 4 is a direction perpendicular to the direction of a fold 43 (hereinafter described) running across a plurality of the elastic members 42.

Fig. 12 illustrates a pattern of arranging the bonds 41.

The construction and configuration of the composite stretch panels 4 used in the second embodiment, inclusive of the dimension and pitch of the bonds 41, are the same as with the composite stretch panels of the first embodiment.

The outer sheet 21, the inner sheet 22, and the elastic members 42 that construct the composite stretch panels 4 in the second embodiment may be of the same types as used in the first embodiment.

The disposable pull-on diaper of the second embodiment exhibits excellent performance with its waist flap F. For example, the waist flap F helps sweat as released to evaporate and escape and has good breathability, softness, and leakage resistance.

More specifically, sweat absorbed by the holding sheet 27, the skin facing surface-defining sheet of the waist flap F, migrates to the adjacent hydrophilic sheet 29 and diffuses therein. Since the folds 43 of the composite stretch panel 4 provide spaces, the sweat having moved to the hydrophilic sheet 29 is allowed to evaporate rapidly. The waist flap F is thus superior in allowing sweat to evaporate and escape.

Although the holding sheet 27 as the skin contacting surface-defining sheet, being hydrophobic, is not so absorptive, the skin contacting surface-defining sheet of the waist flap F is pressed onto the hydrophilic sheet 29 by the constrictive force of the waist gather G2 while worn. This allows the sweat adhering to the skin contacting surface of the waist flap F to migrate to the hydrophilic sheet 29.

If the garment facing surface-defining sheet and the skin contacting surface-defining sheet are each formed of a hydrophilic sheet, both the skin contacting surface and the garment facing surface of the waist flap F become sticky with sweat absorbed by the hydrophilic sheets. In contrast, the surfaces are kept dry with no stickiness because both the skin contacting surface and the garment facing surface of the waist flap F are formed by the hydrophobic holding sheet 27 and the hydrophobic outer sheet 21, respectively.

The folds 43 of the composite stretch panel 4 moves during perspiration to bring about improved quick-drying properties. When a wearer sweats, after the folds 43 in contact with the sweaty part of the wearer's skin stretch to become flat, they are less retractable because of increased friction. As a result, the position of gathering moves to a dry part of the skin, and the diaper, particularly the waist flap F is kept in close contact with the wet part of the skin, whereby the sweat is efficiently absorbed by the composite stretch panel 4. The sweat absorbed into the composite stretch panel 4 diffuses in the hydrophilic sheet 29 then evaporates and dries quickly.

The below-waist gather G1 and the waist gather G2 are formed of the composite stretch panels 4. Since these gathers provide stretchability substantially without using a hot melt adhesive, the diaper has good breathability in both the front section A and the rear section B, and the outer sheet 21 and the inner sheet 22 of the outer cover 2 are allowed to show their essential texture and feel soft or non-stiff.

The waist flap F used in the second embodiment has four sheet components: the holding sheet 27, the hydrophilic sheet 29, the inner sheet 22, and the outer sheet 21. It has one extra sheet, as compared with a waist flap F having three sheet components: a holding sheet 27, a hydrophilic sheet 29 (or a hydrophilic inner sheet 22), and an outer sheet 21 as used in third to fifth embodiments hereinafter described, and is prone to be less breathable and less soft. It is therefore preferred that the hydrophilic sheet 29 and the inner sheet 22 (and the outer sheet 21) be joined together by means of the bonds 41. By joining the hydrophilic sheet 29 and the inner sheet 22 without using a hot melt adhesive, reduction in breathability and softness of the waist flap F, which might otherwise be caused by an increased amount of a hot melt adhesive, can be averted.

While the hydrophilic sheet 29 is provided on the back of the absorbent body 3, it is protected from contact with urine absorbed by the absorbent body 3 because of the presence of the backsheet 33 provided at the back of the absorbent body 3.

In the composite stretch panel 4 contractibility of the elastic members 42, being little or not hindered, is made full use of. When the stretch characteristics of, for example, the below-waist portion D are graphically represented by plotting the circumference as abscissa and the tensile load as ordinate, the characteristic curve has a gentle slope, indicating high stretchability. The characteristic curve in an extension mode and that in a contraction mode show a small difference of load, indicating a small variation in load with a variation in circumference. This means that the diaper applies an equal wearing pressure to an infant with a smaller waist and an infant with a larger waist. As a result, the tendencies of a diaper to slide/slip down on an infant of relatively small size and to leave indentation marks on an infant of relatively large size are eliminated, and there is provided a diaper that fits wearers of broader range of sizes.

Thus, the diaper of the present embodiment gently fits a wearer and hardly slides down during use by virtue of its high contractibility. The diaper shows its full contractibility as a whole, which offers the advantage of compactness. Additionally, the amount of the elastic members 42 may be reduced, which is economically advantageous. The composite stretch panel 4 gathers to form on its interior side folds 43 that are to come into contact with the wearer's body to provide good cushioning properties and gentle fit with little damage to the skin during use. The folds 43 are also formed on the exterior side of the outer cover 2 (the exterior side during use) to make the diaper feel pleasant to the touch.

According to the disposable diapers disclosed in Patent Documents 2 and 3 *supra*, the hydrophobic sheet and the hydrophilic sheet are joined with an adhesive. Patent Document 2 is silent on the application pattern of the adhesive. Patent Document 3 mentions that the adhesive is applied discretely but does not disclose any further details of application. It appears that the waist flaps of the disposable diapers disclosed in Patent Documents 2 and 3 form folds due to contraction of the elastic members. However, it is believed that the 4-layer structure (the hydrophobic sheet, the hydrophilic sheet, the elastic members, and the hydrophobic sheet) gathers in a unit to form folds while forming little space between each hydrophobic sheet and the hydrophilic sheet. This tendency seems to be especially conspicuous with the disposable diaper of Patent Document 3 on account of the tape form of the elastic member used in the waist flap.

Therefore, the disposable diapers of Patent Documents 2 and 3 may not be seen as sufficiently allowing perspiration to evaporate and escape through the waist flap. In this respect, further improvement has been awaited. The problem also applies to other absorbent articles with a waist flap than disposable diapers.

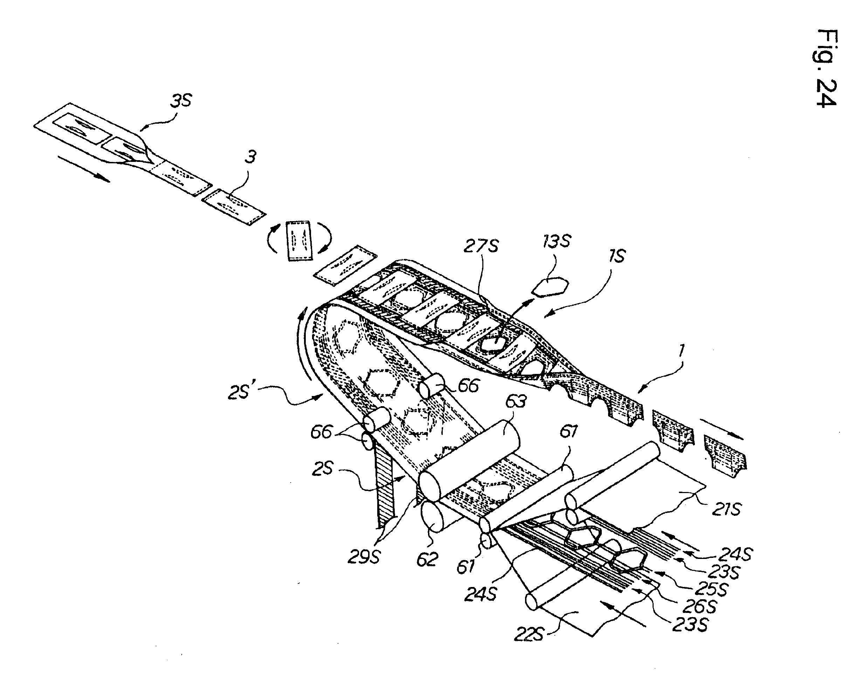

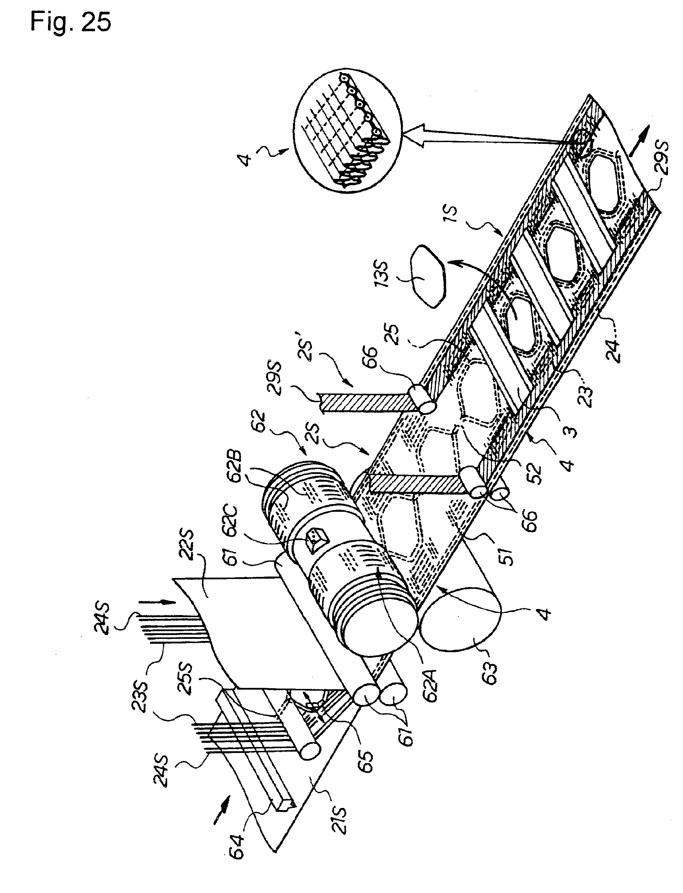

A method for continuously producing the disposable pull-on diaper of the second embodiment will then be described with reference to the accompanying drawing. Fig. 24 is a perspective showing the whole of the method. Fig. 25 is a view focused on the step of forming composite stretch panels, functionless regions, and leg elastic member cut regions in the present method.

As illustrated in Figs. 24, 25, and 16, the present method carries out the step of joining the outer sheet 21 and the inner sheet 22 by the bonds 41 to form composite stretch panels 4 and the step of depriving elastic members 23 and 26 of contractibility to form functionless regions 51 by using the same roller 62.

The present method is a method of producing a disposable pull-on diaper including the steps of (1) joining a continuous outer sheet 21 S (a continuous web of an outer sheet 21) and a continuous inner sheet 22S (a continuous web of an inner sheet 22) while disposing continuous elastic members 23S, 24S, 25S, and 26S (elastic members 23, 24, 25, and 26, respectively, of continuous length) therebetween to form a continuous outer cover 2S (an outer cover 2 of continuous length), (2) bonding continuous hydrophilic sheets 29S (a pair of continuous webs of hydrophilic sheets 29) to the side of the continuous inner sheet 22S of the continuous outer cover 2S to provide a continuous outer cover 2S having a pair of continuous hydrophilic sheets 29S (hereinafter referred to as a continuous web assembly 2S'), (3) bonding absorbent bodies 3 to the side of the continuous inner sheet 22S of the continuous web assembly 2S' each via a joint region 15 (see Fig. 4) and folding both edges of the continuous outer sheet 21 S over the absorbent bodies 3 to provide continuous holding sheets 27S (a pair of continuous webs of holding sheets 27) to form a continuous diaper 1S (a diaper 1 of continuous form), and (4) cutting the continuous diaper 1S into individual diapers 1A.

The present method will be described in detail. A continuous outer sheet 21 S is used as a web material of an outer sheet 21, a continuous inner sheet 22S is used as a web material of an inner sheet 22, and a continuous hydrophilic sheet 29S is used as a web material of a hydrophilic sheet 29. Two webs of the continuous hydrophilic sheet 29S are used, one for the front waist flap F, and the other for the rear waist flap F. Both edge portions of the continuous outer sheet 21 S are folded over the side of the continuous inner sheet 22S each to become a continuous holding sheet 27S (the details of which will be described *infra*).

The continuous elastic members 23S, 24S, 25S, and 26S (elastic members 23, 24, 25, and 26 of continuous length) are introduced into between the continuous outer sheet 21S and the continuous inner sheet 22S in their stretched state. Continuous frontal crotch elastic members 26S (frontal crotch elastic members 26 of continuous length) are omitted from Fig. 25. Before the continuous outer sheet 21S and the continuous inner sheet 22S are joined, a hot melt adhesive is applied to their facing sides in respective predetermined patterns by means of, e.g., an adhesive applicator 64 (see Fig. 25). The continuous leg elastic members 25S are introduced into between the continuous outer sheet 21S and the continuous inner sheet 22S while being rocked across the moving sheets by rocking guides 65 (see Fig. 25).

The two continuous sheets 21S and 22S with the continuous elastic members 23S, 24S, 25S, and 26S disposed therebetween are pressed between a pair of nip rollers 61. There is thus obtained a continuous outer cover 2S having the two continuous sheets 21S and 22S and the continuous elastic members 23S, 24S, 25S, and 26S sandwiched therebetween. The below-waist elastic members 23 are not bonded with a hot melt adhesive in the parts between near each side edge of the absorbent body 3 and each side seal 11. Similarly, the waist elastic members 24 are not bonded with a hot melt adhesive in the parts between the opposite side seals 11.

In this stage, the continuous outer cover 2S has formed therein none of composite stretch panels 4, functionless regions 51, and a leg elastic member cut region 52.

The continuous outer cover 2S not having formed composite stretch panels 4 is then introduced between a composite stretch panel-forming roller 62 and an anvil roller 63. The composite stretch panel-forming roller 62 has on its peripheral surface a composite stretch panel-forming parts 62A that function to form composite stretch panels 4, functionless region-forming parts 62B that function to form elasticization functionless regions 51, and a leg elastic member cutting region-forming part 62C that functions to form a leg elastic member cut region 52.

The anvil roller 63 is a roller with a smooth surface and backs up the composite stretch panel-forming roller 62.

The leg elastic member cut region-forming part 62C of the composite stretch panel-forming roller 62 is a part by which the continuous leg elastic members 25S are cut in the laterally middle part of the crotch section C to form leg elastic member cut regions 52. The leg elastic member cut region-forming part 62C is composed of a number of projections or cutting blades. The continuous leg elastic members 25S are cut by the leg elastic member cut region-forming part 62C to provide leg elastic members 25 spaced apart in the diaper lateral direction in the laterally middle part of the crotch section C.

Introducing the continuous outer cover 2S having none of composite stretch panels 4, functionless regions 51, and leg elastic member cut regions 52 between the composite stretch panel-forming roller 62 having the mentioned configuration and the anvil roller 63 results in the formation of the continuous outer cover 2S having formed in prescribed portions thereof the composite stretch panels 4, functionless regions 51, and leg elastic member cut regions 52.

Subsequently, the continuous outer cover 2S having the composite stretch panels 4 formed therein and each of the continuous hydrophilic sheets 29S having a hot melt adhesive Q (see Fig. 26) applied to one side thereof are joined in a fashion such that the continuous inner sheet 22S and the hot melt adhesive Q face each other and introduced between a pair of nip rollers 66 to form the continuous web assembly 2S'. The continuous hydrophilic sheets 29S and the continuous outer cover 2S may be bonded by heat sealing.

The disposable pull-on diaper 1A of the second embodiment is produced in a usual manner for the production of disposable pull-on diapers in a transverse feed system, except for using the continuous web assembly 2S' having the composite stretch panels 4, functionless regions 51, and leg elastic member cut regions 52.

According to the present method, disposable pull-on diapers having the composite stretch panels 4 and the functionless regions 51 can be produced efficiently because the step of joining the outer sheet 21 and the inner sheet 22 at the bonds 41 to make the composite stretch panels 4 and the step of rendering the elastic members 23 and 26 functionless to form the functionless regions 51 are achieved by using the same roller 62.

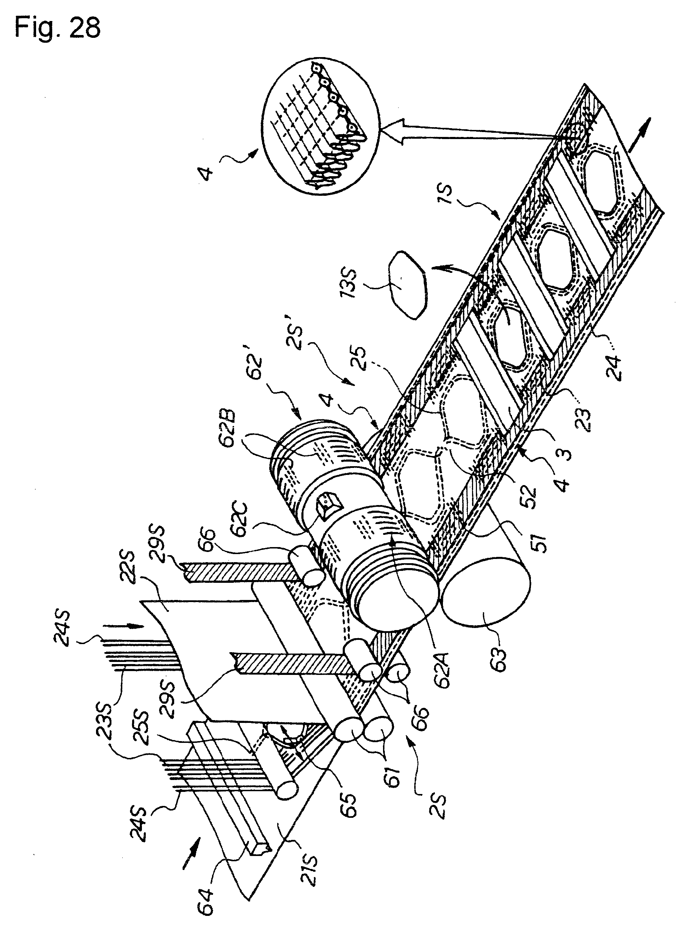

In the method illustrated in Fig. 25, while joining the continuous outer cover 2S and the continuous hydrophilic sheets 29S is preceded by the formation of composite stretch panels 4 to make the continuous web assembly 2S', the position of the joining step is not limited to this. As illustrated in Fig. 28, forming the composite stretch panels 4 may be preceded by joining the continuous hydrophilic sheets 29S to the continuous outer cover 2S to make the web assembly 2S'. In this case, the continuous hydrophilic sheets 29S, the continuous inner sheet 22S, and the continuous outer sheet 21 S may be joined together all at once by making the bonds 41 to form the composite stretch panels 4 instead of applying a hot melt adhesive between the continuous hydrophilic sheets 29S and the continuous inner sheet 22S. The mentioned manner of joining is advantageous in that the amount of the hot melt adhesive to be used is reduced.

The present invention will further be described with reference to other embodiments. The description will generally be confined to the differences from the second embodiment. Common parts and elements are identified by the same numerals as in the second embodiment and will not be redundantly described. The configuration of the second embodiment applies to the other embodiments unless otherwise specified. The absorbent articles according to the other embodiments produce the same effects as with the absorbent article of the second embodiment.

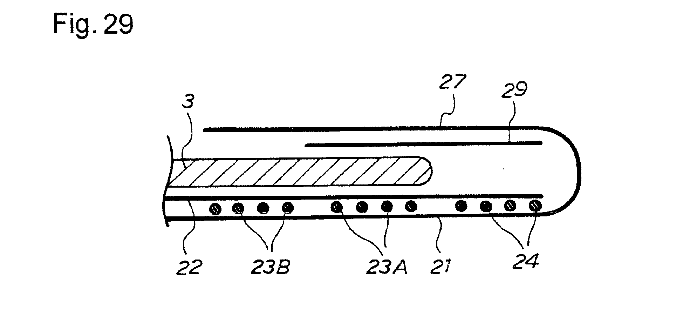

A disposable pull-on diaper according to a third embodiment of the invention is different from that of the second embodiment in the position of disposing the hydrophilic sheets 29 as illustrated in Fig. 29.

In the third embodiment shown in Fig. 29, the hydrophilic sheet 29 is disposed on the skin facing side of each longitudinal end portion of the absorbent body 3, that is, between the holding sheet 27 (the skin contacting surface-defining sheet) and the skin facing side of the longitudinal end portion of the absorbent body 3. Other factors of the hydrophilic sheet 29, including the size, shape, and location in a plan view, may be the same as in the second embodiment.

The hydrophilic sheet 29 and the holding sheet 27 may be bonded with a hot melt adhesive. They may be bonded by heat sealing or ultrasonic bonding, in which case the total amount of the hot melt adhesive to be used is reduced. The hydrophilic sheet 29, the absorbent body 3, and the inner sheet 22 may be bonded with, for example, a hot melt adhesive.

The third embodiment is otherwise structurally and functionally the same as the second embodiment.

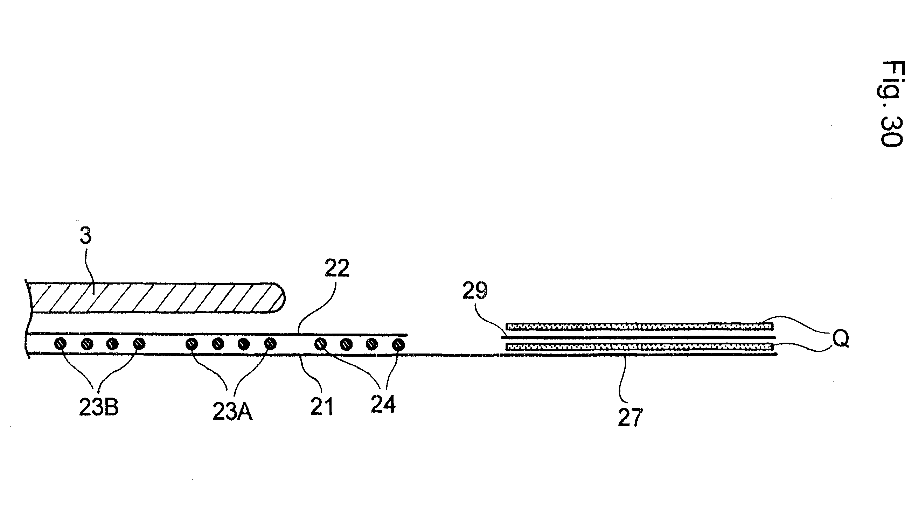

The longitudinal end portions of the disposable pull-on diaper according to the third embodiment are fabricated, for example, as follows. As illustrated in Fig. 30, a hydrophilic sheet 29 is bonded to the inner side of a holding sheet 27 before the latter is folded back, and a hot melt adhesive Q is then applied to the hydrophilic sheet 29 on the side to face the absorbent body 3. Thereafter, the holding sheet 27 and the hydrophilic sheet 29 are folded as a unit over the skin facing side of the absorbent body 3.

The third embodiment has the same effects as the second embodiment and additionally produces the following effect by virtue of the difference of the location of the hydrophilic sheet 29. Since the hydrophilic sheet 29 is placed on the skin facing side of each longitudinal end portion of the absorbent body 3, it has a larger area on which it is capable of absorbing sweat. Therefore, sweat absorbed by the part of the hydrophilic sheet 29 where the absorbent body 3 exists in the thickness direction readily spreads in the hydrophilic sheet 29 and migrates to the waist flap F. As a result, sweat is allowed to evaporate and escape through and along the waist flap F, which leads to improvement of the absorbent article as a whole in terms of allowing sweat to evaporate and escape.

In the third embodiment, the hydrophilic sheet 29 may be disposed not to overlap the absorbent member 3, that is, disposed in only the waist flap F, while not shown. In such a modification, sweat absorbed by the absorbent body 3 is retained mostly in the absorbent core 34.

The third embodiment may allow urine absorbed by the absorbent body 3 to diffuse through the hydrophilic sheets 29 covering the skin facing side of the longitudinal end portions of the absorbent body 3, providing a higher possibility of the urine leaking from the waist flap F. In fact, nevertheless, urine seldom reaches the longitudinal end portion of the absorbent body 3. Besides, since the hydrophilic sheet 29 is covered with the holding sheet 27 and the outer sheet 21, both being formed of a hydrophobic sheet, there is little possibility of urine actually leaking from the skin contacting side or the garment facing side of the waist flap F.

As previously stated, it is possible to bond the hydrophilic sheet 29 to the inner side of the holding sheet 27 through ultrasonic bonding. In this case, the amount of the hot melt adhesive to be used is reduced, the breathability and softness of the waist flap F are improved, and there is little likelihood of a hot melt adhesive striking through to the skin contacting surface of the waist flap F.

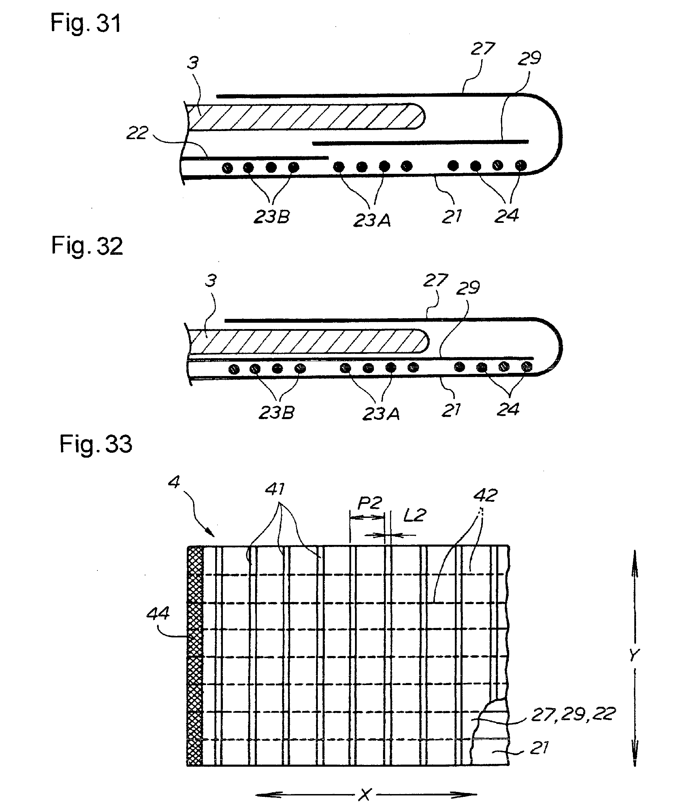

A disposable pull-on diaper according to a fourth embodiment will be described. As illustrated in Fig. 31, the fourth embodiment is different from the second embodiment primarily in that the inner sheet 22 is absent in the waist flap F and near the longitudinal end of the absorbent body 3.

Describing specifically, while the hydrophilic sheet 29 in the fourth embodiment is equal to that in the second embodiment in terms of size, shape, location in a plan view, and the like, the inner sheet 22 is substantially absent in the waist flap F and near the longitudinal end of the absorbent body 3. The lower edge portion (the edge portion farther from the waist opening edge) of hydrophilic sheet 29 and the longitudinal end portion of the inner sheet 22 overlap slightly and are bonded to each other at the overlap.

Having no inner sheet 22, the waist flap F is composed of three sheets: the holding sheet 27, the hydrophilic sheet 29, and the outer sheet 21. Unlike the first and third embodiments, the composite stretch panel-forming sheets in the fourth embodiment are the hydrophilic sheet 29 and the outer sheet 21 (the garment facing surface-defining sheet). The fourth embodiment is otherwise structurally the same as the second embodiment.

In a modification of the fourth embodiment, the inner sheet 22 may be omitted from the crotch section C, that is, the crotch section C of the outer cover 2 may be formed of a single layer of the outer sheet 21 (not shown).

The fourth embodiment has the same effects as the second embodiment and additionally produces the following effect.

Unlike the second embodiment, the fourth embodiment does not have the inner sheet 22, which is usually formed of a hydrophobic sheet, between the hydrophilic sheet 29 and the outer sheet 21. There is, of necessity, no hot melt adhesive used to bond the hydrophilic sheet 29 and the inner sheet 22, which results in improved properties of the waist flap F in terms of allowing sweat to evaporate and escape.

Furthermore, since the number of the sheets constructing the waist flap F is smaller by one than in the second embodiment, the amount of the hot melt adhesive to be used is so reduced, bringing about improvement in breathability and softness of the waist flap F.

A fifth embodiment will be described. As shown in Fig. 32, the disposable pull-on diaper of the fifth embodiment is different from that of the second embodiment primarily in that the inner sheet is formed of the hydrophilic sheet 29. Accordingly, the fifth embodiment does not have an inner sheet 22 independent of the hydrophilic sheet 29 unlike the second to fourth embodiments. The composite stretch panel-forming sheets are the hydrophilic sheet 29 serving as an inner sheet and the outer sheet 21 serving as a garment facing surface-defining sheet.

The disposable pull-on diaper of the fifth embodiment has the same effects as in the fourth embodiment. Although the diaper of the fifth embodiment exhibits sufficient leakage resistance in its below-waist portion D, there is a possibility of slight urine leakage in the crotch section C. In the practice, however, there is very little likelihood of urine being brought into contact with the hydrophilic sheet 29 serving as an inner sheet because leakage from the crotch section C is virtually prevented by the side cuffs 35 rising along the absorbent body 3.

In a modification of the fifth embodiment, the above-mentioned leakage problem with the crotch section C of the fifth embodiment can be settled by forming the inner sheet 22 of a hydrophobic material and hydrophilizing only the part of the inner sheet that corresponds to the waist flap F and the below-waist portion D (i.e., the part corresponding to the front section A and the rear section B) while leaving the other part (i.e., the pat corresponding to the crotch section C) hydrophobic.

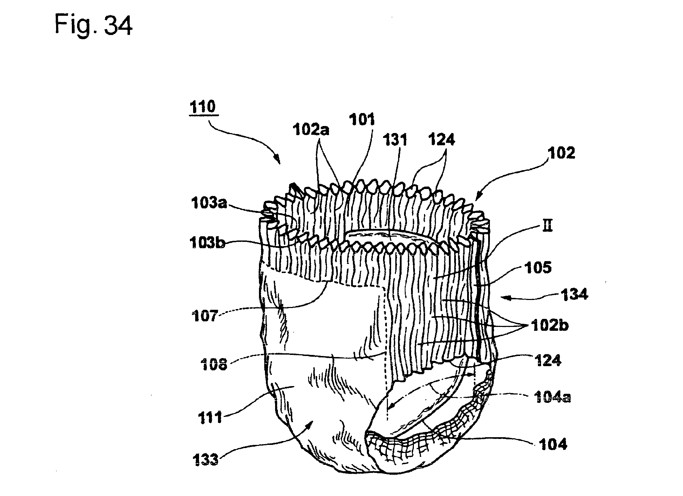

Fig. 34 is a schematic perspective illustration of a pull-on diaper 110 as an embodiment of the pull-on article according to the present invention in a state worn by an infant. The diaper of the present embodiment has a waist opening 101 and a pair of leg openings 104. The diaper 110 has a fluted panel having nearly circular openings open to at least part of the circumferential edge of the waist opening 101 or at least part of the circumferential edge of each leg opening 104. As used herein, the term "fluted panel" is a structure composed of two sheets 103a and 103b joined to each other and elasticized with elastic members to form folds 102a and 102b, respectively, the folds 102a and the folds 102b forming tubular spaces therebetween that are open at one or both longitudinal ends of the individual tubes. As will be described later, the folds 102a and the folds 102b are able to provide vacant spaces between themselves and the wearer's skin and between themselves and a garment, respectively. The folds 102a and 102b are able to freely move and change in shape without being restrained by the elastic members to let air in and out as if they breathe.

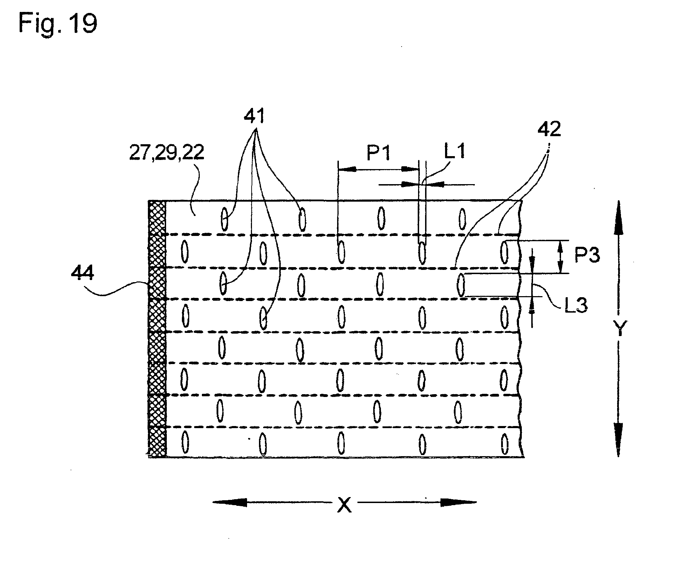

The configuration of the composite stretch panel 4 is not limited to those illustrated in Figs. 12, 16, and 23. For example, the bonds 41 may be arranged in a staggered pattern as in Fig. 19, in which the bonds 41 in adjacent rows extending in the stretch direction (direction X) are offset by half the pitch (P1/2).

Specifically, the bonds 41 are arranged to line up in both the stretch direction (direction X) of the composite stretch panel and the direction perpendicular thereto (direction Y), forming lines of seals. The bond 41 alternates with two adjacent elastic members in a seal line in the perpendicular direction (direction Y). In the composite stretch panel 4, there is a region having no bonds 41 between any line of bonds 41 in the stretch direction (direction S) and an adjacent line of bonds 41. The region continuously extends between the two end portions 44 of the composite stretch panel 4. The elastic member 42 is disposed unfixedly along that region. As a result, there are formed two folds 43 between bonds 41 adjacent in the stretch direction (direction X) of the composite stretch panel 4.

In order to assure formation of folds 43 continuously extending over a plurality of the elastic members 42, it is preferred that the bonds 41 be arranged at a pitch P1 (see Fig. 19) of 1 to 30 mm, more preferably 6 to 20 mm, in the stretch direction (direction X) of the composite stretch panel 4 with the composite stretch panel 4 being in the stretched state (as in Fig. 19); that the individual bonds 41 have a length L1 (see Fig. 19) of 0.1 to 5 mm, more preferably 0.2 to 1.5 mm in the same direction and in the same state; and that the ratio of the pitch P1 to the length L1 (P1/L1) be in the range of from 1.1 to 300, more preferably from 4 to 100.

The composite stretch panel 4 may have other configurations. For example, it may have the configuration as depicted in Figs. 33, in which the facing composite stretch panel-forming sheets 21 and 22 are bonded together by bonds 41 that are discontinuous in the stretch direction (direction X) of the panel 4 and continuous in the direction perpendicular to the stretch direction (direction Y), and each of the sheets 21 and 22 form folds 43 extending continuously over a plurality of the elastic members 42.

The description about the bonds 41 discretely arranged in the direction (direction Y) perpendicular to the stretch direction of the composite stretch panel 4, including the pitch in the stretch direction (direction X) of the composite stretch panel, applies as appropriate to the continuous bonds 41.

In the case where the composite stretch panel 4 has the continuous bonds 41, the elastic members 42 are fixed to the composite stretch panel-forming sheets 21 and 22 at every bond 41. This provides the advantage that the folds 43 form more easily than when the bonds 41 are discrete in the direction (direction Y) perpendicular to the stretch direction of the composite stretch panel 4 even if the pitch, length, and so on of the bonds 41 are not strictly set.

Fig. 34 is a perspective of a disposable pull-on diaper 110 as a preferred embodiment. The diaper 110 illustrated in Fig. 34 is of pull-on type having an outer cover 102 forming a waist opening and a pair of leg openings. The outer cover 102 has fluted panels each formed by two sheets 103a and 103b joined together at discretely provided bonds 121 and a plurality of elastic members 109 disposed in non-bonded regions between the two sheets such that they stretch and retract independently of the sheets. The sheets 103a and 103b elasticized with the elastic members 109 form folds 102a and folds 102b, respectively. The individual folds 102a and the individual folds 102b face each other to make pairs of folds, and each pair of folds makes a tubular space therebetween thereby to make a fluted structure. Along at least one end of the fluted panel, the basal parts of the folds formed of one of the sheets and those of the folds formed of the other sheet are bonded to each other in pairs to make an array of nearly circular openings. Such an array of openings defines at least part of the circumferential edge of the waist opening or at least part of the circumferential edge of each leg opening.

Fig. 34 is a schematic perspective illustration of a pull-on diaper as an embodiment of the pull-on absorbent article according to the present invention in a state worn by an infant.
The diaper of the present embodiment has a waist opening 101 and a pair of leg openings 104. The diaper 110 has a fluted panel having nearly circular openings open to at least part of the circumferential edge of the waist opening 101 or at least part of the circumferential edge of each leg opening 104. As used herein, the term "fluted panel" is a structure composed of two sheets 103a and 103b joined to each other and elasticized with elastic members to form folds 102a and 102b, respectively, the folds 102a and the folds 102b forming tubular spaces therebetween that are open at one or both longitudinal ends of the individual tubes. As will be described later, the folds 102a and the folds 102b are able to form vacant spaces between themselves and the skin and between themselves and a garment, respectively. The folds 102a and 102b are able to freely move and change in shape without being restrained by the elastic members to let air in and out as if they breathe.

The pull-on diaper (pull-on article) of the present embodiment has the fluted panel in such a configuration that the open ends of the fluted panel are open around at least part of the waist opening edge or at least part of the leg opening edge. It is preferred that the fluted panel be disposed such that the open ends thereof are open around edges of both the waist opening and the leg openings. It is preferred that the fluted panels be disposed such that the whole circumference of the waist opening edge is defined by arrays of the openings of the fluted panels as with the case of the diaper 110 shown in Fig. 34. In the present embodiment, the fluted panel forming the front body portion of the diaper 110 has its folds 102a and 102b ended along a border line 107. The diaper 110 has an absorbent body 131 disposed inside the outer cover 102 in a portion 111 below the border line 107. It is preferred that the inner sheet 103a and the outer sheet 103b in the portion 111, i.e., the area where the absorbent body 131 is disposed not be bonded to each other at other than the perimeter of the portion 111. It is preferred that the below-waist elastic members be fixed along both side edges of the portion 111 and that the part of the elastic members within the area where the absorbent body is disposed be rendered substantially functionless, specifically by being cut or segmentalized. With the elastic members rendered functionless in the portion 111, the absorbent body 131 is prevented from wrinkling and allowed to exhibit its absorbing performance.

As in the present embodiment, the fluted panel is preferably disposed in the vicinity of each sidebone portion 105 with a length such that the folds 102 extend from the edge of the waist opening 101 to a part 104a of the leg opening edge and with a width such that the array of the folds reaches the border line 108 adjoining the portion 111 where the absorbent body is located. While Fig. 34 illustrates the front body portion 133 of the diaper, the positional relationship between the fluted panel and the portion 111 in which the absorbent body is fixed in the front body portion 133 applies to the back body portion 134 of the diaper.

As described, the diaper of the present embodiment has the fluted panel disposed in such a configuration as to improve the diaper functions comprehensively. More specifically, the fluted panel is not disposed in an area where a body fluid discharge is concentrated so as not to impede the absorptivity of the absorbent body but in other areas where weight is put on feel to the touch and outer appearance, i.e., the below-waist portion and the sidebone portion to provide these portions with longitudinally extending, soft folds. Beside this configuration, the elastic members are ingeniously arranged to improve the stretchability of the article as a whole thereby to provide enhanced comfort during wear. In other words, the fluted panel is disposed so as to enhance the functions of the individual components taking into consideration the three-dimensional structure of a pull-on type article. The above mentioned effects of the fluted panel in improving various characteristics or functions required of pull-on type articles are not achievable with conventional breathable composite stretch panels merely disposed in parts of the articles.

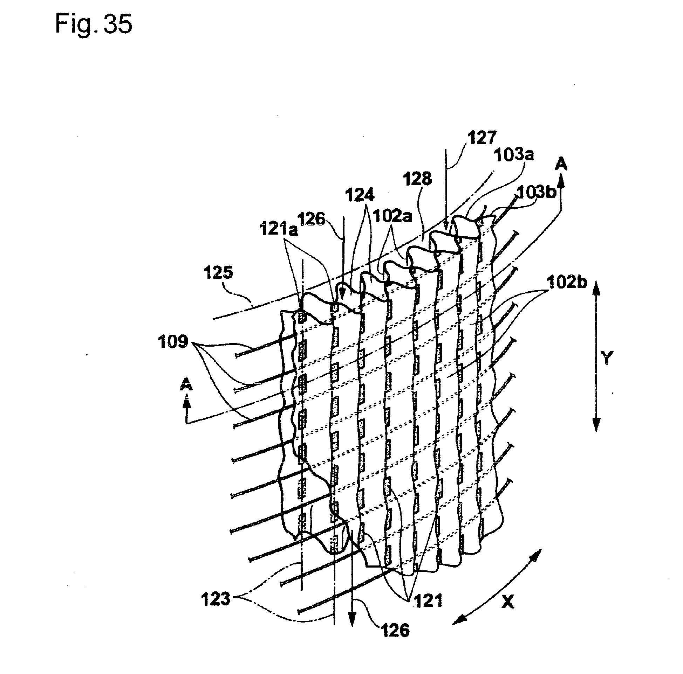

Fig. 35 is an enlarged fragmental perspective of the part indicated by Roman numeral II in Fig. 34 with part cut away. The fluted panel will be described in more detail with reference to Fig. 35. The fluted panel used in the diaper of the present embodiment has the elastic members 109 arranged to extend in the direction substantially perpendicular to the folds 102 between the inner sheet 103a and the outer sheet 103b, the inner and the outer sheets being joined at the bonds 121 that are discretely provided at such positions not to fix any elastic member 109. Each of the sheets 103a and 103b is nonwoven fabric. The discretely provided bonds 121 at which the sheets 103a and 103b are bonded are formed by heat embossing at such positions not to overlap any elastic member 109. The inside folds 102a and the outside folds 102b face oppositely to make a fluted structure as illustrated. The bonds (debossed regions) 121 are lined up in the vertical direction to form rows of lines 123, each line 123 running in the valley between adjacent folds 102 across the elastic members 109. For example, the debossed regions make a line on the individual lines 123. The lines of the debossed regions (lines 123) form valleys between folds, thereby making a neatly fluted structure. In the present invention, the line of debossed regions (bonds) that defines the valley between adjacent folds will be referred to as a seal line. While the seal line may be any line formed of at least two bonds as long as a fluted structure is obtained, it is preferred that every seal line run perpendicularly to the elastic members so that folds may continuously extend perpendicularly to the stretch direction of the elastic members. The individual bonds making each seal line may be formed between adjacent elastic members as illustrated in Fig. 35 or for every two or more elastic members. The seal line may be straight, curved, or piecewise straight.

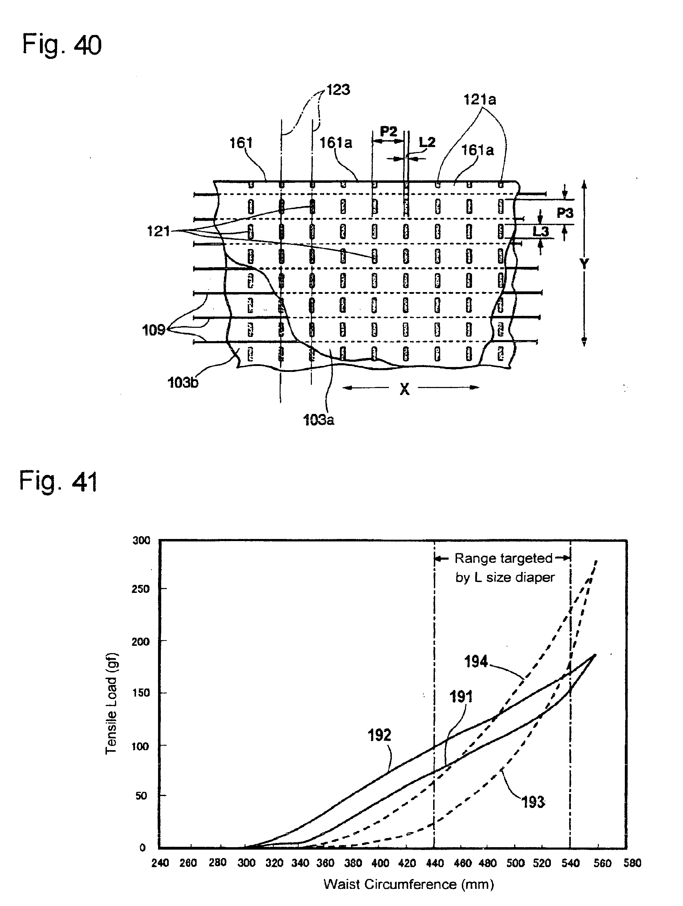

It is preferred for the fluted panel to form nearly circular neat openings at one or both ends thereof open on the diaper edge, such as the waist opening edge. As used herein, the term "nearly circular" is intended to include not only a true circle and an oval but also other rounded closed loops, such as a rounded rectangle and a rounded rhombus. In order for the fluted panel to have an edge with such neat openings, the basal parts of the folds (102a or 102b) formed of one of the sheets and those of the folds (102b or 102a) formed of the other sheet should be bonded to each other in pairs at the bonds 121a along the edge of the fluted panel thereby to make the openings 124 as illustrated. The edge treatment for forming an array of such neat openings 124 along the sheet edge will be described later with reference to Fig. 40.

While a diaper is worn by, for instance, an infant and deformed with the wearer's motion (e.g., changing a posture or breathing), the fluted panel changes in shape to let air in and out from the openings 124 of the tubes in the direction indicated by arrow 126 or in the opposite direction as if it breathes along with the wearer's movement. As a result, good ventilation is obtained. It is desirable that the tubes of the fluted panel be also open at the other end thereof so that the air flowing in the direction of the arrow 126 is let out from the other opening to provide a better ventilation. Similar ventilation is also obtained through the tubular spaces defined by the inner folds of the fluted panel and the wearer's body. For instance, air is let to flow through the openings 128 in the direction indicated by arrow 127 or in the opposite direction, whereby overhydration inside the diaper is prevented to protect the infant's skin from a diaper rash.

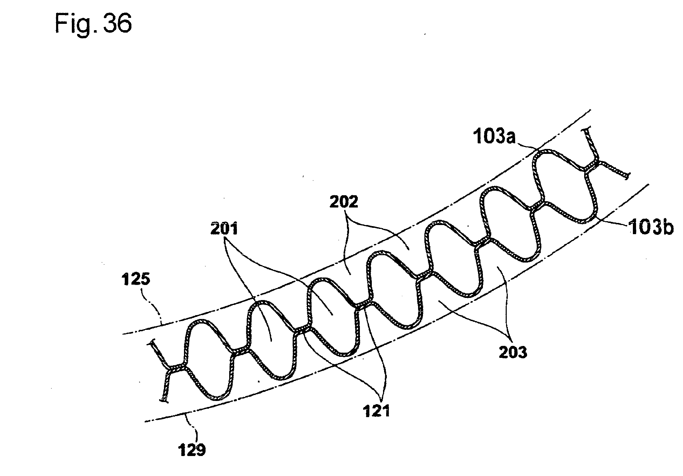

Fig. 36 is an enlarged cross-section taken along line A-A of Fig. 35. As is understood from Fig. 36, the fluted panel used in the pull-on article of the present invention forms tubular vacant spaces 201 between the inner and the outer sheets 103a and 103b, tubular vacant spaces 203 between the inner sheet 103a and the wearer's body, and, in addition, tubular vacant spaces 203 between the outer sheet 103b and a garment 129 put on over the diaper. Hence, the pull-on article of the invention has an advantage of providing various paths for ventilation to achieve satisfactory breathability.

In the present embodiment, the folds 102 formed by the sheet 103a and those formed by the sheet 103b protrude outward in opposite directions. Each fold 102 is defined by a curved surface and has an arcuate cross-section. The curved surface continuously extends in the direction (direction Y) perpendicular to the stretch direction (direction X). As shown in Fig. 35, in the present embodiment, one fold is formed between adjacent seal lines 123, and the fold 102 extends continuously across the elastic members 109 without being obstructed by the elastic members 109. A number of such folds 102 are arrayed to make a fluted structure with an airy neat appearance as a whole.

As shown in Fig. 35, the elastic members 109 run between valleys of the folds of the sheet 103a and valleys of the folds of the sheet 103b and fixed to neither the sheet 103a nor the sheet 103b at any positions including the positions between the valleys mentioned above, and the sheets 103a and 103b are discretely bonded at the bonds 121 (they are not bonded continuously). Therefore, as compared with a conventional composite stretch panel having a sheet material and elastic members fixed to each other and integrally gathering, the fluted panel has reduced stiffness and increased freedom of deforming its folds against outer force. In addition, the fluted panel has a voluminous hand in its thickness direction and a soft and pleasant feel to the touch.

Since the sheets 103a and 103b are bonded discretely, the amount of an adhesive to be used is reduced. When, in particular, the sheets 103a and 103b are bonded by heat fusion as in the present embodiment, there is produced a more remarkable effect in reducing the amount of an adhesive used in the diaper. The number of the materials necessary to make the fluted panel is thus decreased, which leads to reduction in the number of the processing steps involved, to achieve improved production efficiency. The resulting product exhibits better breathability and higher moisture permeability by virtue of the reduction of regions having a hot melt adhesive.

In the diaper 110 of the present invention, the elastic members are fixed to the inner sheet 103a and the outer sheet 103b at part of the circumferential edge of the leg opening 104 illustrated in Fig. 34. The part at which the elastic member is thus fixed will also be referred to as a leg opening fixing part. The elastic members may be fixed at other than the mentioned leg opening fixing part as long as the excellent effects of the fluted structure are not impaired. For example, the elastic members in the present embodiment are fixed near the border line 108 and in the sidebone portions (side seals) 105 illustrated in Fig. 34. The elastic members disposed near the waist opening 101 (the elastic members 109 arranged in the area between the border line 107 and the edge of the waist opening 101) have no fixed points at other than the side seals 105. It is preferred in the present invention that the elastic members not be fixed by two or more debossed regions (bonds) proximate to each other. By minimizing the number of fixing points of the elastic members, the stretch characteristics essentially possessed by the elastic members are made effective use of in the diaper to provide good comfort, as will be discussed later.

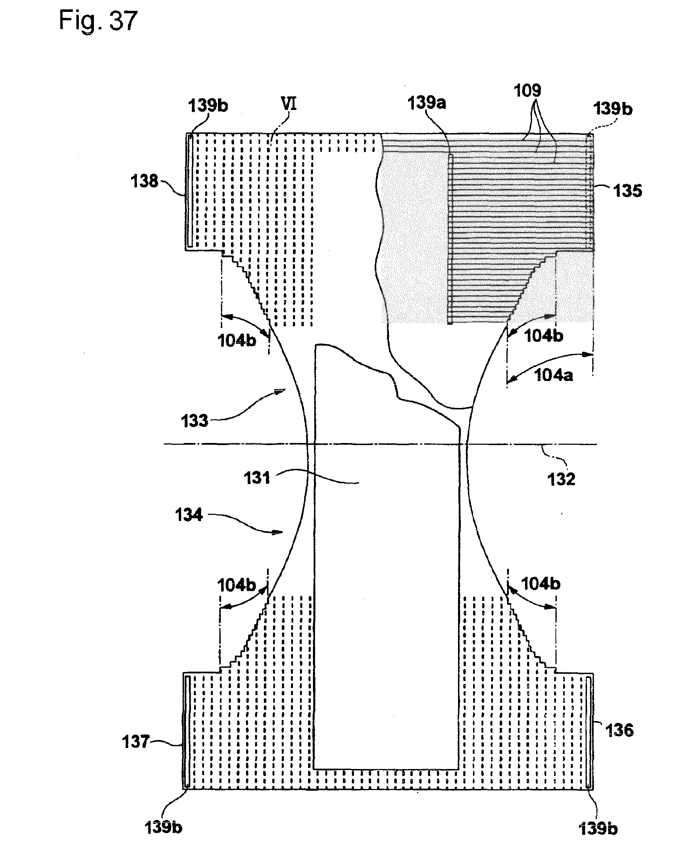

Fig. 37 is a schematic plan of the inner side of the pull-on diaper of Fig. 34 in its flat-out, uncontracted state with part cut away, which shows where the elastic members are fixed. The elastic members are in their stretched state to display a two-dimensional view.

In an unfolded and uncontracted state as illustrated, the diaper of the present embodiment is equal to an intermediate assembly of sheet form that is to be further processed into a finished diaper. In the manufacture of the diaper, such an intermediate assembly of sheet form as prepared is softly folded about a valley fold 132 with the elastic members 109 in their stretched state so that the front body portion 133 and the back body portion 134 may face each other. The facing side edges 135 and 136 and the facing side edges 137 and 138 are joined together, respectively, to make a pull-on diaper illustrated in Fig. 35. Other members may be added where needed, but it is preferred not to provide a leg gather or a turnback around the leg opening edge or the waist opening edge.

The plan view of Fig. 37 has its part cut away to show the elastic members 109 being disposed between the inner sheet and the outer sheet. An absorbent body 131 is placed on the inner sheet. The end of each elastic member located at leg opening fixing parts 104b is fixed to the inner and the outer sheets. Some elastic members have their end fixed at a fixing part 139a near the border line 108 as stated above. Some elastic members have their end fixed at a fixing part 139b near the side edges 135, 136, 137, or 138.

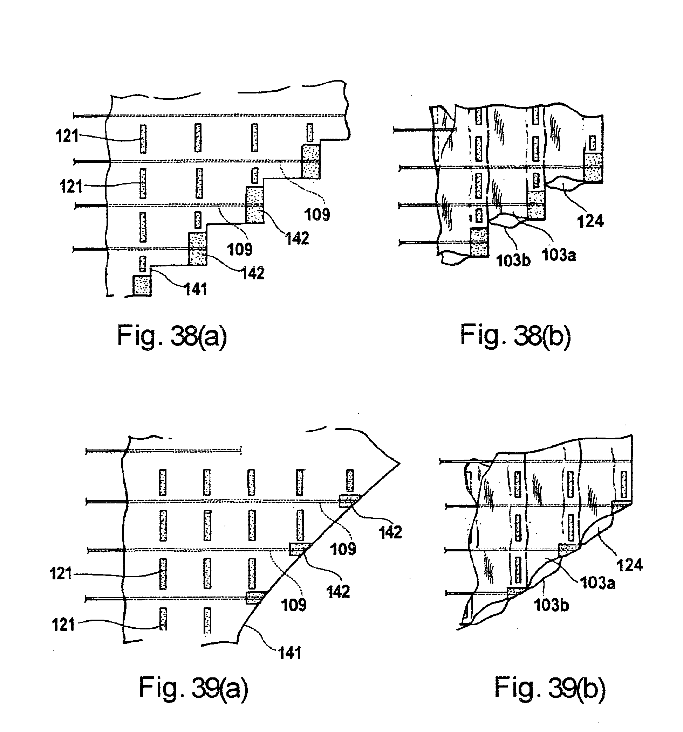

The fixed state of the elastic members 109 at the leg opening fixing part 104b will be described in greater detail. Fig. 38(a) schematically illustrates a preferred fixing form 1 at the leg opening fixing part. It is an enlarged fragmental plan of the leg opening fixing part 104b of the plan shown in Fig. 37. In the fixing form I, the leg opening has a stepped edge 141, and each elastic member 109 has its end fixed at a fixing part 142 provided on each step riser. Fixing is preferably carried out by, though non-limitedly, heat pressing predetermined positions of the elastic members between the inner and the outer sheets. As previously described, the elastic members 109 are disposed not to pass through the discretely arranged debossed regions (bonds 121).

Fig. 38(b) is a schematic perspective of the part shown in Fig. 38(a) (in a flat-out, uncontracted state) that is in a contracted and therefore fluted state while worn. It is thus understood that the elastic members fixed in the fixing form I retract to cause the inner sheet 103a and the outer sheet 103b to gather, making nearly circular openings 124.

Fig. 39(a) is an enlarged plan schematically illustrates another preferred fixing form 2 at the leg opening fixing part. It is an enlarged illustration of the leg opening fixing part 104b shown in Fig. 37. According to this fixing form 2, the edge 141 of the leg opening fixing part is treated to have a gentle curve. The elastic members 109 are fixed at the fixing parts 142. The method of fixing the elastic members 109 and the positional relationship between the elastic members 109 and the debossed bonds 121 are the same as with the fixing form 1.

Fig. 39(b) is a schematic perspective of the part shown in Fig. 39(a) with the elastic members 109 retracted. In the case of the fixing form 2, there is formed a good fluted structure having nearly circular openings 124 obliquely arrayed along the gentle curve of the leg opening edge.

It is preferred that each fixing part 142 in the fixing forms 1 and 2 be positioned on the line 123 of the bonds 121, thereby to form openings of a fluted panel along the leg opening edge.

A preferred structure of the fluted panel will be described in detail.

Fig. 40 is an enlarged plan of region VI of the diaper shown in Fig. 37. In the present embodiment, the inner sheet 103a and the outer sheet 103b are discretely bonded to each other at fusion bonds 121 arrayed in both the longitudinal direction of the elastic members 109 (direction X) and the direction (direction Y) perpendicular thereto. The elastic members 109 are arranged in a parallel relation relative to one another. In the case where a plurality of elastic members are in a parallel relation relative to one another as in the present embodiment, the elastic members in that region stretch in the body circumferential direction (direction X), and the seal lines 123 extend in the body longitudinal direction (direction Y). The pattern of arranging the bonds 121 is not limited to the mentioned arrangement, i.e., a rectangular lattice pattern, and may be a diamond lattice pattern, a staggered pattern, and the like.

It is preferred in the present embodiment that the pitch P3 of the bonds 121 in direction Y be 1 to 40 mm, more preferably 2 to 15 mm; that the length L3 of the individual bonds 121 be 0.5 to 20 mm, more preferably 1 to 10 mm in direction Y; and that the ratio of the pitch P3 to the length L3 (P3/L3) be in a range of from 1.05 to 80, more preferably of from 1.05 to 15.

In order to assure formation of folds continuously extending over a plurality of the elastic members, it is preferred that the bonds be arranged at a pitch P2 of 1 to 30 mm, more preferably 2 to 15 mm; that the individual bonds have a length L2 of 0.1 to 5 mm, more preferably 0.2 to 1.5 mm; and that the ratio of the pitch P2 to the length L2 (P2/L2) be in the range of from 1.1 to 300, more preferably from 4 to 100.

It is preferred that the seal lines 123 that form the folds 102 (see Fig. 34) of the fluted panel be continuous over a length crossing with at least three elastic members 109. The number of the folds formed in a diaper is decided as appropriate to the size and use of the diaper, taking into consideration the pitches P2 and P3 and lengths L2 and L3 of the bonds and the pitch-to-length ratios P2/L2 and P3/L3. The number of the elastic members 9 used in a diaper is decided appropriately according to the diaper size (the body weight and average amount of bodily waste, such as urine, of targeted wearers), the side seal length, and the shape of the leg openings. The pattern of arranging the bonds 121 may be altered as appropriate. The individual bonds 121 may have an appropriate shape, such as a rectangle, an oval, a circle, or a rhombus.

In the present embodiment, the waist opening edge 161 (see Fig. 40) is obtained as a result of cutting along a line passing through the bonds 121a. By such an edge treatment, each open end 161a of the folds of the fluted panel has its both sides fixed at the bonds 121a so as to form a neat, nearly circular opening 124 (see Figs. 34 and 35) while the diaper is worn.

The above described materials of the nonwoven fabrics providing the upper sheet and the lower sheet and the elastic members 41 may be used in the present embodiment as well with no particular limitation.

In order for the fluted panel of the pull-on article according to the present embodiment to form protruding folds with a satisfactory cross-section, it is preferred to use elastic members having a prescribed stretch ratio and stretch stress. The elastic members are preferably fixed to sheet materials in a 20% to 1000% extended state, more preferably in a 50% to 400% extended state. Upon the thus fixed elastic members retracting, the nonwoven fabrics gather and protrude outwardly (in opposing directions relative to their fusion bonded facing sides) to form folds.

The height of the folds is variable with fold forming properties and cushioning properties and can be designed freely by selecting the pattern of arrangement and the pitch of the bonds and the materials of the sheets and the elastic members. The height of the folds is preferably about 1 to 15 mm per side. High folds are formed by spacing the bonds at a sufficient distance and using elastic members having a stretch ratio enough to retract so as to bring adjacent bonds close to form folds with a desired height. A fluted panel feeling soft and voluminous is thus obtained. To form folds with a height h on one side, the distance between adjacent bonds must be at least 2h. When the distance is just 2h, i.e., the minimum for making 2h high folds, the elastic members must contract such that the adjacent bonds may almost come into contact with each other.

Among preferred materials of the elastic members is natural (or synthetic) rubber. An elastic member made of natural (or synthetic) rubber may have a thickness of 0.05 to 1.5 mm and a width of 0.2 to 5 mm and preferably has a low modulus. A preferred modulus is, while depending on the cross-sectional area, such that a monofilament with a thickness of 0.35 mm and a width of 0.91 mm has a stress at 100% elongation of about 1 to 70 gf, more preferably about 1 to 40 gf, even more preferably about 1 to 30 gf.

Also included in preferred elastic members is Spandex fiber (elastic polyurethane fiber). A monofilamentous Spandex fiber having a fineness preferably of 10 to 3360 denier, more preferably of 70 to 1120 denier, is used. "Denier" is a unit of thickness of a yarn. A yarn weighing one gram per 9,000 meters is one denier. A plurality of such Spandex fibers are used as stretched at a stretch ratio of 30% to 500%.

A fluted panel that softly stretches and retracts to form neat and nice folds can be obtained by disposing a plurality of monofilaments of the above-described low modulus elastic member at a high stretch ratio preferably of 100% or higher, more preferably of 200% or higher.

The pull-on article of the present embodiment exhibits excellent stretch characteristics because of the effective use of the fluted panel. Fig. 41 is a graph showing the relation between waist circumference (mm) and tensile load (gf) of the diaper shown in Fig. 34. This diaper has a waist circumference (the circumference at the waist opening edge) of 300 mm in a relaxed state (the state before application of tension). As a tension is applied from the inside of the waist opening edge in a direction to widen the circumference, a solid curve 192 is plotted. As the tension is decreased, a solid curve 191 is depicted. On the other hand, when the same test is carried on a conventional diaper (a commercially available elasticized, but not fluted, diaper from Company A), there are plotted dotted curves 193 and 194. Taking a look at, for instance, the region between waist circumferences 440 mm and 540 mm (the region between the dashed dotted lines), which is a range targeted by L size diapers, it is seen that the diaper of the present embodiment has good stretch characteristics, showing a mild change in tensile load. That is, the rise of the stress-strain curve of the diaper of the present embodiment is not so steep as that of the conventional diaper. Taking into consideration prevention of sliding down during wear and of leaving marks, it is preferred that the solid curve 191 have a smaller slope, indicating a smaller change in tensile load.

The pull-on article of the present embodiment exhibits good stretchability and breathability, feels soft to the touch, gives comfort to a wearer, and has an attractive decorative appearance. Such a superior pull-on article as the present embodiment can be produced efficiently in a continuous manner without involving complicated steps in accordance with the method described below.

The method of producing the pull-on article includes the steps of feeding a web of an inner sheet and a web of an outer sheet while interposing a plurality of elastic members therebetween in a state stretched in the longitudinal direction of the webs, discretely bonding the webs together at other than positions where the elastic members extend to make a composite sheet web, and cutting out a central portion of the composite sheet web along a closed cutting line to make an opening. The step of making an opening is carried out as follows. Each of the elastic members intersecting with the cutting line is fixed to the inner sheet and/or the outer sheet at fixing points that are positioned outside the closed cutting line, proximate to the intersections between the elastic member and the cutting line, and on the seal lines of the bonds. The composite sheet is then cut along the cutting line passing inside each fixing point. The composite sheet having the resulting opening is cut so that the opening is divided into leg openings. The composite sheet is then formed into an article having the fluted panel.

The method of producing the pull-on article 110 will be described in more detail.

According to the method of this embodiment, a pull-on article having the above described fluted panel is produced efficiently and economically through a continuous series of steps.

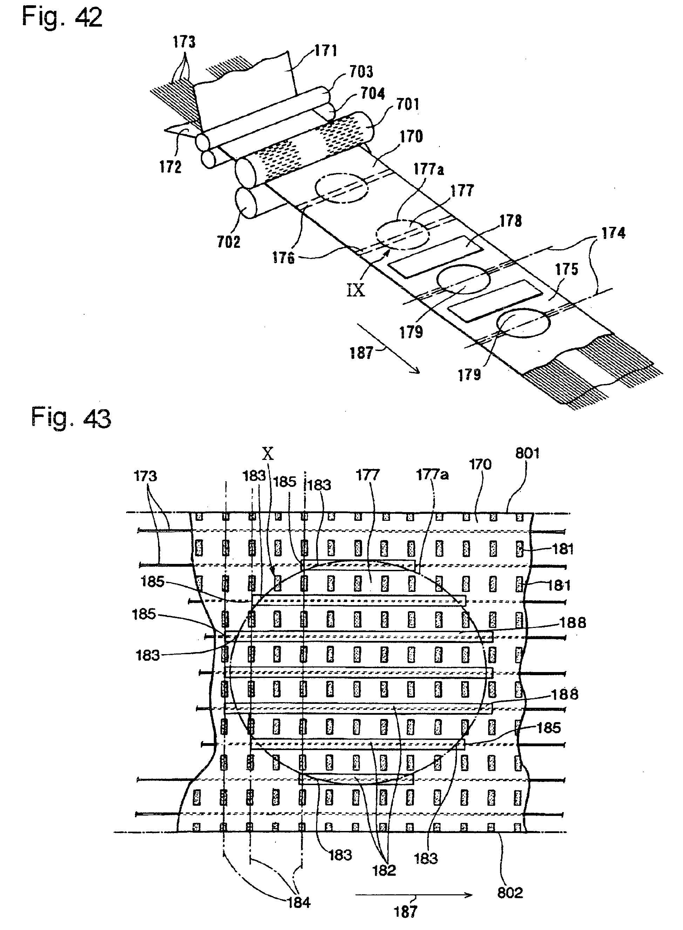

The method of the invention will be described in detail based on its preferred embodiment with reference to the accompanying drawing. Fig. 42 is a perspective view schematically illustrating part of the steps of the method according to the invention. As illustrated in Fig. 42, a plurality of elastic members 173 are arranged in parallel to each other in their stretched state on a moving sheet web 172, and another sheet web 171 is superposed on the elastic member side of the web 172. The two sheet webs are preferably passed between a pair of rollers 703 and 704 to be superposed on each other.

The resulting stack of the webs is discretely united together at other than positions where the elastic materials 173 are disposed preferably by fusion bonding by, for example, heat embossing or ultrasonic embossing to obtain a composite sheet web 170. Heat embossing or ultrasonic embossing is carried out by, for example, passing the stack between an embossing roller 701 having projections on its peripheral surface in a pattern corresponding to the pattern of fusion bonds to be formed and a backup roller 702. While the discrete bonding of the webs may be achieved with an adhesive, fusion bonding as described is preferred.

The elastic members 173 are then fixed to the sheet webs 171 and 172 only at necessary positions of the composite sheet web 170 (elastic member fixing step). The elastic member fixing step may be performed by heat embossing or ultrasonic embossing. The elastic member fixing step may also be conducted by applying an adhesive to either the two sheets or the elastic members 173 and pressing the adhesive-applied parts. The adhesive is preferably applied to, for example, the sheet 172 before disposing the elastic members 173 and the other sheet 171 before superposing on the sheet 172, or the elastic members 173 before or after being disposed on the sheet 172.

In the embodiment illustrated in Fig. 42, the elastic members are fixed at bonds 176. After additional necessary steps (including the steps of cutting to make an opening 177 and attaching an absorbent member 178), the composite sheet web is cut along cutting lines 174 within the respective bonds 176 to make an intermediate assembly 175 which, if relaxed from tension, gathers to form folds in predetermined areas to provide the fluted panels. The intermediate assembly 175 in its flat-out, uncontracted state is equal to the flat-out, uncontracted state of the diaper shown in Fig. 37. As previously described, the intermediate assembly 175 is softly folded about the valley fold 132 (see Fig. 37), and sealed along the side edges 135, 136, 137, and 138 (see Fig. 37) to complete a diaper of the embodiment shown in Fig. 34.

Making the opening 177 will be described.

The opening 177 is to be divided into half moon-shaped halves 179 when the composite sheet web is cut into individual intermediate assemblies 175. Each half 179 of the opening 177 becomes one of a pair of leg openings 104 (see Fig. 34) of a finished diaper. The opening 177 is made by cutting the composite sheet along a cutting line 177a. The manner of cutting will be described in detail by way of Fig. 43, in which an enlarged plan of region IX, inclusive of the portion that is to be cut off to make the opening 177, of Fig. 42 is shown. For the sake of easier understanding, Fig. 43 depicts a small number (nine) of elastic members being arranged in equally spaced, parallel relation. In fact, however, it is preferred that a greater number of (e.g., 20 to 50) elastic members are arranged in other than the middle portion of the web to obtain the diaper of the embodiment illustrated in Fig. 34.

Before cutting, the elastic members 173 crossing the opening 177 are fixed to both the sheets 171 and 172 at fixing points 185 any of which is outside the opening 177, on a seal line 184, and proximate to an intersection 183 between the cutting line 177a and the respective elastic members 173. The elastic member 173 crossing the opening 177 are each fixed continuously between the opposite two intersections with the cutting line 177a, i.e., between the two fixing points 185 which are on the respective seal line. That is, the elastic members across the opening 177 are fixed continuously in the bonds 188. Previously providing the bonds 188 having a certain length in the machine direction 187 makes it easy to provide the fixing bonds in a continuous production line.

The method of fixing the elastic members is not particularly limited and may be application of an adhesive or heat fusion.

The absorbent member 178 is attached to the inner sheet (sheet 171 in Fig. 42) using an adhesive.

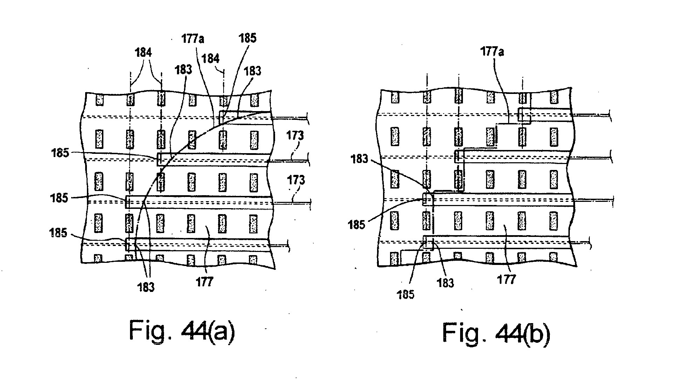

The composite sheet is then punched along the cutting line 177a to make the opening 177. The edge of the opening is described in more detail by way of Fig. 44(a). Fig. 44(a) is a further enlarged plan of the region X of Fig. 43. In this embodiment of cutting, the cutting line 177a is a gently curving line. Cutting along such a curving line results in the formation of a gently curving leg opening edge as depicted in Fig. 39(a). Every elastic member 173 in Fig. 44 is fixed on a seal line 184. On making the assembly into a diaper, the opening 177 resulting from the cutting becomes a leg opening, and the fixing points 185 become the fixing points 142 (see Fig. 39). By arranging these fixing parts accurately on the seal lines, the composite sheet, when fabricated into a diaper, will be a fluted panel forming satisfactory open ends between the seal lines as illustrated in Fig. 39(b).

When the cutting line is step-like as in Fig. 44(b), the opening edge will be a stepped edge having fixing points 85 therealong to provide a fluted panel with a stepped edge shown in Figs. 38(a) and 38(b).

In the above described method of diaper production, the edges 801 and 802 (see Fig. 43) of the composite sheet are preferably treated to be a chain of the debossed bonds 181. By such an edge treatment, there is provided a fluted panel having the upper sheet 171 and the lower sheet 172 secured by the debossed bonds along their edge 801 (which will form the waist opening 101 of Fig. 34) and edge 801 thereby to form neat openings 124 (see Fig. 34) along these edges when fabricated into a diaper. Such a treatment of the edges 801 and 802 may be achieved either by arranging debossed bonds on the edges 801 and 802 when the stack of the sheet webs is embossed using the embossing roller 701 (see Fig. 42) or by trimming each edge along a line connecting the debossed bonds.

Hence, the diaper production method of the invention makes it feasible to produce a diaper having satisfactory fluted panels in an efficient and continuous manner through a sequence of steps. After making the intermediate assembly 175, there is no need to add any complicated step.

While the present invention has been described with several preferred embodiments thereof, the invention is not construed as being limited thereto.

For example, the composite stretch panel 4 may be used to form other than the below-waist gather G1 and the waist gather G2.

The composite stretch panel 4 forming the below-waist gather G1 may be used in only the regions laterally outward of the absorbent body 3.

The composite stretch panel 4 may be used to make a gather in the crotch section C, for example, leg gathers G3 or the frontal crotch gather G4. In the case where the gather in the crotch section C is formed of the composite stretch panel 4, it is preferred that there be continuity between the folds 43 of the composite stretch panel 4 applied to the crotch section and the folds of the composite stretch panel 4 of the below-waist gather G1 in the longitudinal direction of the outer cover 2. It is more preferred that the continuous folds 43 extend to the leg openings 13.

The outer cover 2 may be a laminate of the outer sheet 21 of the same contour as the outer cover 2 and two inner sheets 22 spacedly disposed, one in the front section A and the other in the rear section B. in this modification, the crotch section C of the outer cover 2 is formed solely of the outer sheet 21.

The outer sheet 21 may be composed of three separate sheets, one forming the front section A, one forming the crotch section C, and one forming the rear section B, connected in the longitudinal direction of the outer sheet 21. The same may apply to the inner sheet 22.

The present invention may be applied not only disposable pull-on diapers but disposable open style diaper with fasteners. The invention is also applicable to pull-on (pants type) sanitary napkins and the like.

While the folds 43 of the composite stretch panel 4 in the diaper 1 or 1A are continuous between two elastic members 42 located along opposite edges in the direction (direction Y) perpendicular to the stretch direction of the composite stretch panel 4, the elastic members 42 located near the opposite edges in direction Y may be bonded to the outer sheet 21 and the inner sheet 22. In this case, nevertheless, it is desirable that the folds 43 be continuous over a length crossing with at least three, preferably at least a half, more preferably 70% or more, of the total number of the elastic members 42. The elastic member 42 bonded to the sheets 21 and 22 may have its end(s) located inboard of the end portion(s) 44 of the composite stretch panel 4 in the stretch direction (direction X).

The number of the elastic members 42 used in the composite stretch panel 4 is decided appropriately according to the size and use of the composite stretch panel 4 and may be, for example, 5 to 30. The bonds 41 may be formed by not only fusion bonding but ultrasonic bonding or applying an adhesive, such as a hot melt adhesive.

The pattern of arranging the bonds 41 may be changed appropriately. The individual bonds 41 may have an appropriate shape, such as a rectangle, an oval, a circle, or a rhombus.

While in the second to fifth embodiments the holding sheet 27 is integral with the outer sheet 21, they may be formed of separate sheets. In the latter case, the holding sheet 27 is preferably disposed with its upper edge (the edge farther from the crotch section C) aligned with the longitudinal end edge of the outer sheet 21. When the holding sheet 27 is a separate sheet, too, the part of the holding sheet that overlaps the longitudinal end portion of the absorbent body 3 is bonded to that portion of the absorbent body 3 over the whole width of that portion with an adhesive so that the longitudinal end portion of the absorbent body 3 is secured to the outer cover 2.

The outer sheet 21 and the inner sheet 22 do not need to be separate sheets. A single sheet may be folded in two facing panels, one serving as the outer sheet 21, and the other as the inner sheet 22.

The outer cover 2 may be a laminate of the outer sheet 21 of the same contour as the outer cover 2 and two inner sheets 22 spacedly disposed, one in the front section A and the other in the rear section B. In this modification, the crotch section C of the outer cover 2 is formed solely of the outer sheet 21.

The outer sheet 21 may be composed of three separate sheets, one forming the front section A, one forming the crotch section C, and one forming the rear section B, connected in the longitudinal direction of the outer sheet 21. The same may apply to the inner sheet 22.

In the second to fifth embodiments, the skin contacting surface-defining hydrophobic sheet, the hydrophilic sheet, and the garment facing surface-defining hydrophobic sheet are provided in the waist flap F of each of the front section and the rear section, and the composite stretch panel 4 gathers to form the waist gather G2 in each of the front section and the rear section. Such a structure may be present only in the waist flap F of the rear section.

The absorbent article according to the invention may be dispensed with the outer cover 2 bonded to the garment facing side of the absorbent body 3. For example, the topsheet and the backsheet covering the top side and the back side of the absorbent core, respectively, may be extended longitudinally outward of the absorbent core to form the waist flaps.

In the description given above, particulars of a certain embodiment that have been omitted to avoid redundancy can appropriately be complemented by the corresponding description of other embodiments. Particulars described as being characteristic of a certain embodiment can apply to other embodiments appropriately. Particulars of every embodiment are appropriately interchangeable between embodiments.

Examples

The invention will now be illustrated in greater detail, but it should be understood that the invention is not deemed to be limited thereto.

Example 1

Example 1 incorporating the second embodiment of the invention was prepared. A hydrophobic through-air nonwoven fabric made of PE sheath/PET core conjugate fibers and having a basis weight of 20 g/m$^2$ was used as an outer sheet 21 and a holding sheet 27. A hydrophobic spunbonded nonwoven fabric made of PE sheath/PET core conjugate fibers and having a basis weight of 16 g/m$^2$ was used as an inner sheet 22. A hydrophilized SMMS nonwoven fabric made of PP fibers and having a basis weight of 10 g/m$^2$ was used as a hydrophilic sheet 29 independent of the inner sheet 22.

In Example 1, the outer sheet 21 and the inner sheet 22 were bonded together via bonds 41 that were discretely arranged in both the stretch direction of the composite stretch panel 4 and the direction perpendicular thereto. The hydrophilic sheet 29 was disposed on the absorbent body facing side of the inner sheet 22 in the areas corresponding to the upper below-waist subportions D 1 and the waist portions F. The hydrophilic sheet 29 was bonded on the entire area thereof to the inner sheet 22 with a hot melt adhesive. The hydrophilic sheet 29 and the holding sheet 27 were bonded over the entire facing area thereof with a hot melt adhesive.

Example 2

Example 2 incorporating the fifth embodiment of the invention was prepared. The same nonwoven fabric as the outer sheet 21 of Example 1 was used as the outer sheet 21 and the holding sheet 27. Unlike Example 1, a hydrophilic sheet 29 was not provided in the form of a sheet independent of the inner sheet 22. That is, the inner sheet 22 was totally formed of the hydrophilic sheet 29. The same nonwoven fabric as the hydrophilic sheet 29 of Example 1 was used as the hydrophilic sheet 29 of Example 2. Example 2 was otherwise the same as Example 1.

Example 3

An alteration was added to Example 1 to prepare Example 3. Example 3 is the same as Example 1, except for using a hydroentangled nonwoven fabric made of 70 wt% rayon and 30 wt% PET and having a basis weight of 40 g/m$^2$ as the hydrophilic sheet 29.

Reference Example 1

A modification was added to Example 2 to prepare Reference Example 1. Reference Example 1 is the same as Example 2, except that the same hydrophobic sheet as used as the inner sheet 22 in Example 1 was used as an inner sheet 22. The composite stretch panel of Reference Example 1 was formed of two hydrophobic sheets (outer sheet 21 and the inner sheet 22) facing each other.

Reference Example 2

A modification was added to Example 2 to prepare Reference Example 2. Reference Example 2 is the same as Example 2, except that that outer sheet 21 and the inner sheet 22 were bonded all over to each other. Reference Example 2 is a comparative example in which the outer sheet 21 and the inner sheet 22 failed to form a composite stretch panel 4 on account of their allover adhesion.

Reference Example 3

A modification was added to Reference Example 2 to make Reference Example 3. Reference Example 3 is the same as Reference Example 2, except that the nonwoven fabric used as the inner sheet 22 in Reference Example 1 was used as the inner sheet 22. Reference Example 3 is for reference in that the outer sheet 21 and the inner sheet 22 failed to form a composite stretch panel 4 on account of their allover adhesion and that a hydrophilic sheet was not provided.

Examples and Reference Examples prepared above were evaluated for (1) fluid wicking ability, (2) fluid retentivity, (3) single pour quick-drying properties, and (4) repeated pour quick-drying properties in accordance with the previously described method of measuring fluid spread area, the previously described method of measuring fluid retention, a method of measuring drying time (single pour) described below, and a method of measuring drying time (repeated pour) described below, respectively. The fluid wicking ability and the fluid retentivity were measured on the hydrophilic sheet alone.

Method of measuring drying time (single pour):

A sample cut out of the waist portion F is fixed on its holding sheet side to a hot plate in its stretched out state. Water (0.1 ml) is syringed into between the holding sheet 27 and the hot plate, and the sample is pressed down with a load of 1.0 kPa for about 5 seconds. The time required for the sample to dry (drying time) is measured. The BT plate of KES-F7 Thermo Labo II type from Kato Tech Co., Ltd. (10 x 10 $cm^2$; 33°C) is used as a hot plate. The measuring environment is 23°C, 50% RH, and a wind speed of 1.0 m/sec.

The drying time is obtained from the amount of heat dissipation of the BT plate. After water supply, the amount of heat dissipation increases. After all water evaporates, i.e., the sample dries, the amount of heat dissipation reduces to the value before water supply. Accordingly, the time after the water supply to the return to the heat discharge before water supply is taken as a drying time (single pour).

Method of measuring drying time (repeated pour):

A sample is fixed on its holding sheet side to a hot plate in its stretched state in the same manner as in the measurement of drying time (single pour). Water (0.1 ml) is syringed into between the holding sheet 27 of the sample and the hot plate, and the sample is pressed down with a load of 1.0 kPa for about 5 seconds. Five minutes later, 0.1 ml of water is again syringed. Thereafter, water is repeatedly supplied every 5 minutes to supply a total of 0.5 ml of water. The time required for the sample to dry (drying time (repeated pour)) is measured.

The fluid spread area, fluid retention, drying time (single pour), and drying time (repeated pour) of each of Examples and Reference Examples as measured by the method of measuring fluid spread area, the method of measuring fluid retention, the method of measuring drying time (single pour), and the method of measuring drying time (repeated pour), respectively, are shown in Table 1 below.

Table 1

| | Structure | Hydrophilic Sheet | Composite Stretch Panel | Fluid Spread Area ($cm^2$) | Fluid Retention ($g/cm^2$) | Drying Time (Single Pour) (sec) | Drying Time (Repeated Pour) (sec) |
|---|---|---|---|---|---|---|---|
| Example 1 | same as 2nd embodiment | SMMS nonwoven of PP (separate from inner sheet) | yes | 7.5 | 0 | 11 | 9 |
| Example 2 | same as 5th embodiment | SMMS nonwoven of PP (as inner sheet) | yes | 7.5 | 0 | 11 | 13 |
| Example 3 | same as 2nd embodiment | rayon/PET hydroentangled nonwoven (separate from inner sheet) | yes | 3.9 | 47 | 16 | 23 |
| Reference Example 1 | same as 5th embodiment | none (inner sheet is hydrophobic nonwoven) | yes | 0.2 | 0 | 28 | 69 |

(continued)

|  | Structure | Hydrophilic Sheet | Composite Stretch Panel | Fluid Spread Area $(cm^2)$ | Fluid Retention $(g/cm^2)$ | Drying Time (Single Pour) (sec) | Drying Time (Repeated Pour) (sec) |
|---|---|---|---|---|---|---|---|
| Reference Example 2 | do. | SMMS nonwoven of PP (as inner sheet) | no (allover adhesion) | 7.5 | 0 | 9 | 19 |
| Reference Example 3 | do. | none (inner sheet is hydrophobic nonwoven) | no (allover adhesion) | 0.2 | 0 | 46 | 76 |

The following conclusions are drawn from the results in Table 1. The results of Examples 1 to 3 and Reference Example 2 reveal that a waist portion having a hydrophilic sheet between the skin contacting surface-defining sheet (i.e., the holding sheet 27) and a garment facing surface-defining sheet (i.e., the outer sheet 21) achieves smooth evaporation of sweat absorbed thereby, namely, exhibits rapid-drying properties. The results of Examples 1 to 3 show that use of a hydrophilic sheet exhibiting higher fluid wicking ability and lower fluid retentivity has a longer lasting effect in rapid drying even when the waist portion experiences sweating repeatedly. It is also seen from the results of Examples 1 and 2 in comparison with Reference Example 2 that provision of a composite stretch panel having discrete bonds in the waist portion brings about improved sweat vaporization properties. It is also seen mainly from the results of Reference Examples 1 and 3 that a waist portion having no hydrophilic sheet, i.e., composed of only hydrophobic nonwoven fabrics exhibits no quick-drying effect regardless of whether a composite stretch panel is used or not.

Example 4

A through-air nonwoven fabric was prepared by an air-through process using conjugate fibers having a PE sheath and a PET core. The resulting nonwoven fabric was used as an outer sheet 21 in accordance with the embodiment illustrated in Figs. 1 through 4. This nonwoven fabric was not embossed. When assembled into a composite stretch panel 4, the outer sheet 21 was used with its fiber orientation direction coincide with the stretch direction of the composite stretch panel 4. The basis weight, thickness, density, bending stiffness (in the direction corresponding to the stretch direction (direction X) of the composite stretch panel 4 and in the direction perpendicular thereto (direction Y)), and fiber orientation degree of the outer sheet 21 were as shown in Table 2.

Reference Example 4

An SMS (spunbonded-meltblown-spunbonded) nonwoven fabric fabricated from a PP resin in a conventional manner was used as an outer sheet 21. The SMS nonwoven fabric had a three-layer structure composed of upper and lower spunbonded layers of continuous PP fibers and an intermediate meltblown layer. The three layers were formed as follows.
Lower layer: A resin was melted, e.g., in an extruder and spun through a spinning nozzle into continuous filaments to obtain a spunbonded layer.
Intermediate layer: A molten PP resin was spun through a spinneret, and the resulting filaments were blown in a high humidity and high velocity gas into ultrafine filaments, which were collected on a collector into a web, which was put on the lower layer.
Upper layer: A molten resin was spun into continuous filaments in the same manner as described above to form a spunbonded web on the intermediate layer.
The thus obtained three-layered SMS nonwoven fabric was then fusion bonded by means of a heat embossing roller to have increased sheet strength. The embossing simultaneously achieved entanglement (uniting/smoothing) of the three layers. Having been embossed, the resulting nonwoven fabric had ensured sheet strength, integrity, and smoothness and was thin and excellent in hand (e.g., pliability).
The basis weight, thickness, and density of the outer sheet 21, the bending stiffness of the outer sheet 21 in the direction corresponding to the stretch direction (direction X) of a composite stretch panel 4 in which it is to be incorporated and in the direction perpendicular thereto (direction Y), and the degree of orientation of constituent fibers in the outer sheet 21 were as shown in Table 2.

Reference Example 5

A spunbonded nonwoven fabric made from a PP resin was used as an outer sheet 21. The spunbonded nonwoven fabric was fabricated in a conventional manner by melting a resin, e.g., in an extruder and spinning the molten resin through a spinning nozzle into continuous filaments. It is known that a laminate of two thicknesses of the spunbonded nonwoven fabric thus obtained will have increased thickness and hand. To retain the sheet strength, the resulting nonwoven fabric was fusion bonded by means of a heat embossing roller. The heat embossing simultaneously achieved entanglement (uniting/smoothing). Having been embossed, the resulting nonwoven fabric had ensured sheet strength, integrity, and smoothness and was thin and excellent in hand (e.g., pliability). The basis weight, thickness, density, and bending stiffness (in the direction corresponding to the stretch direction (direction X) of a composite stretch panel 4 and in the direction perpendicular thereto (direction Y)) of the outer sheet 21 and the degree of orientation of constituent fibers in the outer sheet 21 were as shown in Table 2.

Evaluation Item:

Disposable pull-on diapers were fabricated using Example 4 and Reference Examples 4 and 5 and evaluated as follows.

(1) A cross-sectional shape of the folds 43 of the composite stretch panel 4 was rated "good" when the shape was neatly curved or "poor" when the shape was angular.
(2) A plan shape of the folds 43 formed of the composite stretch panel 4 was rated "good" when they extended straight or "poor" when they were discontinuous.
(3) The folds 43 were evaluated for feel to the touch (hand) in terms of softness, fullness, and smoothness and rated according to the following standards.

(3-1) Softness
Good: The folds felt soft or gave no discomfort when worn.
Poor: The whole folds felt hard, giving discomfort when worn.
(3-2) Fullness
Good: The whole folds had bouncy fullness (cushioning properties) and recovered the original shape when collapsed.
Poor: The folds lacked fullness and did not recover the original shape when collapsed.
(3-3) Smoothness
Good: The folds were smooth with no scratchiness.
Poor: The folds were scratchy and not smooth.

(4) Comprehensive evaluation
Excellent: The folds had a curved cross-sectional shape, extended straight continuously, and was evaluated to be good in every equality of hand.
Good: The folds had a generally curved cross-sectional shape, extended straight, and was evaluated to be good in hand.
Fair: The cross-sectional shape of the folds was angular in part. The folds had small continuity. Not all the qualities of hand were rated good.
Poor: Neat folds were not formed. The folds were rated poor in every quality of hand.

Table 2

| | Example 4 | Reference Example 4 | Reference Example 5 |
|---|---|---|---|
| Process of Fabrication | through-air | SMS | spunbonding |
| Embossing | no | yes | yes |
| Material | PET/PE | PP/r-PP | PP |
| Basis Weight (g/m$^2$) | 20 | 15 | 16 |
| Thickness (mm) | 0.4 | 0.18 | 0.22 |
| Density (g/cm$^3$) | 0.050 | 0.083 | 0.072 |

(continued)

|  | Example 4 | Reference Example 4 | Reference Example 5 |
|---|---|---|---|
| Bending Stiffness in Direction X (cN·cm$^2$/cm) | 0.0117 | 0.0075 | 0.0225 |
| Bending Stiffness in Direction Y (cN·cm$^2$/cm) | 0.0031 | 0.0021 | 0.0107 |
| Degree of Orientation | 1.50 | 1.15 | 1.18 |
| (1) Cross-sectional Shape of Folds | good | poor | poor |
| (2) Plan Shape of Folds | good | poor | poor |
| (3) Hand of Folds |  |  |  |
| (3-1) Softness | good | good | poor |
| (3-2) Fullness | good | poor | poor |
| (3-3) Smoothness | good | poor | poor |
| (4) Comprehensive Evaluation | excellent | fair | poor |

Example 4 and Reference Examples 4 and 5 reveal the following.
The folds of Example 4 have a neatly curved cross-sectional shape and extend continuously in the diaper longitudinal direction because of the high bending stiffness of the outer sheet in direction X. The composite stretch panel of Example 4 forms a satisfactory waist portion because of the difference of bending stiffness between directions X and Y. The folds of Example 4 achieve high levels of hand qualities, i.e., softness, fullness, and smoothness. Thus, the disposable pull-on diaper of Example 4 was excellent in both appearance and feel to the touch.
Reference Example 4 uses an SMS nonwoven fabric having a good hand as an outer sheet. However, the folds have an angular top and are discontinuous in a plan view in the diaper longitudinal direction due to the low bending stiffness of the outer sheet in direction X. Although the folds feel soft, they remain collapsed when pressed by hand. The folds lack fullness (resilience) and smoothness. The folds fall short of desired levels of appearance and fullness.
Reference Example 5 uses a spunbonded nonwoven fabric with high strength as an outer sheet. The folds have an angular top and are discontinuous in the diaper longitudinal direction in a plan view due to the low bending stiffness of the outer sheet in direction X. In the evaluation of hand, the folds are hard as a whole and are therefore considered to give discomfort to a wearer.

Industrial Applicability

The absorbent article of the invention has a below-waist gather and a waist gather in its below-waist portion and waist portion, respectively, and is excellent in breathability and softness in the below-waist and the waist portions. The absorbent article of the invention is designed to make full use of the retractability of the elastic members used to form the below-waist gather and waist gather. The absorbent article of the invention exhibits excellent cushioning properties in the thickness direction in the below-waist and the waist portions. The waist gather of the article gently and comfortably hugs the waist of an infant wearer, leaving little indentation on the skin of the wearer.

**Claims**

1. An absorbent article comprising an absorbent body (3) containing an absorbent core (34) and an outer cover (2) joined to the garment facing side of the absorbent body, the outer cover (2) having a lateral direction and a longitudinal direction and a front section (A), a crotch section (C), and a rear section (B) in the longitudinal direction thereof, the front section and the rear section each having a waist portion (F) and a below-waist portion (D), **characterized in that** the outer cover (2) comprises an outer sheet (21), an inner sheet (22), and a plurality of elastic members (23, 24, 25, 26) between the outer and the inner sheets and has a composite stretch panel (4) having a stretch direction in each of the front section and the rear section, the composite stretch panel forming a waist gather (G2) and a below-waist gather (G1) in the waist portion and the below-waist portion, respectively, in each of the front and the rear sections, wherein the waist gather (G2) is formed over the whole circumference of the waist portion (F) while the below-waist gather (G1) of the below-waist portion (D) is formed only outside opposite side edges of the absorbent body (3), the composite stretch panel (4) having the outer sheet (21) and the inner sheet (22) bonded to each other at bonds (41) discretely provided in the stretch direction (X) and a direction perpendicular to the stretch direction (Y) and having the elastic members (23, 24, 25, 26) disposed at positions other than the bonds and fixed neither to the outer sheet nor the inner sheet at other than their opposite ends so that each of the outer sheet and the inner sheet forms a plurality of folds (43) of the gathers (G1, G2) continuously extending over a plurality of the elastic members, the waist portion (F) having a smaller extension percentage in a middle part (2A) thereof in the lateral direction of the outer cover than outer parts (2B) on both sides of the middle part in the lateral direction of the outer cover, the laterally middle part (2A) of the outer cover being a portion facing the absorbent body (3) in the flat- out state, and wherein the crotch section (C) of the outer cover (2) includes a frontal crotch portion (E) having a plurality of frontal crotch elastic members (26) arranged therein along the width direction of the outer cover (2) to be fixed in their stretched state between the outer sheet (21) and the inner sheet (22), whereby the frontal crotch portion (E) gathers to form a frontal crotch gather (G4).

2. The absorbent article according to claim 1, which is a pull-on article having a pair of side seals (11), a waist opening (12), and a pair of leg openings (13), wherein the outer cover has both lateral side edges of its front section and both lateral side edges of its rear section bonded together.

3. The absorbent article according to claim 2, wherein the composite stretch panel (4) comprises a fluted panel, the fluted panel being formed of the outer sheet (21) and the inner sheet (22) elasticized with the elastic members (23, 24, 25, 26) to form pairs of folds (102a, 102b) having basal parts and facing each other, each pair of folds making a tubular space therebetween, the basal parts of the folds (102a) formed of one of the sheets (103a)_and those of the folds (102b) formed of the other sheet (103b) being bonded to each other in pairs along at least one end edge of the fluted panel to make an array of nearly circular openings, the array of openings (124) forming at least part of the circumferential edge of the waist opening or at least part of the circumferential edge of each leg opening.

4. The absorbent article according to claim 3, wherein the elastic members ending at the circumferential edge of each leg opening are each fixed at an intersection between the circumferential edge of the leg opening and a seal line of the bonds such that the array of openings forms part of the circumferential edge of the leg opening.

5. The absorbent article according to claim 1, wherein the outer sheet (21) is a sheet of fibers, and the direction of orientation of the fibers is substantially coincide with the stretch direction of the composite stretch panel (4).

6. The absorbent article according to claim 1, wherein the inner sheet (22) is a sheet of fibers, and the direction of orientation of the fibers is substantially coincide with the stretch direction of the composite stretch panel (4).

7. The absorbent article according to claim 1, which has a waist flap (F) extending longitudinally outward from each longitudinal end of the absorbent body (3) in each of the front section and the rear section, the waist flap (F) in at least the rear section comprising a hydrophobic, skin contacting surface-defining sheet (27) defining the skin contacting surface of the waist flap, a hydrophobic, garment facing surface-defining sheet (21) defining the garment facing surface of the waist flap, a hydrophilic sheet (29) between the skin contacting surface-defining sheet and the garment facing surface-defining sheet, and elastic members (24) between the hydrophilic sheet and the garment facing surface-defining sheet, the waist flap (F) in at least the rear section has the waist gather formed of the composite stretch panel, and the composite stretch panel (4) being formed of two composite stretch panel-forming sheets (21, 22) facing each other, the composite stretch panel-forming sheets being a separate sheet between the hydrophilic sheet and the garment facing surface-defining sheet and the garment facing surface-defining sheet or being the hydrophilic sheet and the garment facing surface-defining sheet.

8. The absorbent article according to claim 7, wherein the waist flap (F) in the front section comprises the skin contacting surface-defining sheet (27), the garment facing surface-defining sheet (21), the hydrophilic sheet (29), and the elastic members (24), and the waist flap (F) in the front section has the waist gather formed of the composite stretch panel (4).

9. The absorbent article according to claim 7, which comprises the outer cover (2) and a holding sheet (27) covering the skin facing side of a longitudinal end portion of the absorbent body (3), the outer cover (2) comprising an outer sheet (21), an inner sheet (22), and a plurality of elastic members (23) between the outer and the inner sheets, the skin contacting surface-defining sheet being formed of the holding sheet,

the garment facing surface-defining sheet being formed of the outer sheet, and
the hydrophilic sheet (29) being formed of a sheet separate from either the outer sheet or the inner sheet or being formed of the inner sheet.

**Patentansprüche**

1. Absorbierender (saugfähiger) Artikel, der einen absorbierenden Körper (3) mit einem absorbierenden Kern (34) und eine an der kleidungszugewandten Seite des absorbierenden Körpers angebrachte Außenhülle (2) aufweist, wobei die Außenhülle (2) eine laterale Richtung und eine Längsrichtung hat sowie in Längsrichtung einen vorderen Abschnitt (A), einen Schrittabschnitt (C) und einen hinteren Abschnitt (B) aufweist, wobei sowohl der vordere Abschnitt wie auch der hintere Abschnitt einen Taillenabschnitt (F) und einen Hüftabschnitt (D) hat, **dadurch gekennzeichnet, dass**
die Außenhülle (2) eine äußere Schicht (21), eine innere Schicht (22) und zwischen der äußeren und inneren Schicht mehrere elastische Elemente (23,24,25,26) aufweist und sowohl in dem vorderen Abschnitt wie auch dem hinteren Abschnitt einen zusammengesetzten Dehnungseinsatzstreifen (4) mit einer Dehnungsrichtung hat, wobei der zusammengesetzte Dehnungseinsatzstreifen sowohl in dem vorderen wie auch dem hinteren Abschnitt eine Taillenkräuselung (G2) in dem Taillenabschnitt und eine Hüftkräuselung (G1) in dem Hüftabschnitt bildet, wobei die Taillenkräuselung (G2) um den gesamten Umfang des Taillenabschnitts (F) herum gebildet ist, während die Hüftkräuselung (G1) des Hüftabschnitts (D) nur außerhalb gegenüberliegender Seitenkanten des absorbierenden Kerns (3) gebildet ist,
der zusammengesetzte Dehnungseinsatzstreifen (4) konfiguriert ist, derart, dass die äußere Schicht (21) und die innere Schicht (22) an Verbindungsstellen (41), die diskret in die Dehnungsrichtung (X) und in eine zur Dehnungsrichtung senkrechte Richtung (Y) bereitgestellt sind, miteinander verbunden sind, und derart, dass die elastischen Elemente (23,24,25,26) an anderen Positionen als den Verbindungsstellen angeordnet sind und weder an der äußeren Schicht noch an der inneren Schicht befestigt sind, mit Ausnahme an ihren gegenüberliegenden Enden, so dass sowohl die äußere Schicht wie auch die innere Schicht mehrere Falten (43) der Kräuselung (G1,G2) bildet, die sich kontinuierlich über mehrere elastische Elemente hinweg erstrecken,
der Taillenabschnitt (F) in seinem zentralen Teil (2A) eine kleinere prozentuelle Streckbarkeit in die laterale Richtung der Außenhülle hat als in seinen an beiden Seiten des zentralen Teils angrenzenden äußeren Teilen (2B) in die laterale Richtung der Außenhülle, wobei der lateral zentrale Teil (2A) der Außenhülle ein Abschnitt ist, der in dem flach ausgebreiteten Zustand dem absorbierenden Körper (3) gegenüberliegt, und
der Schrittabschnitt (C) der Außenhülle (2) einen vorderen Schrittabschnitt (E) mit mehreren elastischen Elementen (26) hat, die in dem vorderen Schritt entlang der Breitenrichtung der Außenhülle (2) angeordnet sind und in ihrem gedehnten Zustand zwischen der äußeren Schicht (21) und der inneren Schicht (22) befestigt sind, wodurch sich der vordere Schrittabschnitt (E) kräuselt, um eine vordere Schrittkräuselung (G4) zu bilden.

2. Absorbierender Artikel nach Anspruch 1, der ein Anziehartikel mit einem Paar Seitenversiegelungen (11), einer Taillenöffnung (12) und einem Paar Beinöffnungen (13) ist, wobei die beiden lateralen Seitenkanten des vorderen Abschnitts der Außenhülle mit den beiden lateralen Seitenkanten des hinteren Abschnitts der Außenhülle verbunden sind.

3. Absorbierender Artikel nach Anspruch 2, wobei
der zusammengesetzte Dehnungseinsatzstreifen (4) einen gerafften Einsatzstreifen aufweist,
der geraffte Einsatzstreifen aus der äußeren Schicht (21) und der inneren Schicht (22) gebildet ist und mit den elastischen Elementen (23,24,25,26) elastisch gemacht ist, um Paare gegenüberliegender Falten (102a,102b) mit Basisabschnitten zu bilden, wobei die Falten jedes Paar einen rohrförmigen Raum zwischen sich bilden,
die Basisabschnitte der aus der einen Schicht (103a) gebildeten Falten (102a) und diejenigen der aus der anderen Schicht (103b) gebildeten Falten (102b) entlang mindestens einer Endkante des gerafften Einsatzstreifens paarweise miteinander verbunden sind, um eine Anordnung von ungefähr kreisförmigen Öffnungen zu bilden, wobei die Anordnung von Öffnungen (124) mindestens einen Teil der Umfangskante der Taillenöffnung oder mindestens einen Teil der Umfangskante jeder Beinöffnung bildet.

4. Absorbierender Artikel nach Anspruch 3, wobei die an der Umfangskante jeder Beinöffnung endenden elastischen Elemente jeweils an einem Kreuzungspunkt zwischen der Umfangskante der Beinöffnung und einer Versiegelungslinie der Verbindungsstellen befestigt sind, so dass die Anordnung von Öffnungen Teil der Umfangskante der Beinöffnung bildet.

**5.** Absorbierender Artikel nach Anspruch 1, wobei die äußere Schicht (21) eine Faserschicht ist und die Orientierungsrichtung der Fasern im Wesentlichen mit der Dehnungsrichtung des zusammengesetzten Dehnungseinsatzstreifens (4) übereinstimmt.

**6.** Absorbierender Artikel nach Anspruch 1, wobei die innere Schicht (22) eine Faserschicht ist und die Orientierungsrichtung der Fasern im Wesentlichen mit der Dehnungsrichtung des zusammengesetzten Dehnungseinsatzstreifens (4) übereinstimmt.

**7.** Absorbierender Artikel nach Anspruch 1, der sowohl in dem vorderen Abschnitt wie auch in dem hinteren Abschnitt eine Taillenlasche (F) hat, die sich von jedem Längsende des absorbierenden Körpers (3) aus in Längsrichtung auswärts erstreckt,
wobei die Taillenlasche (F) zumindest in dem hinteren Abschnitt aufweist: eine die hautkontaktierende Oberfläche definierende hydrophobe Schicht (27), die die hautkontaktierende Oberfläche der Taillenlasche definiert, eine die kleidungszugewandte Oberfläche definierende hydrophobe Schicht (21), die die kleidungszugewandte Oberfläche der Taillenlasche definiert, eine hydrophile Schicht (29) zwischen der die hautkontaktierende Oberfläche definierenden Schicht und der die kleidungszugewandte Oberfläche definierenden Schicht und elastische Elemente (24) zwischen der hydrophilen Schicht und der die kleidungszugewandte Oberfläche definierenden Schicht,
wobei zumindest in dem hinteren Abschnitt die Taillenlasche (F) die Taillenkräusel aus dem zusammengesetzten Dehnungseinsatzstreifen gebildet hat, und
der zusammengesetzte Dehnungseinsatzstreifen (4) gebildet ist aus zwei den zusammengesetzten Dehnungseinsatzstreifen bildenden gegenüberliegenden Schichten (21,22), wobei die den Dehnungseinsatzstreifen bildenden Schichten sind: eine separate Schicht, die zwischen der hydrophilen Schicht und der die kleidungszugewandte Oberfläche definierenden Schicht angeordnet ist, und die die kleidungszugewandte Oberfläche definierende Schicht, oder die hydrophile Schicht und die die kleidungszugewandte Oberfläche definierende Schicht.

**8.** Absorbierender Artikel nach Anspruch 7, wobei
die Taillenlasche (F) in dem vorderen Abschnitt die die hautkontaktierende Oberfläche definierende Schicht (27), die die kleidungszugewandte Oberfläche definierende Schicht (21), die hydrophile Schicht (29) und die elastischen Elemente (24) aufweist, und
die Taillenlasche (F) in dem vorderen Abschnitt die Taillenkräusel aus dem zusammengesetzten Dehnungseinsatzstreifen (4) gebildet hat.

**9.** Absorbierender Artikel nach Anspruch 7, der die Außenhülle (2) und eine Halteschicht (27) aufweist, die die hautzugewandte Seite eines Längsendeabschnitts des absorbierenden Körpers (3) bedeckt, wobei
die Außenhülle (2) eine äußere Schicht (21), eine innere Schicht (22) und zwischen der äußeren und inneren Schicht mehrere elastische Elemente (23) aufweist,
die die hautkontaktierende Oberfläche definierende Schicht aus der Halteschicht gebildet ist,
die die kleidungszugewandte Oberfläche definierende Schicht aus der äußeren Schicht gebildet ist, und
die hydrophile Schicht (29) aus einer sowohl von der äußeren Schicht wie auch der inneren Schicht separaten Schicht oder aus der inneren Schicht gebildet ist.

**Revendications**

**1.** Article absorbant comprenant un corps absorbant (3) contenant un coeur absorbant (34) et un recouvrement extérieur (2) joint au côté faisant face au vêtement du corps absorbant, le recouvrement extérieur (2) ayant un sens latéral et un sens longitudinal et une section avant (A), une section d'entrejambe (C), et une section arrière (B) dans le sens longitudinal de celui-ci, la section avant et la section arrière comportant chacune une portion de taille (F) et une portion au-dessous de la taille (D), **caractérisé en ce que**
le recouvrement extérieur (2) comprend une feuille extérieure (21), une feuille intérieure (22), et une pluralité d'organes élastiques (23, 24, 25, 26) entre les feuilles extérieure et intérieure et comporte un panneau extensible composite (4) ayant un sens d'extension dans chacune de la section avant et de la section arrière, le panneau extensible composite constituant une fronce de taille (G2) et une fronce au-dessous de la taille (G1) respectivement dans la portion de taille et dans la portion au-dessous de la taille, dans chacune de la section avant et de la section arrière, dans lequel la fronce de taille (G2) est constituée sur toute la circonférence de la portion de taille (F) alors que la fronce au-dessous de la taille (G1) de la portion au-dessous de la taille (D) est constituée uniquement sur des bords extérieurs de côtés opposés du corps absorbant (3),
le panneau extensible composite (4) comportant la feuille extérieure (21) et la feuille intérieure (22) liées l'une à l'autre à des liaisons (41) discrètement fournies dans le sens d'extension (X) et un sens perpendiculaire au sens d'extension (Y) et comportant les organes élastiques (23, 24, 25, 26) disposés à des positions autres que les liaisons et fixés ni sur la feuille extérieure ni sur la feuille intérieure à des positions autres que leurs extrémités opposées de sorte que chacune de la feuille extérieure et de la feuille intérieure forme une pluralité de plis (43) des fronces (G1, G2) s'étendant continuellement sur une pluralité des organes élastiques,

la portion de taille (F) ayant un pourcentage d'extension dans une partie de milieu (2A) de celle-ci dans le sens latéral du recouvrement extérieur plus petit que dans des parties extérieures (2B) des deux côtés de la partie de milieu dans le sens latéral du recouvrement extérieur, la partie de milieu latéralement (2A) du recouvrement extérieur constituant une portion faisant face au corps absorbant (3) dans l'état aplati, et

dans lequel la section d'entrejambe (C) du recouvrement extérieur (2) comprend une portion d'entrejambe frontale (E) comportant une pluralité d'organes élastiques d'entrejambe frontaux (26) agencés dans celle-ci dans le sens de la largeur du recouvrement extérieur (2) à fixer dans leur état étiré entre la feuille extérieure (21) et la feuille intérieure (22), de telle manière que la portion d'entrejambe frontale (E) se fronce pour constituer une fronce d'entrejambe frontale (G4).

**2.** Article absorbant selon la revendication 1, qui est un article à enfiler comportant une paire de joints latéraux (11), une ouverture de taille (12) et une paire d'ouvertures de jambes (13), dans lequel le recouvrement extérieur comporte les deux bords de côtés latéraux de sa section avant et les deux bords de côtés latéraux de sa section arrière liés les uns aux autres.

**3.** Article absorbant selon la revendication 2, dans lequel le panneau extensible composite (4) comprend un panneau plissé,

le panneau plissé étant constitué de la feuille extérieure (21) et de la feuille intérieure (22) élastiquées avec les organes élastiques (23, 24, 25, 26) pour constituer des paires de plis (102a, 102b) comportant des parties basales et se faisant face l'un à l'autre, chaque paire de plis constituant un espace tubulaire entre eux,

les parties basales des plis (102a) constituées de l'une des feuilles (103a) et les plis (102b) constitués de l'autre feuille (103b) étant liées les uns aux autres dans des paires le long d'au moins un bord d'extrémité du panneau plissé pour constituer un réseau d'ouvertures presque circulaires, le réseau d'ouvertures (124) constituant au moins une partie du bord circonférentiel de l'ouverture de taille ou au moins une partie du bord circonférentiel de chaque ouverture de jambe.

**4.** Article absorbant selon la revendication 3, dans lequel chacun des organes élastiques se terminant au bord circonférentiel de chaque ouverture de jambe est fixé à une intersection entre le bord circonférentiel de l'ouverture de jambe et une ligne de joint des liaisons de sorte que le réseau d'ouvertures fasse partie du bord circonférentiel de l'ouverture de jambe.

**5.** Article absorbant selon la revendication 1, dans lequel la feuille extérieure (21) est une feuille de fibres, et le sens d'orientation des fibres coïncide sensiblement avec le sens d'extension du panneau extensible composite (4).

**6.** Article absorbant selon la revendication 1, dans lequel la feuille intérieure (22) est une feuille de fibres, et le sens d'orientation des fibres coïncide sensiblement avec le sens d'extension du panneau extensible composite (4).

**7.** Article absorbant selon la revendication 1, qui comporte un rabat de taille (F) s'étendant longitudinalement vers l'extérieur à partir de chaque extrémité longitudinale du corps absorbant (3) dans chacune de la section avant et de la section arrière,

le rabat de taille (F) dans au moins la section arrière comprenant une feuille hydrophobe de définition de surface de contact avec la peau (27) définissant la surface de contact avec la peau du rabat de taille, une feuille hydrophobe de définition de surface faisant face au vêtement (21) définissant la surface faisant face au vêtement du rabat de taille, une feuille hydrophile (29) entre la feuille de définition de surface de contact avec la peau et la feuille de définition de surface faisant face au vêtement, et des organes élastiques (24) entre la feuille hydrophile et la feuille de définition de surface faisant face au vêtement,

le rabat de taille (F) dans au moins la section arrière a la fronce de taille constituée du panneau extensible composite, et

le panneau extensible composite (4) étant constitué de deux feuilles de formation de panneau extensible composite (21, 22) se faisant face l'une à l'autre, les feuilles de formation de panneau extensible composite étant une feuille distincte entre la feuille hydrophile et la feuille de définition de surface faisant face au vêtement et la feuille de définition de surface faisant face au vêtement ou étant la feuille hydrophile et la feuille de définition de surface faisant face au vêtement.

8. Article absorbant selon la revendication 7, dans lequel le rabat de taille (F) dans la section avant comprend la feuille de définition de surface de contact avec la peau (27), la feuille de définition de surface faisant face au vêtement (21), la feuille hydrophile (29), et les organes élastiques (24), et
le rabat de taille (F) dans la section avant a la fronce de taille constituée du panneau extensible composite (4).

9. Article absorbant selon la revendication 7, qui comprend le recouvrement extérieur (2) et une feuille de maintien (27) recouvrant le côté faisant face à la peau d'une portion d'extrémité longitudinale du corps absorbant (3),
le recouvrement extérieur (2) comprenant une feuille extérieure (21), une feuille intérieure (22), et une pluralité d'organes élastiques (23) entre les feuilles extérieure et intérieure,
la feuille de définition de surface de contact avec la peau étant constituée de la feuille de maintien,
la feuille de définition de surface faisant face au vêtement étant constituée de la feuille extérieure, et
la feuille hydrophile (29) étant constituée d'une feuille distincte de la feuille extérieure et de la feuille intérieure ou étant constituée de la feuille intérieure.

Fig.1

Fig.2

Fig.3

Fig.4

Fig. 5 (a)

34

B                    C

34B          34C

34A

34B          34C

B                    C

Fig. 5 (b)

34A

Fig. 5 (c)

34B     34A     34B

34C     34C

## Fig. 6

## Fig. 7

Fig. 8

Fig. 9

Fig. 10

Stomach side

Elastic member

Fig. 11

Anterior superior iliac spine
Anterior center

Posterior center

Anterior-to-posterior length
at the height of the anterior
superior iliac spine

Fig. 12

Fig. 13

Fig. 14

Fig. 15

Fig. 16

Fig.17(a)

Fig.17(b)

Fig.18(a)

Fig.18(b)

Fig. 19

## Fig. 20

## Fig. 21

## Fig. 22

Fig. **23**

Fig. 24

Fig. 25

Fig. 26

Fig. 27(a)

Fig. 27(b)

Fig. 28

Fig. 29

Fig. 30

Fig. 31

Fig. 32

Fig. 33

Fig. 34

Fig. 35

Fig. 36

Fig. 37

Fig. 38(a)

Fig. 38(b)

Fig. 39(a)

Fig. 39(b)

Fig. 40

Fig. 41

Fig. 42

Fig. 43

Fig. 44(a)

Fig. 44(b)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7217261 B2
- JP 8038548 A
- JP 2003235892 A
- EP 1550424 A
- EP 1547558 A
- US 20040186453 A
- JP 4166150 A

**Non-patent literature cited in the description**

- **SUEO KAWABATA.** HUAI HYOUKANO HYOUJY- UNKATO KAISEKI. The Textile Machinery Society, 10 July 1980